# FASCICULE DE BREVET EUROPEEN

(11) **EP 1 502 590 B1**
(45) Date de publication et mention de la délivrance du brevet: **20.12.2006**
(21) Numéro de dépôt: 04291901.9
(22) Date de dépôt: 27.07.2004
(51) Int. Cl.: A61K 31/428, C07D 277/68, A61P 3/10

(54) **Dérivés d'oximes hétérocycliques, leur procédé de préparation et leur utilisation dans le traitement du diabète de type II**
Heterocyclische Oximderivate, Verfahren zu ihrer Herstellung und Verwendung in der Behandlung von Typ II Diabetes
Heterocyclic oxime derivatives, process for their preparation and use thereof in the treatment of type II diabetes

(30) Priorité: 28.07.2003 FR 0309214
(43) Date de publication de la demande: 02.02.2005
(73) Titulaire: Les Laboratoires Servier, 92415 Courbevoie Cedex (FR)
(72) Inventeur: Leclerc, Véronique, 59000 Lille (FR); Pailloux, Sylvie, 86000 Poitiers (FR); Carato, Pascal, 59000 Lille (FR); Introvigne, Carine, 87270 Chaptelat (FR); Lebegue, Nicolas, 59212 Wignehies (FR); Berthelot, Pascal, 59320 Haubourdin (FR); Dacquet, Catherine, 75017 Paris (FR); Boutin, Jean Albert, 92150 Suresnes (FR); Caignard, Daniel Henri, 78230 Le Pecq (FR); Renard, Pierre, 78150 Le Chesnay (FR)

(56) Documents cités:
- WO-A-01/57002
- WO-A-03/027108

## Description

La présente invention concerne de nouveaux dérivés d'oximes hétérocycliques, leur procédé de préparation et les compositions pharmaceutiques qui les contiennent.

Les composés décrits dans la présente invention sont nouveaux et présentent des propriétés pharmacologiques particulièrement intéressantes : ce sont d'excellents agents hypoglycémiants et hypolipémiants.

Le traitement du diabète non insulino-dépendant de type II reste non satisfaisant en dépit de la mise sur le marché de nombreux dérivés hypoglycémiants oraux destinés à faciliter la sécrétion d'insuline et à favoriser son action au niveau des tissus cibles périphériques.

Lors des dix dernières années, une classe de composés de structure thiazolidinediones (US 5089514, US 5306726) a montré une activité antidiabétique marquée en favorisant la sensibilité à l'insuline dans les tissus périphériques cibles (muscles squelettiques, foie, tissu adipeux) de modèles animaux de diabète non insulino-dépendant de type II. Ces composés réduisent également les taux d'insuline et de lipides dans ces mêmes modèles animaux et induisent *in vitro* la différenciation de lignées cellulaires de préadipocytes en adipocytes (A. Hiragun et al., J. Cell. Physiol., 1988, 134, 124-130 ; R.F. Kleitzen et al., Mol. Pharmacol., 1992, 41, 393-398).
Le traitement des lignées cellulaires préadipocytaires avec la thiazolidinedione Rosiglitazone entraîne une induction de l'expression de gènes spécifiques du métabolisme lipidique comme aP2 et l'adipsine ainsi que l'expression des transporteurs du glucose GLUT1 et GLUT4, suggérant que l'effet des thiazolidinediones observé *in vivo* peut-être médié via le tissu adipeux. Cet effet spécifique est obtenu par la stimulation des facteurs nucléaires de transcription : « peroxisome proliferator-activated receptor gamma » (PPAR γ2). Ces dérivés sont susceptibles de restaurer la sensibilité à l'insuline au niveau des tissus périphériques tels que le tissu adipeux ou les muscles squelettiques (J.E. Gerich, New Engl. Med., 19, 321, 1231-1245).
Néanmoins, les dérivés de structure thiazolidinediones (troglitazone, rosiglitazone) ont montré chez l'homme des effets secondaires inquiétants, notamment des problèmes hépatiques (Script N° 2470, 1999, Sept. 8^{th}, 25).

De nombreux agents hypoglycémiants présentent des effets secondaires importants (hépatiques, cardiaques, hématopoïétiques) qui limitent leur utilisation à long terme dans le traitement du diabète non insulino-dépendant de type II.
Le développement de nouveaux agents thérapeutiques moins toxiques et actifs à long terme est absolument nécessaire dans cette pathologie.
Par ailleurs, l'hyperlipidémie est souvent observée chez les diabétiques (Diabete Care, 1995, 18 (supplément 1), 86/8/93). L'association de l'hyperglycémie avec l'hyperlipidémie favorise le risque de maladies cardiovasculaires chez les diabétiques. L'hyperglycémie, l'hyperlipidémie et l'obésité sont devenues des pathologies du monde moderne marqué par une prise de nourriture en grande quantité et un manque chronique d'exercice.
L'augmentation de la fréquence de ces pathologies appelle au développement de nouveaux agents thérapeutiques actifs dans ces maladies : des composés présentant une excellente activité hypoglycémiante et hypolipémiante en évitant les effets secondaires observés avec les thiazolidinediones sont par conséquent très utiles dans le traitement et/ou la prophylaxie de ces pathologies et particulièrement indiqués dans le traitement des diabètes non insulino-dépendants de type II pour réduire l'insulino-résistance périphérique et normaliser le contrôle du glucose (WO 01/57002).

Les composés de la présente invention, outre leur nouveauté, répondent à ces critères pharmacologiques et constituent d'excellents agents hypoglycémiants et hypolipémiants.

La présente invention concerne plus particulièrement les composés de formule (I) : 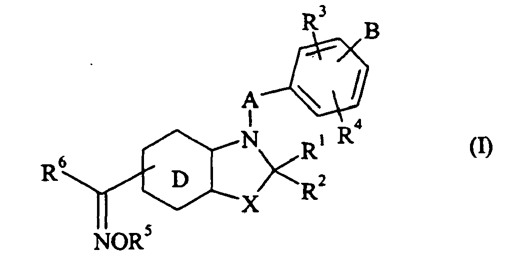 dans laquelle :
- X représente un atome d'oxygène ou de soufre, ou un groupement CH₂ ou 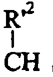 (dans lequel R'² forme avec R² une liaison supplémentaire),
- R¹ et R², identiques ou différents représentent un atome d'hydrogène, un groupement alkyle (C₁-C₆) linéaire ou ramifié, aryle, arylalkyle (C₁-C₆) linéaire ou ramifié, aryloxy, arylalkyloxy (C₁-C₆) linéaire ou ramifié, alkoxy (C₁-C₆) linéaire ou ramifié, hydroxy, amino, alkylamino (C₁-C₆) linéaire ou ramifié ou dialkylamino (C₁-C₆) linéaire ou ramifié,
   ou R¹ et R² forment ensemble un groupement oxo, thioxo ou imino,
   R² pouvant de plus former avec R'² une liaison supplémentaire,
- A représente une chaîne alkylène (C₁-C₆) dont un groupement CH₂ peut être remplacé par un hétéroatome choisi parmi oxygène ou soufre, ou par un groupement NRₐ (où Rₐ représente un atome d'hydrogène ou un groupement alkyle (C₁-C₆) linéaire ou ramifié), ou par un groupement phénylène ou naphtylène,
- R³ et R⁴, identiques ou différents, représentent un atome d'hydrogène ou d'halogène, ou un groupement R, OR ou NRR' (où R et R', identiques ou différents, représentent un atome d'hydrogène ou un groupement alkyle (C₁-C₆) linéaire ou ramifié, alkényle (C₂-C₆) linéaire ou ramifié, alkynyle (C₂-C₆) linéaire ou ramifié, aryle, arylalkyle (C₁-C₆) linéaire ou ramifié, arylalkényle (C₂-C₆) linéaire ou ramifié, arylalkynyle (C₂-C₆) linéaire ou ramifié, hétéroaryle, hétéroarylalkyle (C₁-C₆) linéaire ou ramifié, hétéroarylalkényle (C₂-C₆) linéaire ou ramifié, hétéroarylalkynyle (C₂-C₆) linéaire ou ramifié, cycloalkyle (C₃-C₈), cycloalkyl(C₃-C₈)alkyle(C₁-C₆) linéaire ou ramifié ou polyhalogénoalkyle (C₁-C₆) linéaire ou ramifié),
   ou R³ et R⁴ forment ensemble avec les atomes de carbone qui les portent, lorsqu'ils sont portés par deux atomes de carbone adjacents, un cycle comportant 5 ou 6 chaînons et pouvant contenir un hétéroatome choisi parmi oxygène, soufre et azote,
- R⁵ et R⁶, identiques ou différents, peuvent prendre toutes les valeurs de R tel que défini précédemment,
- D représente :
   un noyau benzénique et dans ce cas X ne peut représenter un groupement 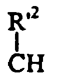 tel que défini précédemment,
   ou D représente un noyau pyridinique, pyrazinique, pyrimidinique ou pyridazinique,
- B représente un groupement alkyle (C₁-C₆) linéaire ou ramifié, ou alkényle (C₂-C₆) linéaire ou ramifié, ces groupements étant substitués :
   ◆ par un groupement de formule (II) : 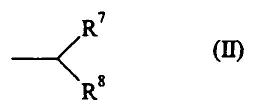 dans laquelle : - R⁷ représente un groupement 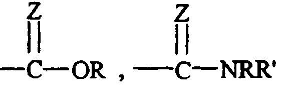 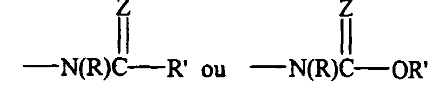 dans lesquels Z représente un atome d'oxygène ou de soufre et R et R', identiques ou différents, peuvent prendre les mêmes valeurs que définis précédemment,
      - et R⁸ représente un groupement aryle, arylalkyle dont la partie alkyle contient de 1 à 6 atomes de carbone et peut être linéaire ou ramifiée, hétéroaryle, hétéroarylalkyle dont la partie alkyle contient 1 à 6 atomes de carbone et peut être linéaire ou ramifiée, CN, tétrazole, ―OR ,―NRR' , 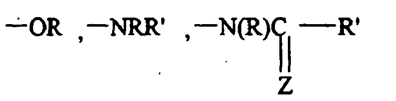 ou 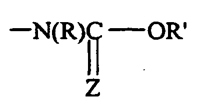 dans lesquels Z est tel que défini précédemment et R et R', identiques ou différents, peuvent prendre les mêmes valeurs que défini précédemment,
   ◆ ou par un groupement R⁹ où R⁹ représente un groupement CN, tétrazole, 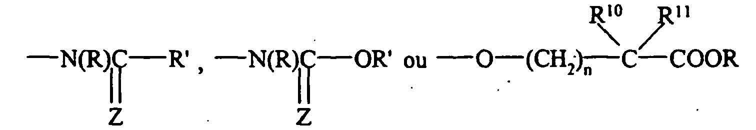 dans lesquels Z est tel que défini précédemment et R et R', identiques ou différents, peuvent prendre les mêmes valeurs que défini précédemment, n représente 0, 1, 2, 3, 4, 5 ou 6, et R¹⁰ et R¹¹, identiques ou différents, représentent un atome d'hydrogène ou un groupement alkyle (C₁-C₆) linéaire ou ramifié étant entendu que R¹⁰ et R¹¹ ne peuvent représenter simultanément un atome d'hydrogène,
ou B représente un groupement de formule (II) ou un groupement R⁹ tels que définis précédemment,
étant entendu que :
* l'oxime R⁶-C(=N-OR⁵)- peut être de configuration Z ou E,
* par aryle on entend un groupement phényle, naphtyle ou biphényle, ces groupements pouvant être partiellement hydrogénés,
* par hétéroaryle on entend tout groupement aromatique mono ou bicyclique contenant 5 à 10 chaînons, pouvant être partiellement hydrogéné sur un des cycles dans le cas des hétéroaryles bicycliques, et contenant 1 à 3 hétéroatomes choisis parmi oxygène, azote et soufre,
les groupements aryle et hétéroaryle ainsi définis pouvant être substitués par 1 à 3 groupements choisis parmi alkyle (C₁-C₆) linéaire ou ramifié, polyhalogénoalkyle (C₁-C₆) linéaire ou ramifié, alkoxy (C₁-C₆) linéaire ou ramifié, hydroxy, carboxy, formyle, NR_{b}R_{c} (dans lequel R_{b} et R_{c}, identiques ou différents, représentent un atome d'hydrogène, un groupement alkyle (C₁-C₆) linéaire ou ramifié, aryle ou hétéroaryle), ester, amido, nitro, cyano, ou atomes d'halogène,
leurs énantiomères et diastéréoisomères ainsi que leurs sels d'addition à un acide ou à une base pharmaceutiquement acceptables.

Parmi les acides pharmaceutiquement acceptables, on peut citer à titre non limitatif les acides chlorhydrique, bromhydrique, sulfurique, phosphonique, acétique, trifluoroacétique, lactique, pyruvique, malonique, succinique, glutarique, fumarique, tartrique, maléïque, citrique, ascorbique, méthanesulfonique, camphorique, oxalique, etc...

Parmi les bases pharmaceutiquement acceptables, on peut citer à titre non limitatif l'hydroxyde de sodium, l'hydroxyde de potassium, la triéthylamine, la tertbutylamine, etc...

Les composés préférés de l'invention sont les composés de formule (I) pour lesquels R¹ et R² forment ensemble un groupement oxo.

Le groupement préféré pour R³ et R⁴ est l'atome d'hydrogène.

Préférentiellement, A représente une chaîne alkylène dont un groupement CH₂ peut être remplacé par un hétéroatome et plus particulièrement par un atome d'oxygène.

Plus particulièrement, l'invention concerne les composés de formule (I) pour lesquels A représente un groupement éthylèneoxy.

Les groupements D préférés sont le noyau benzénique et le noyau pyridinique et plus particulièrement le noyau benzénique.

X représente préférentiellement un atome d'oxygène ou de soufre et plus particulièrement un atome de soufre.

Les groupements R⁵ préférés sont l'atome d'hydrogène et le groupement alkyle, comme par exemple le groupement méthyle.

R⁶ représente avantageusement un groupement phényle non substitué ou substitué par des groupements tels que alkyle, alkoxy, et atomes d'halogène.

Les groupements B préférés sont les groupements alkyle ou alkényle, et plus particulièrement les groupements alkyle, substitués par un groupement 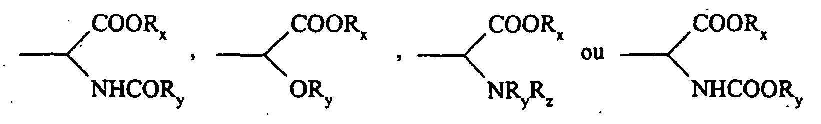 dans lesquels Rₓ, R_{y} et R_{z}, identiques ou différents, représentent :
un atome d'hydrogène ou un groupement alkyle comme par exemple les groupements méthyle, éthyle, propyle, isopropyle, butyle, isobutyle, tertbutyle, pentyle, isopentyle, néopentyle, hexyle,
un groupement polyhalogèno alkyle comme par exemple les groupements trifluorométhyle ou trifluoroéthyle,
ou un groupement phényle ou benzyle.

Plus particulièrement, l'invention concerne les composés de formule (I) pour lesquels B représente un groupement alkyle ou alkényle substitué par un groupement 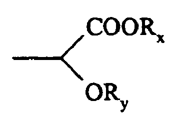 où Rₓ et R_{y} sont tels que défninis précédemment. B représente également avantageusement un groupement 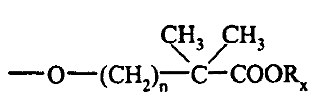 dans lequel n et Rₓ sont tels que définis précédemment.

Très avantageusement, l'invention concerne les composés de formule (I) pour lesquels :
X représente un atome de soufre,
R¹ et R² forment ensemble un groupement oxo,
A représente une chaîne ―CH₂―CH₂―O― ,
R³ et R⁴ représentent simultanément un atome d'hydrogène,
R⁵ représente un atome d'hydrogène ou un groupement alkyle,
R⁶ représente un groupement phényle ou phényle substitué et plus particulièrement substitué par un atome d'halogène, 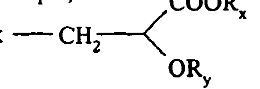
D représente un noyau benzénique,
B représente un groupement dans lequel Rₓ et R_{y} sont tels que définis précédemment.

Encore plus particulièrement, l'invention concerne les composés de formule (I) qui sont :
* le 2-éthoxy-3-{4-[2-(6-[(*E*)(méthoxyimino)(phényl)méthyl]-2-oxo-1,3-benzothiazol-3(2H)-yl)éthoxy]phényl}propanoate de méthyle,
* le 2-éthoxy-3-{4-[2-(6-[(*Z*)(hydroxyimino)(phényl)méthyl]-2-oxo-1,3-benzothiazol-3(2*H*)-yl)éthoxy]phényl}propanoate de méthyle,
* le 3-{4-[2-(6-[(méthoxyimino)(phényl)méthyl]-2-oxo-1,3-benzothiazol-3(2*H*)-yl)éthoxy]phényl}-2-(2,2,2-trifluoroéthoxy)propanoate de méthyle,
* le 3-{4-[2-(6-[(hydroxyimino)(phényl)méthyl]-2-oxo-1,3-benzothiazol-3(2*H*)-yl)éthoxy]phényl}-2-(2,2,2-trifluoro-éthoxy)propanoate de méthyle,
* l'acide 2-éthoxy-3-{4-[2-(6-[(méthoxyimino)(phényl)méthyl]-2-oxo-1,3-benzothiazol-3(2*H*)-yl)éthoxy]phényl}propanoïque,
* l'acide 2-éthoxy-3-{4-[2-(6-[(*E*)(méthoxyimino)(phényl)méthyl]-2-oxo-1,3-benzothiazol-3(2*H*)-yl)éthoxy]phényl}propanoïque,
* l'acide 2-éthoxy-3-{4-[2-(6-[(*E*)(méthoxy imino)(phényl)méthyl]-2-oxo-1,3-benzothiazol-3(2*H*)-yl)éthoxy]phényl} propanoïque énantiomère 1,
* l'acide 2-éthoxy-3-{4-[2-(6-[(*E*)(méthoxyimino)(phényl)méthyl]-2-oxo-1,3-benzothiazol-3(2*H*)-yl)éthoxy]phényl} propanoïque énantiomère 2,
* l'acide 2-éthoxy-3-{4-[2-(6-[(*Z*)(méthoxyimino)(phényl)méthyl]-2-oxo-1,3-benzothiazol-3(2*H*)-yl)éthoxy]phényl}propanoïque,
* l'acide 2-éthoxy-3-{4-[2-(6-[(*Z*)(méthoxyimino) (phényl)méthyl]-2-oxo-1,3-benzothiazol-3(2*H*)-yl)éthoxy]phényl} propanoïque énantiomère 1,
* l'acide 2-éthoxy-3-{4-[2-(6-[(*Z*)(méthoxyimino)(phényl)méthyl]-2-oxo-1,3-benzothiazol-3(2*H*)-yl)éthoxy]phényl} propanoïque énantiomère 2,
* l'acide 2-éthoxy-3-{4-[2-(6-[(hydroxyimino)(phényl)méthyl]-2-oxo-1,3-benzothiazol-3(2*H*)-yl)éthoxy]phényl}propanoïque,
* l'acide 2-éthoxy-3-{4-[2-(6-[(E)(hydroxyimino)(phényl)méthyl]-2-oxo-1,3-benzothiazol-3(2*H*)-yl)éthoxy]phényl}propanoïque,
* l'acide 2-éthoxy-3-{4-[2-(6-[(Z)(hydroxyimino)(phényl)méthyl]-2-oxo-1,3-benzothiazol-3(2*H*)-yl)éthoxy]phényl}propanoïque,
* le 3-{4-[2-(6-[(*Z*)(3-chlorophényl)(méthoxyimino)méthyl]-2-oxo-1,3-benzothiazol-3(2*H*)-yl)éthoxy]phényl}-2-éthoxypropanoate de méthyle,
* le 3-{4-[2-(6-[(3-chlorophényl)(hydroxyimino)méthyl]-2-oxo-1,3-benzothiazol-3(2*H*)-yl)éthoxy]phényl}-2-éthoxypropanoate de méthyle,
* le 2-méthoxy-3-{4-[2-(6-[(méthoxyimino)(phényl)méthyl]-2-oxo-1,3-benzothiazol-3(2*H*)-yl)éthoxy]phényl}propanoate de méthyle,
* l' acide 3-{4-[2-(6-[(*Z*)(3-chlorophényl)(méthoxyimino)méthyl]-2-oxo-1,3-benzothiazol-3(2*H*)-yl)éthoxy]phényl}-2-éthoxypropanoïque,
* l'acide 3-{4-[2-(6-[(méthoxyimino) (phényl)méthyl]-2-oxo-1,3-benzothiazol-3(2*H*)-yl)éthoxy]phényl}-2-(2,2,2-trifluoro éthoxy) propanoïque,
* l'acide 3-{4-[2-(6-[(*E*)(méthoxyimino)(phényl)méthyl]-2-oxo-1,3-benzothiazol-3(2*H*)-yl)éthoxy]phényl}-2-(2,2,2-trifluoroéthoxy) propanoïque,
* l'acide 3-{4-[2-(6-[(*E*)(méthoxyimino)(phényl)méthyl]-2-oxo-1,3-benzothiazol-3(2*H*)-yl)éthoxy]phényl}-2-(2,2,2-trifluoroéthoxy) propanoïque énantiomère 1,
* l'acide 3-{4-[2-(6-[(*E*)(méthoxyimino)(phényl)méthyl]-2-oxo-1,3-benzothiazol-3(2*H*)-yl)éthoxy] phényl}-2-(2,2,2-trifluoroéthoxy) propanoïque énantiomère 2,
* l'acide 3-{4-[2-(6-[(*Z*)(méthoxyimino)(phényl)méthyl]-2-oxo-1,3-benzothiazol-3(2*H*)-yl)éthoxy] phényl}-2-(2,2,2-trifluoroéthoxy) propanoïque,
* l'acide 3-{4-[2-(6-[(*Z*)(méthoxyimino) (phényl)méthyl]-2-oxo-1,3-benzothiazol-3(2*H*)-yl)éthoxy]phényl}-2-(2,2,2-trifluoro éthoxy) propanoïque énantiomère 1,
* l'acide 3-{4-[2-(6-[(*Z*)(méthoxyimino) (phényl)méthyl]-2-oxo-1,3-benzothiazol-3(2*H*)-yl)éthoxy]phényl}-2-(2,2,2-trifluoro éthoxy) propanoïque énantiomère 2,
* l'acide 3-{4-[2-(6-[(*tert*-butoxyimino)(phényl) méthyl]-2-oxo-1,3-benzothiazol-3(2*H*)-yl)éthoxy]phényl}-2-éthoxypropanoïque,
* le 2-[(*tert*-butoxycarbonyl)amino]-3-{4-[2-(6-[(méthoxyimino)(phényl)méthyl]-2-oxo-1,3-benzothiazol-3(2*H*)-yl)éthoxy]phényl}propanoate de méthyle,
* le 2-[(*tert*-butoxycarbonyl)amino]-3-{4-[2-(6-[(hydroxyimino)(phényl)méthyl]-2-oxo-1,3-benzothiazol-3(2*H*)-yl)éthoxy]phényl}propanoate de méthyle,
* le 2-[(butoxycarbonyl)amino]-3-{4-[2-(6-[(méthoxyimino)(phényl)méthyl]-2-oxo-1,3-benzothiazol-3(2*H*)-yl)éthoxy]phényl}propanoate de méthyle,
* le 3-{4-[2-(6-[(méthoxyimino)(phényl)méthyl]-2-oxo-1,3-benzothiazol-3(2*H*)-yl)éthoxy]phényl}-2-[(phénoxycarbonyl)amino]propanoate de méthyle,
* le 2-{[(benzyloxy)carbonyl]amino}-3-{4-[2-(6-[(hydroxyimino)(phényl) méthyl]-2-oxo-1,3-benzothiazol-3(2*H*)-yl)éthoxy]phényl}propanoate de méthyle,
* le 2-[(*tert*-butoxycarbonyl)(méthyl)amino]-3-{4-[2-(6-[(méthoxyimino)(phényl) méthyl]-2-oxo-1,3-benzothiazol-3(2*H*)-yl)éthoxy]phényl}propanoate de méthyle,
* la *N*-(*tert*-butoxycarbonyl)-4-[2-(6-[(hydroxyimino)(phényl)méthyl]-2-oxo-1,3-benzothiazol-3(2*H*)-yl)éthoxy]phénylalanine,
* la *N*-(*tert*-butoxycarbonyl)-4-[2-(6-[(méthoxyimino)(phényl)méthyl]-2-oxo-1,3-benzothiazol-3(2*H*)-yl)éthoxy]phénylalanine,
* le 2-amino-3-{4-[2-(6-[(méthoxyimino)(phényl)méthyl]-2-oxo-1,3-benzothiazol-3(2*H*)-yl)éthoxy]phényl}propanoate de méthyle,
* le 2-amino-3-{4-[2-(6-[(hydroxyimino)(phényl)méthyl]-2-oxo-1,3-benzothiazol-3(2*H*)-yl)éthoxy]phényl}propanoate de méthyle,
* le 3-{4-[2-(6-[(méthoxyimino)(phényl)méthyl]-2-oxo-1,3-benzothiazol-3(2*H*)-yl)éthoxy]phényl}-2-(méthylamino)propanoate de méthyle.

Les énantiomères, diastéréoisomères ainsi que les sels d'addition à un acide ou à une base pharmaceutiquement acceptables des composés préférés de l'invention font partie intégrante de l'invention.

La présente invention concerne également le procédé de préparation des composés de formule (I) caractérisé en ce que l'on utilise comme produit de départ un composé de formule (III) : 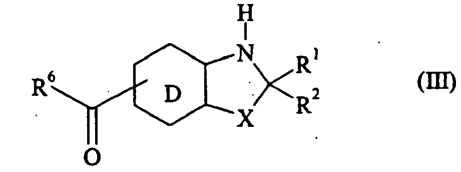 dans laquelle D, R¹, R², R⁶ et X sont tels que définis dans la formule (I),
sur lequel on condense en milieu basique un composé de formule (IV) : 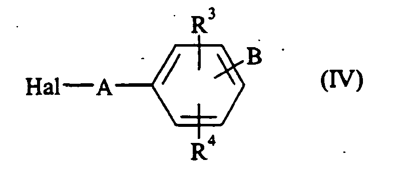 dans laquelle A, B, R³ et R⁴ sont tels que définis dans la formule (I) et Hal représente un atome d'halogène, pour conduire au composé de formule (V) : 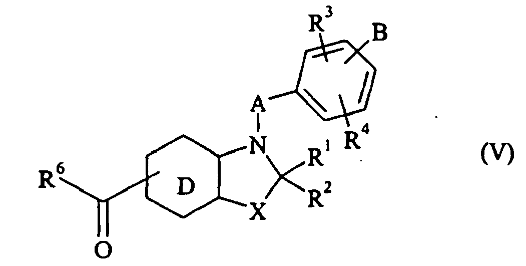 dans laquelle R¹, R², R³, R⁴, R⁶, A, B, D et X sont tels que définis dans la formule (I),
que l'on soumet à l'action d'un composé de formule R⁵O-NH₂ dans laquelle R⁵ est tel que défini dans la formule (I) pour conduire au composé de formule (I) : 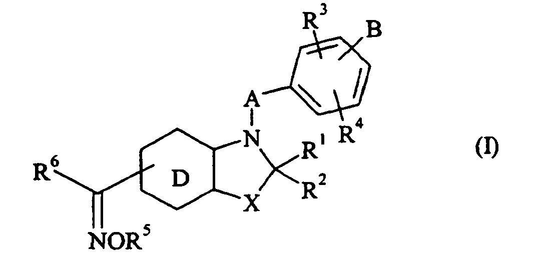 qui peut être purifié selon une technique classique de séparation, que l'on transforme, si on le souhaite en ses sels d'addition à un acide ou à une base pharmaceutiquement acceptable et dont on sépare éventuellement les isomères selon une technique classique de séparation.

Une variante avantageuse concerne le procédé de préparation des composés de formule (I) caractérisé en ce que l'on utilise comme produit de départ un composé de formule (III) : 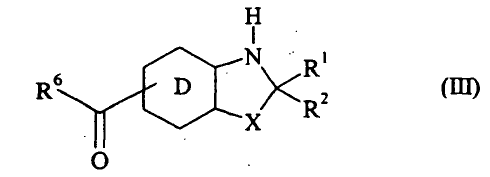 dans laquelle D, R¹, R², R⁶ et X sont tels que définis dans la formule (I),
sur lequel on condense un composé de formule R⁵O-NH₂ dans laquelle R⁵ est tel que défini dans la formule (I) pour conduire au composé de formule (VI) : 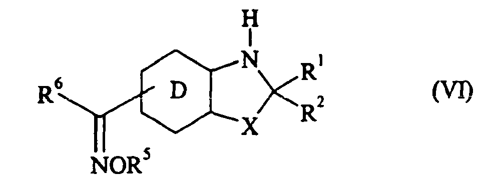 dans laquelle R¹, R², R⁵, R⁶, D et X sont tels que définis dans la formule (I),
sur lequel on condense en milieu basique un composé de formule (IV) : 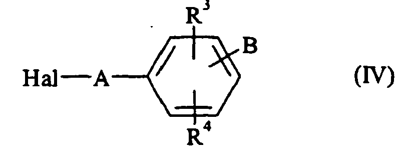 dans laquelle A, B, R³ et R⁴ sont tels que définis dans la formule (I) et Hal représente un atome d'halogène, pour conduire au composé de formule (I) : 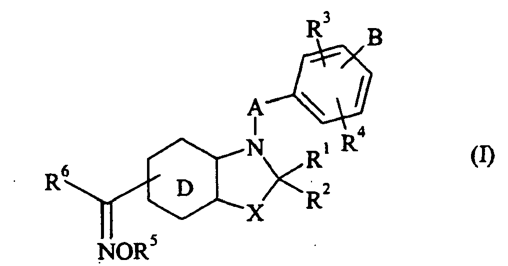 qui peut être purifié selon une technique classique de séparation, que l'on transforme, si on le souhaite en ses sels d'addition à un acide ou à une base pharmaceutiquement acceptable et dont on sépare éventuellement les isomères selon une technique classique de séparation.

Les composés de formule (III) sont commerciaux ou aisément accessibles à l'homme du métier par des réactions de chimie classiques ou décrits dans la littérature.

Un autre aspect de l'invention concerne également les composés de formule (V) : 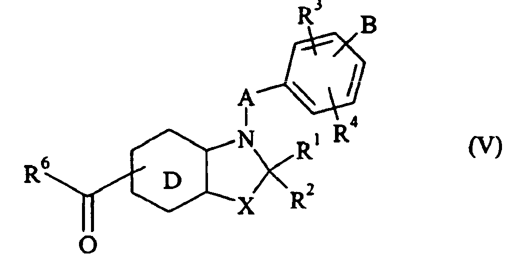 dans lesquels R⁶, D, X, A, R¹, R², R³, R⁴ et B sont tels que définis dans les composés de formule (I), utiles en tant qu'intermédiaires de synthèse des composés de formule (I) et en tant qu'agents hypoglycémiants et hypolipémiants.

Les composés de la présente invention possèdent des propriétés pharmacologiques très intéressantes.

Ces composés montrent notamment une excellente activité dans la réduction des taux de glucose sanguin. Ces propriétés justifient leur application en thérapeutique dans le traitement et/ou la prophylaxie des hyperglycémies, des dyslipidémies et plus particulièrement dans le traitement des diabètes non insulino-dépendants de type II, l'intolérance au glucose, les désordres reliés au syndrome X (incluant l'hypertension, l'obésité, la résistance à l'insuline, l'athérosclérose, l'hyperlipidémie), les maladies artérielles coronaires et d'autres maladies cardiovasculaires (incluant l'hypertension artérielle, l'insuffisance cardiaque, l'insuffisance veineuse), les maladies rénales (incluant les glomérulonéphrites, les gloméruloscléroses, le syndrôme néphrotique, la néphrosclérose hypertensive), les rétinopathies, les désordres reliés à l'activation des cellules endothéliales, le psoriasis, le syndrôme polycystique ovarien, la démence, les complications diabétiques et l'ostéoporose.
Ils peuvent être utilisés comme inhibiteurs de l'aldose réductase, pour améliorer les fonctions cognitives dans la démence et les complications du diabète, les maladies inflammatoires intestinales, les dystrophies myotoniques, les pancréatites, l'artériosclérose, le xanthome.

L'activité de ces composés est également recommandée pour le traitement et/ou la prophylaxie d'autres maladies incluant le diabète de type I, les hypertriglycéridémies, le syndrome X, la résistance à l'insuline, les dyslipidémies chez le diabétique, les hyperlipidémies, l'hypercholestérolémie, l'hypertension artérielle, l'insuffisance cardiaque, les maladies cardiovasculaires notamment l'athérosclérose.

De plus, ces composés sont indiqués pour être utilisés dans la régulation de l'appétit, notamment dans la régulation de la prise de nourriture chez des sujets souffrant de désordres tels que l'obésité, l'anorexie, la boulimie et l'anorexie nerveuse.
Ainsi, ces composés peuvent être utilisés en prévention ou pour le traitement de l'hypercholestérolémie, de l'obésité avec des effets avantageux sur l'hyperlipidémie, l'hyperglycémie, l'ostéoporose, l'intolérance au glucose, la résistance à l'insuline ou les maladies dans lesquelles l'insulino-résistance est un mécanisme physiopathologique secondaire.
L'utilisation de ces composés permet de réduire le cholestérol total, le poids corporel, la résistance à la leptine, le glucose plasmatique, les triglycérides, les LDL, les VLDL ainsi que les acides gras libres plasmatiques. Ils peuvent être utilisés en association avec des inhibiteurs de la HMG CoA réductase, les fibrates, l'acide nicotinique, la cholestyramine, le colestipol, le probucol, le GLP1, la metformine, les biguanides ou les inhibiteurs de la réabsorption du glucose, et peuvent être administrés ensembles ou à des périodes différentes pour agir en synergie chez le patient traité.

Ils présentent par ailleurs une activité dans les pathologies cancéreuses et notamment les cancers hormono-dépendants tels le cancer du sein et le cancer du colon, ainsi qu'un effet inhibiteur des processus d'angiogénèse impliqués dans ces pathologies.

Parmi les compositions pharmaceutiques selon l'invention, on pourra citer plus particulièrement celles qui conviennent pour l'administration orale, parentérale, nasale, per ou transcutanée, rectale, perlinguale, oculaire ou respiratoire et notamment les comprimés simples ou dragéifiés, les comprimés sublinguaux, les sachets, les paquets, les gélules, les glossettes, les tablettes, les suppositoires, les crèmes, les pommades, les gels dermiques et les ampoules buvables ou injectables.

La posologie varie selon le sexe, l'âge et le poids du patient, la voie d'administration, la nature de l'indication thérapeutique, ou des traitements éventuellement associés et s'échelonne entre 0,1 mg et 1 g par 24 heures en 1 ou plusieurs prises.

Les exemples suivants illustrent l'invention et ne la limitent en aucune façon.

### EXEMPLE 1 : 2-Ethoxy-3-{4-[2-(6-[(méthoxyimino)(phényl)méthyl]-2-oxo-1,3-benzothiazol-3(2H)-yl)éthoxy]phényl}propanoate de méthyle

### Stade A : 3-[4-(2-Chloroéthoxy)phényl]-2-éthoxy-2-propénoate de méthyle

Dans 150 ml de tétrahydrofuranne, fraîchement distillé, placés dans un bain d'eau glacée à 0°C, sous Argon, ajouter l'hydrure de sodium (0,0473 mole) puis le 2-éthoxy-2-diéthylphosphonoacétate de méthyle (0,0394 mole) en maintenant la température à 0°C. Après 1 heure d'agitation, ajouter le 4-(2-chloroéthoxy)benzaldéhyde (0,0394 mole) dissous dans le minimum de tétrahydrofuranne, fraîchement distillé, sous Argon à 0°C. Agiter la solution pendant 1 heure à 0°C. Laisser revenir la solution à température ambiante tout en agitant pendant 16 heures. Evaporer le tétrahydrofuranne. Ajouter 100 ml d'eau puis extraire avec deux fois 100 ml de dichlorométhane. La phase organique est lavée à l'eau, séchée sur MgSO₄, puis évaporée. Le produit huileux est purifié sur une colonne de gel de silice avec le mélange d'éluant : éther de pétrole/dichlorométhane (1/1). Le produit du titre est obtenu sous la forme d'une huile.

### Stade B : 3-[4-(2-Chloroéthoxy)phényl]-2-éthoxypropanoate de méthyle

Dans 100 ml de tétrahydrofuranne, ajouter le composé obtenu au stade A (0,0175 mole) puis le palladium sur charbon (0,2 g). Agiter à température ambiante sous hydrogène pendant 1 jour. Filtrer le palladium sur charbon puis évaporer le filtrat. Le produit huileux est purifié sur une colonne de gel de silice avec le mélange d'éluant : éther de pétrole/dichlorométhane (1/1). Le produit du titre est obtenu sous la forme d'une huile.

### Stade C : 3-{4-[2-(6-Benzoyl-2-oxo-1,3-benzothiazol-3(2H)-yl)éthoxy]phény}-2-éthoxypropanoate de méthyle

Dans 20 ml de diméthylformamide, ajouter le carbonate de potassium (0,01044 mole) puis la 6-benzoylbenzothiazolinone (0,00453 mole). Chauffer à 100°C pendant 1 heure. Ajouter le composé obtenu au stade B (0,00348 mole) et chauffer à 150°C pendant 16 heures. Evaporer le diméthylformamide. Reprendre le résidu par 50 ml d'eau puis extraire par deux fois 50 ml de dichlorométhane. La phase organique est séchée sur MgSO₄ puis évaporée. Le résidu est recristallisé dans le méthanol et conduit au produit du titre sous la forme d'un solide.
Point de fusion : 118-119°C

### Stade D :2-Ethoxy-3-{4-[2-(6-[(méthoxyimino)(phényl)méthyl]-2-oxo-1,3-benzothiazol-3(2H)-yl)éthoxy]phényl}propanoate de méthyle

Dans 30 ml de méthanol, ajouter le composé obtenu au stade C (0,00198 mole), le chlorhydrate de O-méthylhydroxylamine (0,00594 mole) et la pyridine (0,00594 mole). Chauffer à reflux pendant 3 heures. Evaporer à sec. Ajouter 100 ml d'acide chlorhydrique (1N) puis extraire par deux fois 50 ml de dichlorométhane. La phase organique est séchée sur MgSO₄ puis évaporée pour conduire au produit du titre.

### EXEMPLE 1a : 2-Ethoxy-3-{4-[2-(6-[(E)-(méthoxyimino)(phényl)méthyl]-2-oxo-1,3-benzothiazol-3(2H)-yl)éthoxy]phényl}propanoate de méthyle

Le composé obtenu dans le stade D de l'Exemple 1 est repris dans de l'éther diéthylique et le composé du titre est précipité de façon sélective et filtré pour conduire à l'isomère (*E*).
Point de fusion : 122-123°C

### EXEMPLE 1b : 2-Ethoxy-3-{4-[2-(6-[(Z)-(méthoxyimino)(phényl)méthyl]-2-oxo-1,3-benzothiazol-3(2H)-yl)éthoxy]phényl}propanoate de méthyle

Le filtrat obtenu dans l'Exemple 1a est évaporé et le résidu obtenu est recristallisé pour conduire au produit du titre.

### EXEMPLE 2 : 2-Ethoxy-3-{4-[2-(6-[(Z)(hydroxyimino)(phényl)méthyl]-2-oxo-1,3-benzothiazol-3(2H)-yl)éthoxy]phényl}propanoate de méthyle

### Stade A : 3-{4-[2-(6-Benzoyl-2-oxo-1,3-benzothiazol-3(2H)-yl)éthoxy]phényl}-2-éthoxypropanoate de méthyle

Dans 20 ml de diméthylformamide, ajouter le carbonate de potassium (0,01044 mole) puis la 6-benzoylbenzothiazolinone (0,00453 mole). Chauffer à 100°C pendant 1 heure. Ajouter le composé obtenu au stade B de l'Exemple 1 (0,00348 mole) et chauffer à 150°C pendant 16 heures. Evaporer le diméthylformamide. Reprendre le résidu par 50 ml d'eau puis extraire par deux fois 50 ml de dichlorométhane. La phase organique est séchée sur MgSO₄ puis évaporée. Le résidu est recristallisé dans le méthanol et conduit au produit du titre sous la forme d'un solide.
Point de fusion : 118-119°C

### Stade B : 2-Ethoxy-3-{4-[2-(6-[(Z)(hydroxyimino)(phényl)méthyl]-2-oxo-1,3-benzothiazol-3(2H)-yl)éthoxy]phényl}propanoate de méthyle

Dans 30 ml de méthanol, ajouter le composé obtenu au stade A (0,00198 mole), le chlorhydrate d'hydroxylamine (0,00594 mole) et la pyridine (0,00594 mole). Chauffer à reflux pendant 3 heures. Evaporer à sec. Ajouter 100 ml d'acide chlorhydrique (1N) puis extraire par deux fois 50 ml de dichlorométhane. La phase organique est séchée sur MgSO₄ puis évaporée. Le composé (*E*) est précipité avec de l'éther diéthylique. Le filtrat est ensuite évaporé pour conduire au produit (*Z*) du titre qui est recristallisé dans le méthanol.
Point de fusion : 114-115°C

### EXEMPLE 3 : 3-{4-[2-(6-[(Méthoxyimino)(phényl)méthyl]-2-oxo-1,3-benzothiazol-3(2H)-yl)éthoxy]phényl}-2-(2,2,2-trifluoroéthoxy)propanoate de méthyle

### Stade A : 3-[4-(2-Chloroéthoxy)phényl]-2-(2,2,2-trifluoroéthoxy)-2-propénoate de méthyle

On procède comme dans le stade A de l'Exemple 1 en remplaçant le 2-éthoxy-2-diéthylphosphonoacétate par le 2-(2,2,2-trifluorométhoxy)-2-diéthylphosphonoacétate.
Solide blanc.
Point de fusion : 50-51°C

### Stade B : 3-[4-(2-Chloroéthoxy)phényl]-2-(2,2,2-trifluoroéthoxy)propanoate de méthyle

On procède comme dans le stade B de l'Exemple 1 à partir du composé obtenu au stade A. Huile.

### Stade C : 3-{4-[2-(6-Benzoyl-2-oxo-1,3-benzothiazol-3(2H)-yl)éthoxy]phényl}-2-(2,2,2-trifluoroéthoxy)propanoate de méthyle

On procède comme dans le stade C de l'Exemple 1 à partir du composé obtenu au stade B.
Solide.
Point de fusion : 46-47°C.

### Stade D : 3-{4-[2-(6-[(Méthoxyimino)(phényl)méthyl]-2-oxo-1,3-benzothiazol-3(2H)-yl)éthoxy]phényl}-2-(2,2,2-trifluoroéthoxy)propanoate de méthyle

On procède comme dans le stade B de l'Exemple 2 à partir du composé obtenu au stade C et en remplaçant l'hydroxylamine par la O-méthylhydroxylamine.
Solide.
Point de fusion : 67-68°C.

### EXEMPLE 4 : 3-{4-[2-(6-[(Hydroxyimino)(phényl)méthyl]-2-oxo-1,3-benzothiazol-3(2H)-yl)éthoxy]phényl}-2-(2,2,2-trifluoroéthoxy)propanoate de méthyle

On procède comme dans le stade B de l'Exemple 2 à partir du composé obtenu au stade C de l'Exemple 3.
Solide.
Point de fusion : 55-56°C.

### EXEMPLE 5 : Acide 2-éthoxy-3-{4-[2-(6-[(méthoxyimino)(phényl)méthyl]-2-oxo-1,3-benzothiazol-3(2H)-yl)éthoxy]phényl}propanoïque

Le composé du titre est obtenu par saponification du composé obtenu dans l'Exemple 1 avec KOH dans un mélange Méthanol/eau.

### EXEMPLE 5a : Acide 2-éthoxy-3-{4-[2-(6-[(E)(méthoxyimino)(phényl)méthyl]-2-oxo-1,3-benzothiazol-3(2H)-yl)éthoxy]phényl}propanoïque

Le composé du titre est obtenu par saponification du composé obtenu dans l'Exemple 1a avec KOH dans un mélange Méthanol/eau.
Solide.
Point de fusion : 151-152°C.

Les Exemples 5a(1) et 5a(2) sont obtenus à partir du composé obtenu dans l'Exemple 5a par séparation chirale sur CHIRALPAK AD avec un mélange n-heptane/isopropanol/acide trifluoroacétique (750/250/1) et une détection à 280nm.

### EXEMPLE 5a(1) : Acide 2-éthoxy-3-{4-[2-(6-[(E)(méthoxyimino)(phényl)méthyl]-2-oxo-1,3-benzothiazol-3(2H)-yl)éthoxy]phényl}propanoique, énantiomère 1

Le composé du titre est obtenu avec une pureté optique supérieure à 99%.

### EXEMPLE 5a(2) : Acide 2-éthoxy-3-{4-[2-(6-[(E)(méthoxyimino)(phényl)méthyl]-2-oxo-1,3-benzothiazol-3(2H)-yl)éthoxy]phényl}propanoique, énantiomère 2

Le composé du titre est obtenu avec une pureté optique de 98,6%.

### EXEMPLE 5b : Acide 2-éthoxy-3-{4-[2-(6-[(Z)(méthoxyimino)(phényl)méthyl]-2-oxo-1,3-benzothiazol-3(2H)-yl)éthoxy]phényl}propanoïque

Le composé du titre est obtenu par saponification du composé obtenu dans l'Exemple 1b avec KOH dans un mélange Méthanol/eau.

Les Exemples 5b(1) et 5b(2) sont obtenus à partir du composé obtenu dans l'Exemple 5b par séparation chromatographique chirale.

### EXEMPLE 5b(1) : Acide 2-éthoxy-3-{4-[2-(6-[(Z)(méthoxyimino)(phényl)méthyl]-2-oxo-1,3-benzothiazol-3(2H)-yl)éthoxy]phényl}propanoique, énantiomère 1

### EXEMPLE 5b(2) : Acide 2-éthoxy-3-{4-[2-(6-[(Z)(méthoxyimino)(phényl)méthyl]-2-oxo-1,3-benzothiazol-3(2H)-yl)éthoxy]phényl}propanoique, énantiomère 2

### EXEMPLE 6 : Acide 2-éthoxy-3-{4-[2-(6-[(hydroxyimino)(phényl)méthyl]-2-oxo-1,3-benzothiazol-3(2H)-yl)éthoxy]phényl}propanoique

On procède comme dans l'Exemple 5 à partir du mélange (*Z*, *E*) obtenu dans l'Exemple 2.
Point de fusion : 84-85°C

### EXEMPLE 6a : Acide 2-éthoxy-3-{4-[2-(6-[(E)(hydroxyimino)(phényl)méthyl]-2-oxo-1,3-benzothiazol-3(2H)-yl)éthoxy]phényl}propanoique

On procède comme dans l'Exemple 5 à partir du composé (*E*) obtenu dans l'Exemple 2.

### EXEMPLE 6b : Acide 2-éthoxy-3-{4-[2-(6-[(Z)(hydroxyimino)(phényl)méthyl]-2-oxo-1,3-benzothiazol-3(2H)-yl)éthoxy]phényl}propanoique

On procède comme dans l'Exemple 5 à partir du composé (*Z*) obtenu dans l'Exemple 2.

### EXEMPLE 7 : 3-{4-[2-(6-[(Z)(3-Chlorophényl)(méthoxyimino)méthyl]-2-oxo-1,3-benzothiazol-3(2H)-yl)éthoxy]phényl}-2-éthoxypropanoate de méthyle

### Stade A : 3-{4-[2-(6-(3-Chlorobenzoyl)-2-oxo-1,3-benzothiazol-3(2H)-yl)éthoxy]phényl}-2-éthoxypropanoate de méthyle

On procède comme dans le stade C de l'Exemple 1 en remplaçant la 6-benzoylbenzothiazolinone par la 6-(3-chloro)benzoylbenzothiazolinone.
Point de fusion : 100-101°C.

### Stade B : 3-{4-[2-(6-[(Z)(3-Chlorophényl)(méthoxyimino)méthyl]-2-oxo-1,3-benzothiazol-3(2H)-yl)éthoxy]phényl}-2-éthoxypropanoate de méthyle

On procède comme dans le stade B de l'Exemple 2 à partir du composé obtenu au stade A et en remplaçant l'hydroxylamine par la O-méthylhydroxylamine. Solide.
Point de fusion : 113-114°C.

### EXEMPLE 8 : 3-{4-[2-(6-[(3-Chlorophényl)(hydroxyimino)méthyl]-2-oxo-1,3-benzothiazol-3(2H)-yl)éthoxy]phényl}-2-éthoxypropanoate de méthyle

On procède comme dans le stade B de l'Exemple 2 à partir du composé obtenu au stade A de l'Exemple 7. Les deux composés (Z) et (E) ne sont pas séparés.
Solide.
Point de fusion : 146-147°C.

### EXEMPLE 9 : 2-Méthoxy-3-{4-[2-(6-[(méthoxyimino)(phényl)méthyl]-2-oxo-1,3-benzothiazol-3(2H)-yl)éthoxy]phényl}propanoate de méthyle

On procède comme dans l'Exemple 1 en remplaçant le 2-éthoxy-2-diéthylphosphonoacétate par la 2-méthoxy-2-diéthylphosphonoacétate.
Point de fusion : 142-144°C

### EXEMPLE 10 : 2-[(Tert-butoxycarbonyl)amino]-3-{4-[2-(6-[(méthoxyimino) (phényl)méthyl] -2-oxo-1,3-benzothiazol-3(2H)-yl)éthoxy]phényl} propanoate de méthyle

### Stade A : 2-[(Tert-butoxycarbonyl)amino]-3-(4-hydroxyphényl)propanoate de méthyle

25 g de 2-amino-3-(4-hydroxyphényl)propionate de méthyle (128 mmol) sont dissous dans 110 ml de méthanol. La température est abaissée à 0°C et 53,4 ml de triéthylamine puis 30,73 g de di-*tert*-butyldicarbonate sont successivement additionnés. Le mélange est laissé revenir à température ambiante et le milieu réactionnel agité pendant une nuit.
Le solvant est évaporé et le résidu purifié par colonne de chromatographie sur gel de silice (éluant : cyclohexane/acétate d'éthyle 7/3) afin d'obtenir le composé du titre sous la forme d'un solide blanc.
Point de fusion : 106-108°C.

### Stade B : 2-[(Tert-butoxycarbonyl)amino]-3-[4-(2-chloroéthoxy)phényl]propanoate de méthyle

5 g (16,94 mmol) du composé obtenu au stade A sont mis en solution dans 100 ml d'acétone et 7,02 g de carbonate de potassium sont additionnés. Le mélange est porté au reflux du solvant et agité pendant 1 heure, puis 24,29 g de 1-bromo-2-chloro-éthane sont ajoutés et le milieu réactionnel est agité au reflux pendant 12 jours.
Le mélange est filtré et le filtrat évaporé. Le résidu obtenu est purifié par colonne de chromatographie sur gel de silice (éluant : éther de pétrole/AcOEt 8/2) pour donner le composé du titre sous la forme d'un solide blanc.
Point de fusion : 52-54°C.

### Stade C : 3-{4-[2-(6-Benzoyl-2-oxo-1,3-benzothiazol-3(2H)-yl)éthoxy]phényl}-2-[(tert-butoxycarbonyl)amino]propanoate de méthyle

3,6 g de 6-benzoylthiazolinone sont dissous dans 40 ml de diméthylformamide puis 4,87 g de carbonate de potassium sont additionnés et la température est amenée à 80°C pendant 1 heure. 6,05 g du composé obtenu au stade B sont alors ajoutés et le milieu réactionnel est agité à 100°C pendant une nuit.
Le mélange est filtré et le solvant évaporé à sec. Le résidu est repris par de l'acétate d'éthyle et lavé avec une solution saturée d'hydrogénocarbonate de sodium. Les phases organiques regroupées sont séchées sur sulfate de magnésium et concentrées. Le second résidu est purifié par colonne de chromatographie sur gel de silice (éluant : éther de pétrole/AcOEt 6/4). Le composé du titre est obtenu sous la forme d'un solide jaune.
Point de fusion : 76-79°C.

### Stade D : 2-[(Tert-butoxycarbonyl)amino]-3-{4-[2-(6-[(méthoxyimino)(phényl) méthyl] -2-oxo-1,3-benzothiazol-3(2H)-yl)éthoxy]phényl}propanoate de méthyle

On procède comme dans le stade B de l'Exemple 2 à partir du composé obtenu au stade C et en remplaçant l'hydroxylamine par la O-méthylhydroxylamine.
Huile.

### EXEMPLE 11 : 2-[(Tert-butoxycarbonyl)amino]-3-{4-[2-(6-[(hydroxyimino)(phényl) méthyl] -2-oxo-1,3-benzothiazol-3(2H)-yl)éthoxy]phényl}propanoate de méthyle

On procède comme dans le stade B de l'Exemple 2 à partir du composé obtenu au stade C de l'Exemple 10. Les deux composés (Z) et (E) ne sont pas séparés.
Point de fusion : 80-82°C.

### EXEMPLE 12 : 2-[(Butoxycarbonyl)amino]-3-{4-[2-(6-[(méthoxyimino)(phényl) méthyl]-2-oxo-1,3-benzothiazol-3(2H)-yl)éthoxy]phényl}propanoate de méthyle

### Stade A : 2-Amino-3-{4-[2-(6-benzoyl-2-oxo-1,3-benzothiazol-3(2H)-yl)éthoxy]phényl} propanoate de méthyle, trifluoroacetate

4 g du composé obtenu au stade C de l'Exemple 10 sont dissous dans 200 ml de dichlorométhane et la température est abaissée à 0°C. 12,47 ml d'acide trifluoroacétique sont alors ajoutés et le milieu réactionnel est agité à température ambiante pendant 4 heures.
Le solvant est évaporé, le mélange est repris par de l'eau et le pH ajusté à 7-8. La phase aqueuse est extraite au dichlorométhane puis les phases organiques sont séchées (MgSO₄) et évaporées. Le résidu est trituré dans de l'éther diéthylique et essoré pour donner le composé du titre sous la forme d'un solide blanc.
Point de fusion : 130-132°C.

### Stade B : 3-{4-[2-(6-Benzoyl-2-oxo-1,3-benzothiazol-3(2H)-yl)éthoxy]phényl}-2-[(butoxycarbonyl)amino]propanoate de méthyle

700 mg du composé obtenu au stade A, sont mis en solution dans 10 ml d'acétate d'éthyle puis 0,494 ml de triéthylamine sont additionnés et la température est abaissée à 0°C. 0,229 ml de chloroformiate de butyle sont ajoutés et le milieu est laissé revenir à température ambiante et agité pendant une nuit.
Le milieu réactionnel est filtré et le filtrat concentré. Le résidu est purifié par colonne de chromatographie sur gel de silice (éluant : éther de pétrole/AcOEt 7/3). Le composé du titre est obtenu sous la forme d'un solide blanc.
Point de fusion : 74-76°C.

### Stade C : 2-[(Butoxycarbonyl)amino]-3-{4-[2-(6-[(méthoxyimino)(phényl)méthyl]-2-oxo-1,3-benzothiazol-3(2H)-yl)éthoxy]phényl}propanoate de méthyle

On procède comme dans le stade B de l'Exemple 2 à partir du composé obtenu dans le stade B, en remplaçant l'hydroxylamine parla O-méthylhydroxylamine.

### EXEMPLE 13 : 3-{4-[2-(6-[(Méthoxyimino)(phényl)méthyl]-2-oxo-1,3-benzothiazol-3(2H)-yl)éthoxy]phényl}-2-[(phénoxycarbonyl)amino]propanoate de méthyle

### Stade A : 3-{4-[2-(6-Benzoyl-2-oxo-1,3-benzothiazol-3(2H)-yl)éthoxy]phényl}-2-[(phénoxycarbonyl)amino]propanoate de méthyle

1,5 g du composé obtenu au stade A de l'Exemple 12, sont mis en solution dans 20 ml d'acétate d'éthyle puis 1,06 ml de triéthylamine sont additionnés et la température est abaissée à 0°C. 0,637 ml de chloroformiate de phényle sont ajoutés et le milieu est laissé revenir à température ambiante et agité pendant une nuit.
Le milieu réactionnel est filtré et le filtrat concentré. Le résidu est purifié par colonne de chromatographie sur gel de silice (éluant : éther de pétrole/AcOEt 6/4). Le produit du titre est obtenu sous la forme d'un solide blanc.
Point de fusion : 74-76°C.

### Stade B : 3-{4-[2-(6-[(Méthoxyimino)(phényl)méthyl]-2-oxo-1,3-benzothiazol-3(2H)-yl)éthoxy]phényl}-2-[(phénoxycarbonyl)amino]propanoate de méthyle

On procède comme dans le stade B de l'Exemple 2 à partir du composé obtenu dans le stade A, en remplaçant l'hydroxylamine parle O-méthylhydroxylamine.

### EXEMPLE 14 : 2-{[(Benzyloxy)carbonyl]amino}-3-{4-[2-(6-[(hydroxyimino)(phényl) méthyl]-2-oxo-1,3-benzothiazol-3(2H)-yl)éthoxy]phényl}propanoate de méthyle

### Stade A : 3-{4-[2-(6-Benzoyl-2-oxo-1,3-benzothiazol-3(2H)-yl)éthoxy]phényl}-2-{[(benzyloxy)carbonyl]amino}propanoate de méthyle

1,5 g du composé obtenu au stade A de l'Exemple 12 sont mis en solution dans 20 ml d'acétate d'éthyle, puis 1,06 ml de triéthylamine sont additionnés et la température est abaissée à 0°C. 0,725 ml de chloroformiate de benzyle sont ajoutés et le milieu est laissé revenir à température ambiante et agité pendant une nuit.
Le milieu réactionnel est filtré et le filtrat concentré. Le résidu est purifié par colonne de chromatographie sur gel de silice (éluant : éther de pétrole/AcOEt 6/4). Le composé du titre est obtenu sous la forme d'un solide blanc.
Point de fusion : 64-68°C.

### Stade B : 2-{[(Benzyloxy)carbonyl]amino}-3-{4-[2-(6-[(hydroxyimino)(phényl) méthyl]-2-oxo-1,3-benzothiazol-3(2H)-yl)éthoxy]phényl}propanoate de méthyle

On procède comme dans le stade B de l'Exemple 2 à partir du composé obtenu au stade A.

### EXEMPLE 15 : 2-(Acétylamino)-3-{4-[2-(6-[(méthoxyimino)(phényl)méthyl]-2-oxo-1,3-benzothiazol-3(2H)-yl)éthoxy]phényl}propanoate de méthyle

### Stade A : 2-(Acétylamino)-3-{4-[2-(6-benzoyl-2-oxo-1,3-benzothiazol-3(2H)-yl)éthoxy]phényl}propanoate de méthyle

500 mg du composé obtenu au stade A de l'Exemple 12, sont mis en solution dans 10 ml d'acétate d'éthyle puis 0,353 ml de triéthylamine sont additionnés et la température est abaissée à 0°C. 0,121 ml de chlorure d'acétyle sont ajoutés et le milieu est laissé revenir à température ambiante et agité pendant une nuit.

Le milieu réactionnel est filtré et le filtrat concentré. Le résidu est recristallisé dans un mélange toluène/isopropanol (95/5). Le composé du titre est obtenu sous la forme d'un solide blanc.
Point de fusion : 142-144°C.

### Stade B : 2-(Acétylamino)-3-{4-[2-(6-[(méthoxyimino)(phényl)méthyl]-2-oxo-1,3-benzothiazol-3(2H)-yl)éthoxy]phényl}propanoate de méthyle

On procède comme dans le stade B de l'Exemple 2 à partir du composé obtenu au stade A en remplaçant l'hydroxylamine par la O-méthylhydroxylamine.

### EXEMPLE 16 : 2-(Butyrylamino)-3-{4-[2-(6-[(hydroxyimino)(phényl)méthyl]-2-oxo-1,3-benzothiazol-3(2H)-yl)éthoxy]phényl}propanoate de méthyle

### Stade A : 3-{4-[2-(6-Benzoyl-2-oxo-1,3-benzothiazol-3(2H)-yl)éthoxy]phényl}-2-(butyrylamino)propanoate de méthyle

500 mg du composé obtenu au stade A de l'Exemple 12 sont mis en solution dans 10 ml d'acétate d'éthyle, puis 0,353 ml de triéthylamine sont additionnés et la température est abaissée à 0°C. 0,177 ml de chlorure de butyryle sont ajoutés et le milieu est laissé revenir à température ambiante et agité pendant une nuit.
Le milieu réactionnel est filtré et le filtrat concentré. Le résidu est purifié par colonne de chromatographie sur gel de silice (éluant : éther de pétrole/AcOEt 6/4). Le composé du titre est obtenu sous la forme d'un solide blanc.
Point de fusion : 67-69°C.

### Stade B : 2-(Butyrylamino)-3-{4-[2-(6-[(hydroxyimino)(phényl)méthyl]-2-oxo-1,3-benzothiazol-3(2H)-yl)éthoxy]phényl}propanoate de méthyle

On procède comme dans le stade B de l'Exemple 2 à partir du composé obtenu au stade A.

### EXEMPLE 17 : 2-(Benzoylamino)-3-{4-[2-(6-[(hydroxyimino)(phenyl)methyl]-2-oxo-1,3-benzothiazol-3(2H)-yl)éthoxy]phényl}propanoate de méthyle

### StadeA : 2-(Benzoylamino)-3-{4-[2-(6-benzoyl-2-oxo-1,3-benzothiazol-3(2H)-yl)éthoxy]phényl}propanoate de méthyle

500 mg du composé obtenu au stade A de l'Exemple 12 sont mis en solution dans 10 ml d'acétate d'éthyle, puis 0,353 ml de triéthylamine sont additionnés et la température est abaissée à 0°C. 0,197 ml de chlorure de benzoyle sont ajoutés et le milieu est laissé revenir à température ambiante et agité pendant une nuit.
Le milieu réactionnel est filtré et le filtrat concentré. Le résidu est purifié par colonne de chromatographie sur gel de silice (éluant : éther de pétrole/AcOEt 7/3). Le composé du titre est obtenu sous la forme d'un solide blanc.
Point de fusion : 124-126°C.

### Stade B : 2-(Benzoylamino)-3-{4-[2-(6-[(hydroxyimino)(phenyl)methyl]-2-oxo-1,3-benzothiazol-3(2H)-yl)éthoxy]phényl}propanoate de méthyle

On procède comme dans le stade B de l'Exemple 2 à partir du composé obtenu au stade A.

### EXEMPLE 18 : 3-{4-[2-(6-[(Méthoxyimino)(phényl)méthyl]-2-oxo-1,3-benzothiazol-3(2H)-yl)éthoxy]phényl}-2-[(phénylacétyl)amino]propanoate de méthyle

### Stade A : 3-{4-[2-(6-Benzoyl-2-oxo-1,3-benzothiazol-3(2H)-yl)éthoxy]phényl}-2-[(phénylacétyl)amino]propanoate de méthyle

500 mg du composé obtenu au stade A de l'Exemple 12 sont mis en solution dans 10 ml d'acétate d'éthyle, puis 0,353 ml de triéthylamine sont additionnés et la température est abaissée à 0°C. 0,226 ml de chlorure de phénylacétyle sont ajoutés et le milieu est laissé revenir à température ambiante et agité pendant une nuit.
Le milieu réactionnel est filtré et le filtrat concentré. Le résidu est purifié par colonne de chromatographie sur gel de silice (éluant : éther de pétrole/AcOEt 8/2). Le composé du titre est obtenu sous la forme d'un solide blanc.
Point de fusion : 122-124°C.

### Stade B : 3-{4-[2-(6-[(Méthoxyimino)(phényl)méthyl]-2-oxo-1,3-benzothiazol-3(2H)-yl)éthoxy]phényl}-2-[(phénylacétyl)amino]propanoate de méthyle

On procède comme dans le stade B de l'Exemple 2 à partir du composé obtenu au stade A et en remplaçant l'hydroxylamine par la O-méthylhydroxylamine.

### EXEMPLE 19 : 2-Amino-3-{4-[2-(6-[(méthoxyimino)(phényl)méthyl]-2-oxo-1,3-benzothiazol-3(2H)-yl)éthoxy]phényl}propanoate de méthyle

Dissoudre le composé obtenu dans l'Exemple 10 (1 éq) dans 250 ml de DCM anhydre et ajouter goutte à goutte le TFA (20 éq). Laisser sous agitation pendant 5 heures, évaporer. Reprendre par de l'eau et alcaliniser par K₂CO₃ 10%. Extraire à l'acétate d'éthyle. Laver les phases organiques à l'eau, sécher sur MgSO₄, filtrer et évaporer. Reprendre par un minimum de méthanol et faire buller HCl gaz dans le milieu pendant 30 minutes. Evaporer à sec et reprendre par de l'éther diisopropylique. Filtrer le précipité, et recristalliser dans de l'éther diéthylique.
Point de fusion : 112-114°C.

### EXEMPLE 20 : 2-Amino-3-{4-[2-(6-[(hydroxyimino)(phényl)méthyl]-2-oxo-1,3-benzothiazol-3(2H)-yl)éthoxy]phényl}propanoate de méthyle

On procède comme dans l'Exemple 19 à partir du composé obtenu dans l'Exemple 11.
Point de fusion : 119-122°C.

### EXEMPLE 21 : 2-[(Tert-butoxycarbonyl)(méthyl)amino]-3-{4-[2-(6-[(méthoxyimino) (phényl) méthyl]-2-oxo-1,3-benzothiazol-3(2H)-yl)éthoxy]phényl} propanoate de méthyle

### Stade A : 3-{4-[2-(6-Benzoyl-2-oxo-1,3-benzothiazol-3(2H)-yl)éthoxy]phényl}-2-[(tert-butoxycarbonyl)(méthyl)amino]propanoate de méthyle

Dissoudre le composé obtenu au stade C de l'Exemple 10 (1 éq) et le CH₃I (2 éq) dans 55 ml de DMF anhydre et placer dans un bain de glace à 0°C. Ajouter NaH (1,5 éq) par portions. Laisser revenir à température ambiante. Hydrolyser, acidifier avec HCl 1N et filtrer. Purifier le produit brut obtenu par chromatographie sur gel de silice (éluant : éther diéthylique/toluène 2/8).
Huile.

### Stade B : 2-[(Tert-butoxycarbonyl)(méthyl)amino]-3-{4-[2-(6-[(méthoxyimino) (phényl) méthyl]-2-oxo-1,3-benzothiazol-3(2H)-yl)éthoxy]phényl} propanoate de méthyle

On procède comme dans le stade B de l'Exemple 2 à partir du composé obtenu au stade A et en remplaçant l'hydroxylamine par la O-méthylhydroxylamine.

### EXEMPLE 22 : 3-{4-[2-(6-[(Méthoxyimino)(phényl)méthyl]-2-oxo-1,3-benzothiazol-3(2H)-yl)éthoxy]phényl}-2-(méthylamino)propanoate de méthyle

On procède comme dans l'Exemple 19 à partir du composé obtenu dans l'Exemple 21.

### EXEMPLE 23 : 2-(2-Hydroxyphényl)-3-{4-[2-(6-[(méthoxyimino)(phényl)méthyl]-2-oxo-1,3-benzothiazol-3(2H)-yl)éthoxy]phényl}propanoate de méthyle

### 1^{er} Mode opératoire :

### Stade A : 6-Benzoyl-3-(2-{4-[(2-oxo-2,3-dihydro-1-benzofuran-3-yl)méthyl]phénoxy} éthyl)-1,3-benzothiazol-2(3H)-one

On procède comme dans le stade C de l'Exemple 1 à partir de la 6-benzoylbenzothiazolinone et de la 3-[4-(2-chloroéthoxy)benzyl]-1-benzofuran-2(3*H*)one.

### Stade B : 6-[(Méthoxyimino)(phényl)méthyl]-3-(2-{4-[(2-oxo-2,3-dihydro-1-benzofuran-3-yl)méthyl]phénoxy}éthyl)-1,3-benzothiazol-2(3H)-one

On procède comme dans le stade B de l'Exemple 2 à partir du composé obtenu au stade A et en remplaçant l'hydroxylamine par la O-méthylhydroxylamine.

### Stade C : 2-(2-Hydroxyphényl)-3-{4-[2-(6-[(méthoxyimino)(phényl)méthyl]-2-oxo-1,3-benzothiazol-3(2H)-yl)éthoxy]phényl}propanoate de méthyle

1,5 g du composé obtenu au stade B dans 50 ml de méthanol et 0,1 ml d'acide sulfurique concentré sont portés au reflux pendant 2 heures. Le solvant est ensuite évaporé, le résidu hydrolysé et le précipité obtenu est chromatographié sur gel de silice (éluant : AcOEt/toluène 5/95).
Point de fusion : 80-82°C.

### 2^{ème} Mode opératoire :

### Stade A : 3-{4-[2-(6-Benzoyl-2-oxo-1,3-benzothiazol-3(2H)-yl)éthoxy]phényl}-2-(2-hydroxyphényl)propanoate de méthyle

On procède comme dans le stade C de l'Exemple 1 à partir de la 6-benzoylbenzothiazolinone et du 3-[4-(2-chloroéthoxy)phényl]-2-(2-hydroxyphényl) propanoate de méthyle.
Point de fusion : 119-121°C.

### Stade B : 2-(2-Hydroxyphényl)-3-{4-[2-(6-[(méthoxyimino)(phényl)méthyl]-2-oxo-1,3-benzothiazol-3(2H)-yl)éthoxy]phényl}propanoate de méthyle

On procède comme dans le stade D de l'Exemple 1 à partir du composé obtenu au stade A.
Point de fusion : 80-82°C.

### EXEMPLE 24 : Acide 2-(2-hydroxyphényl)-3-{4-[2-(6-[(méthoxyimino)(phényl) méthyl]-2-oxo-1,3-benzothiazol-3(2H)-yl)éthoxy]phényl}propanoique

Le produit du titre est obtenu après saponification du composé obtenu dans l'Exemple 23 avec un mélange NaOH/Dioxanne.

### EXEMPLE 25 : 2-Benzyl-3-{4-[2-(6-[(méthoxyimino)(phényl)méthyl]-2-oxo-1,3-benzothiazol-3(2H)-yl)éthoxy]phényl}propanoate de méthyle

### Stade A : 2-{4-[2-(6-Benzoyl-2-oxo-1,3-benzothiazol-3(2H)-yl)éthoxy]benzyl}-3-phénylpropanoate de méthyle

On procède comme dans le stade C de l'Exemple 1 en remplaçant le [(2-chloroéthoxy)phényl]-2-éthoxypropanoate de méthyle par le 2-benzyl-3-[4-(2-chloroéthoxy)phényl]propanoate de méthyle.
Le composé du titre est obtenu sous la forme d'un solide beige.
Point de fusion : 65-67°C.

### Stade B : 2-Benzyl-3-{4-[2-(6-[(méthoxyimino)(phényl)méthyl]-2-oxo-1,3-benzothiazol-3(2H)-yl)éthoxy]phényl}propanoate de méthyle

On procède comme dans le stade B de l'Exemple 2 à partir du composé obtenu au stade A et en remplaçant l'hydroxylamine par la O-méthylhydroxylamine.

### EXEMPLE 26 : 2-{4-[2-(6-[(Méthoxyimino)(phényl)méthyl]-2-oxo-1,3-benzothiazol-3(2H)-yl)éthoxy]benzyl}-4-phenylbutanoate de méthyle

### Stade A : 2-{4-[2-(6-Benzoyl-2-oxo-1,3-benzothiazol-3(2H)-yl)éthoxy]benzyl}-4-phénylbutanoate de méthyle

On procède comme dans le stade C de l'Exemple 1 en remplaçant le [(2-chloroéthoxy)phényl]-2-éthoxypropanoate de méthyle par le 2-[4-(2-chloroéthoxy)benzyl]-4-phénylbutanoate de méthyle.
Solide blanc.
Point de fusion : 51-54°C.

### Stade B : 2-{4-[2-(6-[(Méthoxyimino)(phényl)méthyl]-2-oxo-1,3-benzothiazol-3(2H)-yl)éthoxy]benzyl}-4-phenylbutanoate de méthyle

On procède comme dans le stade B de l'Exemple 2 à partir du composé obtenu au stade A en remplaçant l'hydroxylamine par la O-méthylhydroxylamine. Les deux composés (Z) et (E) ne sont pas séparés.
Solide blanc.
Point de fusion : 80-83°C.

### EXEMPLE 27 : 2-{4-[2-(6-[(Hydroxyimino)(phényl)méthyl]-2-oxo-1,3-benzothiazol-3(2H)-yl)éthoxy]benzyl}-4-phenylbutanoate de méthyle

On procède comme dans le stade B de l'Exemple 2 à partir du composé obtenu au stade A de l'Exemple 26. Les deux composés (Z) et (E) ne sont pas séparés.
Solide beige.
Point de fusion : 58-60°C.

### EXEMPLE 28 : 2-{4-[2-(6-[(Hydroxyimino)(phényl)méthyl]-2-oxo-1,3-benzothiazol-3(2H)-yl)éthoxy]benzyl}-5-phénylpentanoate de méthyle

### Stade A : 2-{4-[2-(6-Benzoyl-2-oxo-1,3-benzothiazol-3(2H)-yl)éthoxy]benzyl}-5-phénylpentanoate de méthyle

On procède comme dans le stade C de l'Exemple 1 en remplaçant la 3-[4-(2-chloroéthoxy)phényl]-2-éthoxypropanoate de méthyle par le 2-[4-(2-chloroéthoxy)benzyl]-5-phénylpentanoate de méthyle.
Solide beige.
Point de fusion : 55-57°C.

### Stade B : 2-{4-[2-(6-[(Hydroxyimino)(phényl)méthyl]-2-oxo-1,3-benzothiazol-3(2H)-yl)éthoxy]benzyl}-5-phénylpentanoate de méthyle

On procède comme dans le stade B de l'Exemple 2 à partir du composé obtenu au stade A.

### EXEMPLE 29 : N-(Tert-butoxycarbonyl)-4-[2-(6-[(hydroxyimino)(phényl)méthyl]-2-oxo-1,3-benzothiazol-3(2H)-yl)éthoxy]phénylalanine

Le composé du titre est obtenu par saponification en milieu basique du composé obtenu dans l'Exemple 11.
Point de fusion : Décomposition après 250°C.

### EXEMPLE 30 : N-(Tert-butoxycarbonyl)-4-[2-(6-[(méthoxyimino)(phényl)méthyl]-2-oxo-1,3-benzothiazol-3(2H)-yl)éthoxy]phénylalanine

Le composé du titre est obtenu par saponification en milieu basique du composé obtenu dans l'Exemple 10.

### EXEMPLE 31 : 2-{4-[2-(6-[(Méthoxyimino)(phényl)méthyl]-2-oxo-1,3-benzothiazol-3(2H)-yl)éthoxy]phénoxy}-2-méthylpropanoate d'éthyle

### Stade A : 2-{4-[2-(6-Benzoyl-2-oxo-1,3-benzothiazol-3(2H)-yl)éthoxy]phénoxy}-2-méthylpropanoate d 'éthyle

A une solution de 6-benzoyl-benzothiazolinone (6,7 mmol) en présence de K₂CO₃ dans 20 ml de DMF chauffée à 80°C pendant 2 heures, est ajouté le 2-[4-(2-chloroéthoxy)phénoxy]-2-méthyl propanoate d'éthyle. Le milieu réactionnel est chauffé à 120°C pendant 5 jours, refroidi, hydrolysé par 100 ml d'eau et alcalinisé par NaOH 1N. Le précipité obtenu est filtré puis recristallisé dans le cyclohexane pour conduire au produit du titre sous la forme d'une poudre blanche.
Point de fusion : 120-121°C.

### Stade B : 2-{4-[2-(6-[(Méthoxyimino)(phényl)méthyl]-2-oxo-1,3-benzothiazol-3(2H)-yl)éthoxy]phénoxy}-2-méthylpropanoate d'éthyle

Une solution du composé obtenu au stade A (500 mg) et de chlorhydrate de O-methylhydroxylamine (165 mg) dans 15 ml de pyridine est chauffée à reflux pendant 3 heures. Le milieu réactionnel est hydrolysé par 100 ml d'eau glacée, acidifié par HCl 6N puis extrait à l'acétate d'éthyle. La phase organique est séchée sur sulfate de magnésium et évaporée à sec sous pression réduite. Le résidu est alors purifié sur gel de silice, éluant AcOEt/EP 2/8, pour conduire au produit du titre sous la forme d'une huile blanche (cire).

### EXEMPLE 32 : 2-{4-[2-(6-[(Hydroxyimino)(phényl)méthyl]-2-oxo-1,3-benzothiazol-3(2H)-yl)éthoxy]phénoxy}-2-méthylpropanoate d'éthyle

Une solution du composé obtenu au stade A de l'Exemple 31 (500 mg) et de chlorhydrate d'hydroxylamine (137 mg) dans 10 ml de pyridine est chauffée à reflux pendant 3 heures. Le milieu réactionnel est hydrolysé par 100 ml d'eau glacée et acidifié par HCl 6N. Le précipité obtenu est alors filtré, lavé à l'eau puis à l'éther de pétrole et purifié sur gel de silice (éluant AcOEt/EP 2/8) pour conduire au produit du titre sous la forme d'une poudre blanche.
Point de fusion : 60-62°C.

### EXEMPLE 33 : Acide 2-{4-[2-(6-[(méthoxyimino)(phényl)méthyl]-2-oxo-1,3-benzothiazol-3(2H)-yl)éthoxy]phénoxy}-2-méthylpropanoique

A une solution du composé obtenu au stade B de l'Exemple 31 (0,8 g) dans 5 ml d'éthanol 95°, est ajouté l'hydroxyde de potassium (100 mg) dissout dans 1 ml d'éthanol 95°. Le milieu réactionnel est chauffé à reflux durant une nuit. Après refroidissement à température ambiante, la solution est acidifiée par HCl 1N. Le précipité obtenu est alors filtré puis purifié sur phase inverse RP18, éluant MeOH/H₂O 6/4, pour conduire au produit du titre sous la forme d'une poudre blanche.
Point de fusion : Décomposition après 170°C.

### EXEMPLE 34 : Acide 2-{4-[2-(6-[(hydroxyimino)(phényl)méthyl]-2-oxo-1,3-benzothiazol-3(2H)-yl)éthoxy]phénoxy}-2-méthylpropanoique

A une solution du composé obtenu dans l'Exemple 32 (0,5 g) dans 5 ml d'éthanol 95°, est ajouté l'hydroxyde de potassium (107 mg) dissout dans 1 ml d'éthanol 95°. Le milieu réactionnel est chauffé à reflux durant une nuit. Après refroidissement à température ambiante, la solution est acidifiée par HCl 1N. Le précipité obtenu est alors filtré puis purifié sur phase inverse RP18, éluant MeOH/H₂O 6/4, pour conduire au produit du titre sous la forme d'une poudre blanche.
Point de fusion : Décomposition après 170°C.

### EXEMPLE 35 : 2-{4-[2-(6-[(3-Chlorophényl)(méthoxyimino)méthyl]-2-oxo-1,3-benzothiazol-3(2H)-yl)éthoxy]phénoxy}-2-méthylpropanoate d'éthyle

### Stade A : 2-{4-[2-(6-(3-Chlorobenzoyl)-2-oxo-1,3-benzothiazol-3(2H)-yl)éthoxy]phénoxy}-2-méthylpropanoate d'éthyle

A une solution de 6-(3'-chloro-benzoyl)-benzothiazolinone (1,8 g) en présence de K₂CO₃ (1,72 g) dans 20 ml de DMF chauffée à 80°C pendant 2 heures, est ajouté le 2-[4-(2-chloroéthoxy)phénoxy]-2-méthylpropanoate d'éthyle (1,96 g). Le milieu réactionnel est chauffé à 120°C pendant 5 jours, refroidi, hydrolysé par 100 ml d'eau et alcalinisé par NaOH 1N. Le précipité obtenu est filtré puis recristallisé dans le cyclohexane pour conduire au produit du titre sous la forme d'une poudre blanche.
Point de fusion : 125-126°C.

### Stade B : 2-{4-[2-(6-[(3-Chlorophényl)(méthoxyimino)méthyl]-2-oxo-1,3-benzothiazol-3(2H)-yl)éthoxy]phénoxy}-2-méthylpropanoate d'éthyle

Une solution du composé obtenu au stade A (1,00 g) et de chlorhydrate de O-methylhydroxylamine (309 mg) dans 15 ml de pyridine est chauffée à reflux pendant 3 heures. Le milieu réactionnel est hydrolysé par 100 ml d'eau glacée, acidifié par HCl 6N. Le précipité obtenu est filtré, lavé à l'eau puis recristallisé dans l'éther de pétrole, en le refroidissant au congélateur, pour conduire au produit du titre sous la forme d'une poudre blanche.
Point de fusion : 93-98°C.

### EXEMPLE 36 : Acide 2-{4-[2-(6-[(3-chlorophényl)(méthoxyimino)méthyl]-2-oxo-1,3-benzothiazol-3(2H)-yl)éthoxy]phénoxy}-2-méthylpropanoique

A une solution du composé obtenu dans l'Exemple 35 (0,4 g) dans 5 ml d'éthanol 95°, est ajouté l'hydroxyde de potassium (40 mg) dissout dans 1 ml d'éthanol 95°. Le milieu réactionnel est chauffé à reflux durant une nuit. Après refroidissement à température ambiante, la solution est acidifiée par HCl 1N. Le précipité obtenu est alors filtré puis purifié sur phase inverse RP18, éluant MeOH/H₂O 6/4, pour conduire au produit du titre sous la forme d'une poudre blanche.
Point de fusion : dégradation à partir de 230°C.

### EXEMPLE 37 : 2-{4-[2-(6-[(3-Chlorophényl)(hydroxyimino)méthyl]-2-oxo-1,3-benzothiazol-3(2H)-yl)éthoxy]phénoxy}-2-méthylpropanoate d'éthyle

Une solution du composé obtenu au stade A de l'Exemple 35 (1,30 g) et de chlorhydrate d'hydroxylamine (335 mg) dans 10 ml de pyridine est chauffée à reflux pendant 3 heures. Le milieu réactionnel est hydrolysé par 100 ml d'eau glacée et acidifié par HCl 6N. Le précipité obtenu est alors filtré, lavé à l'eau puis à l'éther de pétrole puis purifié sur phase inverse RP18, éluant MeOH/H₂O 6/4, pour conduire au produit du titre sous la forme d'une poudre blanche.

### EXEMPLE 38 : Acide 2-{4-[2-(6-[(3-chlorophényl)(hydroxyimino)méthyl]-2-oxo-1,3-benzothiazol-3(2H)-yl)éthoxy]phénoxy}-2-méthylpropanoique

A une solution du composé obtenu dans l'Exemple 37 (0,6 g) dans 5 ml d'éthanol 95°, est ajouté l'hydroxyde de potassium (90 mg) dissout dans 1 ml d'éthanol 95°. Le milieu réactionnel est chauffé à reflux durant une nuit. Après refroidissement à température ambiante, la solution est acidifiée par HCl 1N. Le précipité obtenu est alors filtré puis purifié sur phase inverse RP18, éluant MeOH/H₂O 6/4, pour conduire au produit du titre sous la forme d'une poudre blanche.
Point de fusion : dégradation à partir de 170°C.

### EXEMPLE 39 : 5-{4-[2-(6-[(Hydroxyimino)(phényl)méthyl]-2-oxo-1,3-benzothiazol-3(2H)-yl)éthoxy]phénoxy}-2,2-diméthylpentanoate d'éthyle

### Stade A : 5-{4-[2-(6-Benzoyl-2-oxo-1,3-benzothiazol-3(2H)-yl)éthoxy]phénoxy}-2,2-diméthylpentanoate d'éthyle

A une solution de 6-benzoyl-benzothiazolinone (1 g) en présence de K₂CO₃ (1,08 g) dans 10 ml de DMF chauffée à 80°C pendant 2 heures, est ajouté le 5-[4-(2-chloroéthoxy)phénoxy]-2,2-diméthyl pentanoate d'éthyle (1,41 g). Le milieu réactionnel est chauffé à 120°C pendant 5 jours puis hydrolysé par 100 ml d'eau. La solution est extraite par de l'acétate d'éthyle, séchée sur du sulfate de magnésium puis évaporée à sec sous pression réduite. Le résidu est purifié sur gel de silice, éluant AcOEt/EP 2/8, pour conduire au produit du titre sous la forme d'une poudre blanche.
Point de fusion : 82-84°C.

### Stade B : 5-{4-[2-(6-[(Hydroxyimino)(phényl)méthyl]-2-oxo-1,3-benzothiazol-3(2H)-yl)éthoxy]phénoxy}-2,2-diméthylpentanoate d'éthyle

On procède comme dans l'Exemple 37 à partir du composé obtenu au stade A.
Point de fusion : 66-68°C.

### EXEMPLE 40 : {4-[2-(6-[(Hydroxyimino)(phényl)méthyl]-2-oxo-1,3-benzothiazol-3(2H)-yl)éthoxy]phényl}acétonitrile

### Stade A : {4-[2-(6-Benzoyl-2-oxo-1,3-benzothiazol-3(2H)-yl)éthoxy]phényl} acétonitrile

On procède comme dans le stade C de l'Exemple 1 en remplaçant le 3-[4-(2-chloroéthoxy)phényl]-2-éthoxypropanoate de méthyle par le [4-(2-chloroéthoxy) phényl]acétonitrile.
Solide blanc.
Point de fusion : 160-162°C.

### Stade B : {4-[2-(6-[(Hydroxyimino)(phényl)méthyl]-2-oxo-1,3-benzothiazol-3(2H)-yl)éthoxy]phényl}acétonitrile

On procède comme dans l'Exemple 37 à partir du composé obtenu au stade A.

### EXEMPLE 41 : 3-{4-[2-(6-[(Hydroxyimino)(phényl)méthyl]-2-oxo-1,3-benzothiazol-3(2H)-yl)éthoxy]phényl}propanenitrile

### Stade A : 3-{4-[2-(6-Benzoyl-2-oxo-1,3-benzothiazol-3(2H)-yl)éthoxy] phényl}propane nitrile

On procède comme dans le stade C de l'Exemple 1 en remplaçant le 3-[4-(2-chloroéthoxy)phényl]-2-éthoxypropanoate de méthyle par le 3-[4-(2-chloroéthoxy) phényl]propanenitrile.
Solide blanc.
Point de fusion : 106-108°C.

### Stade B : 3-{4-[2-(6-[(Hydroxyimino)(phényl)méthyl]-2-oxo-1,3-benzothiazol-3(2H)-yl)éthoxy]phényl}propanenitrile

On procède comme dans l'Exemple 37 à partir du composé obtenu au stade A.

### EXEMPLE 42 : 6-[(Hydroxyimino)(phényl)méthyl]-3-{2-[4-(1H-tétrazol-5-ylméthyl) phénoxy]éthyl}-1,3-benzothiazol-2(3H)-one

### Stade A : 6-Benzoyl-3-{2-[4-(1H-tétrazol-5-ylméthyl)phénoxy]éthyl}-1,3-benzothiazol-2(3H)-one

On procède comme dans le stade C de l'Exemple 1 en remplaçant le 3-[4-(2-chloroéthoxy)phényl]-2-éthoxypropanoate de méthyle par le 5-[4-(2-chloroéthoxy) benzyl]-1*H*-tétrazole.
Solide blanc.
Point de fusion : 140-142°C.

### Stade B : 6-[(Hydroxyimino)(phényl)méthyl]-3-{2-[4-(1H-tétrazol-5-ylméthyl) phénoxy]éthyl}-1,3-benzothiazol-2(3H)-one

On procède comme dans l'Exemple 37 à partir du composé obtenu au stade A.

### EXEMPLE 43 : 6-[(Hydroxyimino)(phényl)méthyl]-3-(2-{4-[2-(1H-tétrazol-5-yl)éthyl]phénoxy}éthyl)-1,3-benzothiazol-2(3H)-one

### Stade A : 6-Benzoyl-3-(2-{4-[2-(1H-tétrazol-5-yl)éthyl]phénoxy}éthyl)-1,3-benzothiazol-2(3H)-one

On procède comme dans le stade C de l'Exemple 1 en remplaçant le 3-[4-(2-chloroéthoxy)phényl]-2-éthoxypropanoate de méthyle par le 5-{2-[4-(2-chloroéthoxy) phényl]éthyl}-1H-tétrazole.
Solide blanc.
Point de fusion : 120-124°C.

### Stade B : 6-[(Hydroxyimino)(phényl)méthyl]-3-(2-{4-[2-(1H-tétrazol-5-yl)éthyl]phénoxy}éthyl)-1,3-benzothiazol-2(3H)-one

On procède comme dans l'Exemple 37 à partir du composé obtenu au stade A.

### EXEMPLE 44 : 2-{4-[2-(6-[(Hydroxyimino)(phényl)méthyl]-2-oxo-1,3-benzothiazol-3(2H)-yl)éthoxy]phényl}éthylcarbamate de tert-butyle

### Stade A : 2-{4-[2-(6-Benzoyl-2-oxo-1,3-benzothiazol-3(2H)-yl)éthoxy]phényl} éthylcarbamate de tert-butyle

On procède comme dans le stade C de l'Exemple 1 en remplaçant le 3-[4-(2-chloroéthoxy)phényl]-2-éthoxypropanoate de méthyle par le 2-[4-(2-chloroéthoxy) phényl]éthylcarbamate de *tert*-butyle.
Solide blanc.
Point de fusion : 114-116°C.

### Stade B : 2-{4-[2-(6-[(Hydroxyimino)(phényl)méthyl]-2-oxo-1,3-benzothiazol-3(2H)-yl)éthoxy]phényl}éthylcarbamate de tert-butyle

On procède comme dans l'Exemple 37 à partir du composé obtenu au stade A.

### EXEMPLE 45 : N-(2-{4-[2-(6-[(Hydroxyimino)(phényl)méthyl]-2-oxo-1,3-benzothiazol-3(2H)-yl)éthoxy]phényl}éthyl)acétamide

### Stade A : N-(2-{4-[2-(6-Benzoyl-2-oxo-1,3-benzothiazol-3(2H)-yl)éthoxy]phényl} éthyl)acétamide

On procède comme dans le stade C de l'Exemple 1 en remplaçant le 3-[4-(2-chloroéthoxy)phényl]-2-éthoxypropanoate de méthyle par le N-{2-[4-(2-chloroéthoxy) phényl]éthyl}acétamide.
Solide blanc.
Point de fusion : 108-110°C.

### Stade B : N-(2-{4-[2-(6-[(Hydroxyimino)(phényl)méthyl]-2-oxo-1,3-benzothiazol-3(2H)-yl)éthoxy]phényl}éthyl)acétamide

On procède comme dans l'Exemple 37 à partir du composé obtenu au stade A.

### EXEMPLE 46 : 2-{4-[2-(6-[(Méthoxyimino)(phényl)méthyl]-2-oxo-1,3-benzothiazol-3(2H)-yl)éthoxy]phénoxy}propanoate d'éthyle

### Stade A : 2-{4-[2-(6-Benzoyl-2-oxo-1,3-benzothiazol-3(2H)-yl)éthoxy]phénoxy} propanoate d'éthyle

On procède comme dans le stade C de l'Exemple 1 en remplaçant le [(2-chloroéthoxy)phényl]-2-éthoxypropanoate de méthyle par le 2-[4-(2-chloroéthoxy)phénoxy]propanoate d'éthyle.
Solide blanc.
Point de fusion : 100-101°C.

### Stade B : 2-{4-[2-(6-[(Méthoxyimino)(phényl)méthyl]-2-oxo-1,3-benzothiazol-3(2H)-yl)éthoxy]phénoxy}propanoate d'éthyle

On procède comme dans le stade B de l'Exemple 2 à partir du composé obtenu au stade A et en remplaçant l'hydroxylamine par la O-méthylhydroxylamine.

### EXEMPLE 47 : 2-{4-[2-(6-[(Méthoxyimino)(phényl)méthyl]-2-oxo-1,3-benzothiazol-3(2H)-yl)éthoxy]phénoxy}butanoate d'éthyle

### Stade A : 2-{4-[2-(6-Benzoyl-2-oxo-1,3-benzothiazol-3(2H)-yl)éthoxy]phénoxy} butanoate d'éthyle

On procède comme dans le stade C de l'Exemple 1 en remplaçant le [(2-chloroéthoxy) phényl]-2-éthoxypropanoate de méthyle par le 2-[4-(2-chloroéthoxy)phénoxy]butanoate d'éthyle.
Solide blanc.
Point de fusion : 113-114°C.

### Stade B : 2-{4-[2-(6-[(Méthoxyimino)(phényl)méthyl]-2-oxo-1,3-benzothiazol-3(2H)-yl)éthoxy]phénoxy}butanoate d'éthyle

On procède comme dans le stade B de l'Exemple 2 à partir du composé obtenu au stade A et en remplaçant l'hydroxylamine par la O-méthylhydroxylamine.

### EXEMPLE 48 : Acide 2-{4-[2-(6-[(hydroxyimino)(phényl)méthyl]-2-oxo-1,3-benzothiazol-3(2H)-yl)éthoxy]phénoxy}propanoïque

### Stade A : Acide 2-{4-[2-(6-benzoyl-2-oxo-1,3-benzothiazol-3(2H)-yl)éthoxy] phénoxy} propanoique

On procède comme dans l'Exemple 33 à partir du composé obtenu au stade A de l'Exemple 46.
Solide blanc.
Point de fusion : 159-160°C.

### Stade B : Acide 2-{4-[2-(6-[(hydroxyimino)(phényl)méthyl]-2-oxo-1,3-benzothiazol-3(2H)-yl)éthoxy]phénoxy}propanoïque

On procède comme dans le stade B de l'Exemple 2 à partir du composé obtenu au stade A.

### EXEMPLE 49 : 2-{4-[2-(6-[N-Méthoxyéthanimidoyl]-2-oxo-1,3-benzothiazol-3(2H)-yl)éthoxy]phénoxy}-2-méthylpropanoate d'éthyle

### Stade A : 2-{4-[2-(6-Acétyl-2-oxo-1,3-benzothiazol-3(2H)-yl)éthoxy]phénoxy}-2-méthylpropanoate d'éthyle

On procède comme dans le stade C et l'Exemple 1 en remplaçant le [(2-chloroéthoxy)phényl]-2-éthoxypropanoate de méthyle par le 2-[4-(2-chloroéthoxy)phénoxy]-2-méthylpropanoate d'éthyle, et en remplaçant la 6-benzoylbenzothiazolinone par la 6-acétylbenzothiazolinone.
Solide blanc.
Point de fusion : 112-113°C.

### Stade B : 2-{4-[2-(6-[N-Méthoxyéthanimidoyl]-2-oxo-1,3-benzothiazol-3(2H)-yl)éthoxy]phénoxy}-2-méthylpropanoate d'éthyle

On procède comme dans le stade B de l'Exemple 2 à partir du composé obtenu au satde A et en remplaçant l'hydroxylamine par la O-méthylhydroxylamine. Les deux composés (Z) et (E) ne sont pas séparés.
Huile incolore.

### EXEMPLE 50 : Acide 2-{4-[2-(6-[N-méthoxyéthanimidoyl]-2-oxo-1,3-benzothiazol-3(2H)-yl)éthoxy]phénoxy}-2-méthylpropanoïque

### 1 ^{er} Mode Opératoire :

On procède comme dans l'Exemple 33 à partir du composé obtenu dans l'Exemple 49.
Solide blanc.
Point de fusion : 145-148°C.

### 2^{ème} Mode Opératoire :

### Stade A : Acide 2-{4-[2-(6-acétyl-2-oxo-1,3-benzothiazol-3(2H)-yl)éthoxy]phénoxy}-2-méthylpropanoïque

On procède comme dans l'Exemple 33 à partir du composé obtenu dans le stade A de l'Exemple 49.
Point de fusion : 175,5-177,5°C.

### Stade B : Acide 2-{4-[2-(6-[N-méthoxyéthanimidoyl]-2-oxo-1,3-benzothiazol-3(2H)-yl)éthoxy]phénoxy}-2-méthylpropanoïque

On procède comme dans le stade D de l'Exemple 1 à partir du composé obtenu dans le stade A.
Solide blanc.
Point de fusion : 145-148°C.

### EXEMPLE 51 : 2-{4-[2-(6-[N-Hydroxyéthanimidoyl]-2-oxo-1,3-benzothiazol-3(2H)-yl)éthoxy]phénoxy}-2-méthylpropanoate d'éthyle

On procède comme dans le stade B de l'Exemple 2 à partir du composé obtenu au stade A dans l'Exemple 49.

### EXEMPLE 52 : Acide 2-{4-[2-(6-[N-hydroxyéthanimidoyl]-2-oxo-1,3-benzothiazol-3(2H)-yl)éthoxy]phénoxy}-2-méthylpropanoïque

On procède comme dans l'Exemple 33 à partir du composé obtenu dans l'Exemple 51.
Solide blanc.
Point de fusion : 185-187°C.

### EXEMPLE 53 : 3-{4-[2-(6-[Cyclopropyl(hydroxyimino)méthyl]-2-oxo-1,3-benzothiazol-3(2H)-yl)éthoxy]phényl}-2-éthoxypropanoate de méthyle

### Stade A : 3-{4-[2-(6-(Cyclopropylcarbonyl)-2-oxo-1,3-benzothiazol-3(2H)-yl)éthoxy]phényl}-2-éthoxypropanoate de méthyle

On procède comme dans le stade C de l'Exemple 1 en remplaçant la 6-benzoylbenzothiazolinone par la 6-(cyclopropylcarbonyl)benzothiazolinone.
Point de fusion : 97-98°C.

### Stade B : 3-{4-[2-(6-[Cyclopropyl(hydroxyimino)méthyl]-2-oxo-1,3-benzothiazol-3(2H)-yl)éthoxy]phényl}-2-éthoxypropanoate de méthyle

On procède comme dans le stade B de l'Exemple 2 à partir du composé obtenu au stade A.

### EXEMPLE 54 : 3-{4-[2-(6-[(3-Chlorophényl)(méthoxyimino)méthyl]-2-oxo-1,3-benzothiazol-3(2H)-yl)éthoxy]phényl}-2-éthoxypropanoate de méthyle

### Stade A : 3-{4-[2-(6-(3-Chlorobenzoyl)-2-oxo-1,3-benzothiazol-3(2H)-yl)éthoxy]phényl}-2-éthoxypropanoate de méthyle

On procède comme dans le stade C de l'Exemple 1 en remplaçant la 6-benzoylbenzothiazolinone par la 6-(3-chlorobenzoyl)benzothiazolinone.

### Stade B : 3-{4-[2-(6-[(3-Chlorophényl)(méthoxyimino)méthyl]-2-oxo-1,3-benzothiazol-3(2H)-yl)éthoxy]phényl}-2-éthoxypropanoate de méthyle

On procède comme dans le stade B de l'Exemple 2 à partir du composé obtenu au stade A et en remplaçant l'hydroxylamine par la O-méthylhydroxylamine.

### EXEMPLE 55 : Acide 3-{4-[2-(6-[(3-chlorophényl)(méthoxyimino)méthyl]-2-oxo-1,3-benzothiazol-3(2H)-yl)éthoxy]phényl}-2-éthoxypropanoïque

On procède comme dans l'Exemple 33 à partir du composé obtenu dans l'Exemple 53.
Point de fusion : 96-98°C.

### EXEMPLE 56 : 3-{4-[2-(6-[(3-Chlorophényl)(hydroxyimino)méthyl]-2-oxo-1,3-benzothiazol-3(2H)-yl)éthoxy]phényl}-2-éthoxypropanoate de méthyle

On procède comme dans l'Exemple 54 en remplaçant au stade B la O-méthylhydroxylamine par l'hydroxylamine.

### EXEMPLE 57 : 3-{4-[2-(6-[(Tert-butoxyimino)(phényl)méthyl]-2-oxo-1,3-benzothiazol-3(2H)-yl)éthoxy]phényl}-2-éthoxypropanoate de méthyle

On procède comme dans le stade B de l'Exemple 2 à partir du composé obtenu au stade C de l'Exemple 1 en remplaçant l'hydroxylamine par la O-(*tert*-butyl)hydroxylamine.

### EXEMPLE 58 : Acide 3-{4-[2-(6-[(tert-butoxyimino)(phényl)méthyl]-2-oxo-1,3-benzothiazol-3(2H)-yl)éthoxy]phényl}-2-éthoxypropanoïque

On procède comme dans l'Exemple 33 à partir du composé obtenu dans l'Exemple 57.
Point de fusion : 80-81°C.

### EXEMPLE 59 : 3-{4-[2-(6-[[(Benzyloxy)imino](phényl)méthyl]-2-oxo-1,3-benzothiazol-3(2H)-yl)éthoxy]phényl}-2-éthoxypropanoate de méthyle

On procède comme dans le stade B de l'Exemple 2 à partir du composé obtenu au stade C de l'Exemple 1 en remplaçant l'hydroxylamine par la O-benzylhydroxylamine.

### EXEMPLE 60 : Acide 3-{4-[2-(6-[[(benzyloxy)imino](phényl)méthyl]-2-oxo-1,3-benzothiazol-3(2H)-yl)éthoxy]phényl}-2-éthoxypropanoïque

On procède comme dans l'Exemple 33 à partir de composé obtenu dans l'Exemple 59.
Point de fusion : 105-106°C.

### EXEMPLE 61 : Acide 3-{4-[2-(6-[(Z)(3-chlorophényl)(méthoxyimino)méthyl]-2-oxo-1,3-benzothiazol-3(2H)-yl)éthoxy]phényl}-2-éthoxypropanoïque

On procède comme dans l'Exemple 33 à partir de composé obtenu dans l'Exemple 7.
Point de fusion : 96-98°C.

### EXEMPLE 62 : Acide 3-{4-[2-(6-[(méthoxyimino)(phényl)méthyl]-2-oxo-1,3-benzothiazol-3(2H)-yl)éthoxy]phényl}-2-(2,2,2-trifluoroéthoxy) propanoïque

On procède comme dans l'Exemple 33 à partir de composé obtenu dans l'Exemple 3.
Point de fusion : 56-57°C.

### EXEMPLE 62a : Acide 3-{4-[2-(6-[(E)(méthoxyimino)(phényl)méthyl]-2-oxo-1,3-benzothiazol-3(2H)-yl)éthoxy]phényl}-2-(2,2,2-trifluoroéthoxy) propanoïque

Le composé obtenu dans l'Exemple 62 est repris dans de l'éther diéthylique. Le composé du titre est précipité sélectivement et filtré.

Les Exemples 62a(1) et 62a(2) sont obtenus à partir du composé obtenu dans l'Exemple 62a par séparation chirale sur CHIRALPAK AD avec un mélange méthanol/eau/acide trifluoroacétique (1000/5/1) et une détection à 285 nm.

### EXEMPLE 62a(1) : Acide 3-{4-[2-(6-[(E)(méthoxyimino)(phényl)méthyl]-2-oxo-1,3-benzothiazol-3(2H)-yl)éthoxy]phényl}-2-(2,2,2-trifluoroéthoxy) propanoïque, énantiomère 1

Le composé du titre est obtenu avec une pureté optique supérieure à 99%.

### EXEMPLE 62a(2) : Acide 3-{4-[2-(6-[(E)(méthoxyimino)(phényl)méthyl]-2-oxo-1,3-benzothiazol-3(2H)-yl)éthoxy]phényl}-2-(2,2,2-trifluoroéthoxy) propanoïque, énantiomère 2

Le composé du titre est obtenu avec une pureté optique supérieure ou égale à 98%.

### EXEMPLE 62b : Acide 3-{4-[2-(6-[(Z)(méthoxyimino)(phényl)méthyl]-2-oxo-1,3-benzothiazol-3(2H)-yl)éthoxy]phényl}-2-(2,2,2-trifluoroéthoxy) propanoïque

Le composé obtenu dans l'Exemple 62 est repris dans de l'éther diéthylique. Le filtrat est récupéré et évaporé pour conduire au composé du titre.

Les Exemples 62b(1) et 62b(2) sont obtenus à partir du composé obtenu dans l'Exemple 62b par séparation sur colonne chirale.

### EXEMPLE 62b(1) : Acide 3-{4-[2-(6-[(Z)(méthoxyimino)(phényl)méthyl]-2-oxo-1,3-benzothiazol-3(2H)-yl)éthoxy]phényl}-2-(2,2,2-trifluoroéthoxy) propanoïque, énantiomère 1

### EXEMPLE 62b(2) : Acide 3-{4-[2-(6-[(Z)(méthoxyimino)(phényl)méthyl]-2-oxo-1,3-benzothiazol-3(2H)-yl)éthoxy]phényl}-2-(2,2,2-trifluoroéthoxy) propanoïque, énantiomère 2

### EXEMPLE 63 : 2-Isopropoxy-3-{4-[2-(6-[(méthoxyimino)(phényl)méthyl]-2-oxo-1,3-benzothiazol-3(2H)-yl)éthoxy]phényl}propanoate de méthyle

### Stade A : 3-{4-[2-(6-Benzoyl-2-oxo-1,3-benzothiazol-3(2H)-yl)éthoxy]phényl}-2-isopropoxypropanoate de méthyle

On procède comme dans l'Exemple 1 aux stades A, B et C en remplaçant au stade A le 2-éthoxy-2-diéthylphosphonoacétate de méthyle par le 2-isopropoxy-2-diéthylphosphonoacétate de méthyle.

### Stade B : 2-Isopropoxy-3-{4-[2-(6-[(méthoxyimino)(phényl)méthyl]-2-oxo-1,3-benzothiazol-3(2H)-yl)éthoxy]phényl}propanoate de méthyle

On procède comme dans le stade D de l'Exemple 1 à partir du composé obtenu au stade A.

### EXEMPLE 64 : Acide 2-isopropoxy-3-{4-[2-(6-[(méthoxyimino)(phényl)méthyl]-2-oxo-1,3-benzothiazol-3(2H)-yl)éthoxy]phényl}propanoïque

On procède comme dans l'Exemple 33 à partir de composé obtenu dans l'Exemple 63.
Point de fusion : 72-74°C.

### EXEMPLE 65 : 3-{4-[2-(6-[(3-Bromophényl)(méthoxyimino)méthyl]-2-oxo-1,3-benzothiazol-3(2H)-yl)éthoxy]phényl}-2-éthoxypropanoate d'éthyle

### Stade A : 3-{4-[2-(6-(3-Bromobenzoyl)-2-oxo-1,3-benzothiazol-3(2H)-yl)éthoxy]phényl}-2-éthoxypropanoate d'éthyle

On procède comme dans l'Exemple 1 aux stades A, B et C en remplaçant au stade A le 2-éthoxy-2-diéthylphosphonoacétate de méthyle par le 2-éthoxy-2-diéthylphosphonoacétate d'éthyle et en remplaçant au stade C la 6-benzoylbenzothiazolinone par la 6-(3-bromobenzoyl)benzothiazolinone.

### Stade B : 3-{4-[2-(6-[(3-Bromophényl)(méthoxyimino)méthyl]-2-oxo-1,3-benzothiazol-3(2H)-yl)éthoxy]phényl}-2-éthoxypropanoate d'éthyle

On procède comme dans le stade D de l'Exemple 1 à partir du composé obtenu au stade A.
Huile.

### EXEMPLE 66 : 3-{4-[2-(6-[(Z)-(3-Bromophényl)(méthoxyimino)méthyl]-2-oxo-1,3-benzothiazol-3(2H)-yl)éthoxy]phényl}-2-éthoxypropanoate d'éthyle

Le composé obtenu dans l'Exemple 65 est repris dans de l'éther diéthylique et le composé du titre est précipité de façon sélective et filtré pour conduire à l'isomère (*Z*).
Point de fusion : 86-88°C.

### EXEMPLE 67 : 5-{4-[2-(6-[(Méthoxyimino)(phényl)méthyl]-2-oxo-1,3-benzothiazol-3(2H)-yl)éthoxy]phénoxy}-2,2-diméthylpentanoate d'éthyle

On procède comme dans le stade D de l'Exemple 1 à partir du composé obtenu au stade A de l'Exemple 39.
Point de fusion : 82-84°C.

### EXEMPLE 68 : Acide 5-{4-[2-(6-[(méthoxyimino)(phényl)méthyl]-2-oxo-1,3-benzothiazol-3(2H)-yl)éthoxy]phénoxy}-2,2-diméthylpentanoïque

### 1^{er} Mode Opératoire :

On procède comme dans l'Exemple 33 à partir de composé obtenu dans l'Exemple 67.
Point de fusion : 127-129°C.

### 2^{ème} Mode Opératoire :

### Stade A : Acide 5-{4-[2-(6-benzoyl-2-oxo-1,3-benzothiazol-3(2H)-yl)éthoxy]phénoxy}-2,2-diméthylpentanoïque

On procède comme dans l'Exemple 33 à partir du composé obtenu dans le stade A de l'Exemple 39.
Point de fusion : 90-92°C.

### Stade B : Acide 5-{4-[2-(6-[(méthoxyimino)(phényl)méthyl]-2-oxo-1,3-benzothiazol-3(2H)-yl)éthoxy]phénoxy}-2,2-diméthylpentanoïque

On procède comme dans le stade D de l'Exemple 1 à partir du composé obtenu dans le stade A.
Point de fusion : 127-129°C.

### EXEMPLE 69 : Acide 3-{4-[2-(6-[(3-bromophényl)(méthoxyimino)méthyl]-2-oxo-1,3-benzothiazol-3(2H)-yl)éthoxy]phényl}-2-éthoxypropanoïque

On procède comme dans l'Exemple 33 à partir du composé obtenu dans l'Exemple 65.
Point de fusion : 58-60°C.

### EXEMPLE 70 : Acide 3-{4-[2-(6-[(Z)-(3-bromophényl)(méthoxyimino)méthyl]-2-oxo-1,3-benzothiazol-3(2H)-yl)éthoxy]phényl}-2-éthoxypropanoïque

Le composé obtenu dans l'Exemple 69 est repris dans de l'éther diéthylique et le composé du titre est précipité de façon sélective et filtré pour conduire à l'isomère (*Z*).
Point de fusion : 74-76°C.

### EXEMPLE 71 : Acide 5-{4-[2-(6-[(hydroxyimino)(phényl)méthyl]-2-oxo-1,3-benzothiazol-3(2H)-yl)éthoxy]phénoxy}-2,2-diméthylpentanoïque

On procède comme dans l'Exemple 33 à partir de composé obtenu dans l'Exemple 39.
Point de fusion : 148-150°C.

### EXEMPLE 72 : 3-{4-[2-(6-[(2-Bromophényl)(méthoxyimino)méthyl]-2-oxo-1,3-benzothiazol-3(2H)-yl)éthoxy]phényl}-2-éthoxypropanoate d'éthyle

### Stade A : 3-{4-[2-(6-(2-Bromobenzoyl)-2-oxo-1,3-benzothiazol-3(2H)-yl)éthoxy]phényl}-2-éthoxypropanoate d'éthyle

On procède comme dans l'Exemple 1 aux stades A, B et C en remplaçant au stade A le 2-éthoxy-2-diéthylphosphonoacétate de méthyle par le 2-éthoxy-2-diéthylphosphonoacétate d'éthyle et en remplaçant au stade C la 6-benzoylbenzothiazolinone par la 6-(2-bromobenzoyl)benzothiazolinone.
Huile.

### Stade B : 3-{4-[2-(6-[(2-Bromophényl)(méthoxyimino)méthyl]-2-oxo-1,3-benzothiazol-3(2H)-yl)éthoxy]phényl}-2-éthoxypropanoate d'éthyle

On procède comme dans le stade D de l'Exemple 1 à partir du composé obtenu au stade A.
Huile.

### EXEMPLE 73 : Acide 3-{4-[2-(6-[(2-bromophényl)(méthoxyimino)méthyl]-2-oxo-1,3-benzothiazol-3(2H)-yl)éthoxy]phényl}-2-éthoxypropanoïque

### 1^{er} Mode Opératoire :

On procède comme dans l'Exemple 33 à partir du composé obtenu dans l'Exemple 72.

### 2^{ème} Mode Opératoire :

### Stade A : Acide 3-{4-[2-(6-(2-bromobenzoyl)-2-oxo-1,3-benzothiazol-3(2H)-yl)éthoxy]phényl}-2-éthoxypropanoïque

On procède comme dans l'Exemple 33 à partir du composé obtenu dans le stade A de l'Exemple 72.
Point de fusion : 64-66°C.

### Stade B : Acide 3-{4-[2-(6-[(2-bromophényl)(méthoxyimino)méthyl]-2-oxo-1,3-benzothiazol-3(2H)-yl)éthoxy]phényl}-2-éthoxypropanoïque

On procède comme dans le stade D de l'Exemple 1 à partir du composé obtenu dans le stade A.

### EXEMPLE 74 : 2-{4-[3-(6-[(Méthoxyimino)(phényl)méthyl]-2-oxo-1,3-benzothiazol-3(2H)-yl)propoxy]phénoxy}-2-méthylpropanoate de méthyle

### Stade A : 2-{4-[3-(6-Benzoyl-2-oxo-1,3-benzothiazol-3(2H)-yl)propoxy]phénoxy}-2-méthylpropanoate de méthyle

On procède comme dans le stade A de l'Exemple 31 en remplaçant le 2-[4-(2-chloroéthoxy)phénoxy]-2-méthylpropanoate d'éthyle par le 2-[4-(3-chloropropoxy) phénoxy]-2-méthylpropanoate de méthyle.

### Stade B : 2-{4-[3-(6-[(Méthoxyimino)(phényl)méthyl]-2-oxo-1,3-benzothiazol-3(2H)-yl)propoxy]phénoxy}-2-méthylpropanoate de méthyle

On procède comme dans le stade D de l'Exemple 1 à partir du composé obtenu au stade A.

### EXEMPLE 75 : Acide 2-{4-[3-(6-[(méthoxyimino)(phényl)méthyl]-2-oxo-1,3-benzothiazol-3(2H)-yl)propoxy]phénoxy}-2-méthylpropanoïque

### 1^{er} Mode Opératoire :

On procède comme dans l'Exemple 33 à partir du composé obtenu dans l'Exemple 74.

### 2^{ème} Mode Opératoire :

### Stade A : Acide 2-{4-[3-(6-benzoyl-2-oxo-1,3-benzothiazol-3(2H)-yl)propoxy]phénoxy}-2-méthylpropanoïque

On procède comme dans l'Exemple 33 à partir du composé obtenu dans le stade A de l'Exemple 74.
Point de fusion : 121-122°C.

### Stade B : Acide 2-{4-[3-(6-[(méthoxyimino)(phényl)méthyl]-2-oxo-1,3-benzothiazol-3(2H)-yl)propoxy]phénoxy}-2-méthylpropanoïque

On procède comme dans le stade D de l'Exemple 1 à partir du composé obtenu dans le stade A.

### EXEMPLE 76 : 2-{3-[3-(6-[(Méthoxyimino)(phényl)méthyl]-1-oxo-1,3-benzothiazol-3(2H)-yl)propoxy]phénoxy}-2-méthylpropanoate de méthyle

### Stade A : 2-{3-[3-(6-Benzoyl-2-oxo-1,3-benzothiazol-3(2H)-yl)propoxy]phénoxy}-2-méthylpropanoate de méthyle

On procède comme dans le stade A de l'Exemple 31 en remplaçant le 2-[4-(2-chloroéthoxy)phénoxy]-2-méthylpropanoate d'éthyle par le 2-[3-(3-chloropropoxy) phénoxy]-2-méthylpropanoate de méthyle.

### Stade B : 2-{3-[3-(6-[(Méthoxyimino)(phényl)méthyl]-2-oxo-1,3-benzothiazol-3(2H)-yl)propoxy]phénoxy}-2-méthylpropanoate de méthyle

On procède comme dans le stade D de l'Exemple 1 à partir du composé obtenu au stade A.

### EXEMPLE 77 : Acide 2-{3-[3-(6-[(méthoxyimino)(phényl)méthyl]-2-oxo-1,3-benzothiazol-3(2H)-yl)propoxy]phénoxy}-2-méthylpropanoïque

### 1^{er} Mode Opératoire :

On procède comme dans l'Exemple 33 à partir du composé obtenu dans l'Exemple 76.

### 2^{ème} Mode Opératoire :

### Stade A : Acide 2-{3-[3-(6-benzoyl-2-oxo-1,3-benzothiazol-3(2H)-yl)propoxy]phénoxy}-2-méthylpropanoïque

On procède comme dans l'Exemple 33 à partir du composé obtenu dans le stade A de l'Exemple 76.
Point de fusion : 79-80°C.

### Stade B : Acide 2-{3-[3-(6-[(méthoxyimino)(phényl)méthyl]-2-oxo-1,3-benzothiazol-3(2H)-yl)propoxy]phénoxy}-2-méthylpropanoïque

On procède comme dans le stade D de l'Exemple 1 à partir du composé obtenu dans le stade A.

### EXEMPLE 78 : 2-{3-[4-(6-[(Méthoxyimino)(phényl)méthyl]-2-oxo-1,3-benzothiazol-3(2H)-yl)butoxy]phénoxy}-2-méthylpropanoate de méthyle

### Stade A : 2-{3-[4-(6-Benzoyl-2-oxo-1,3-benzothiazol-3(2H)-yl)butoxy]phénoxy}-2-méthylpropanoate de méthyle

On procède comme dans le stade A de l'Exemple 31 en remplaçant le 2-[4-(2-chloroéthoxy)phénoxy]-2-méthylpropanoate d'éthyle par le 2-[3-(4-chlorobutoxy) phénoxy]-2-méthylpropanoate de méthyle.

### Stade B : 2-{3-[4-(6-[(Méthoxyimino)(phényl)méthyl]-2-oxo-1,3-benzothiazol-3(2H)-yl)butoxy]phénoxy}-2-méthylpropanoate de méthyle

On procède comme dans le stade D de l'Exemple 1 à partir du composé obtenu au stade A.

### EXEMPLE 79 : Acide 2-{3-[4-(6-[(méthoxyimino)(phényl)méthyl]-2-oxo-1,3-benzothiazol-3(2H)-yl)butoxy]phénoxy}-2-méthylpropanoïque

### 1^{er} Mode Opératoire :

On procède comme dans l'Exemple 33 à partir du composé obtenu dans l'Exemple 78.

### 2^{ème} Mode Opératoire :

### Stade A : Acide 2-{3-[4-(6-benzoyl-2-oxo-1,3-benzothiazol-3(2H)-yl)butoxy]phénoxy}-2-méthylpropanoïque

On procède comme dans l'Exemple 33 à partir du composé obtenu dans le stade A de l'Exemple 78.
Point de fusion : 70-71°C.

### Stade B : Acide 2-{3-[4-(6-[(méthoxyimino)(phényl)méthyl]-2-oxo-1,3-benzothiazol-3(2H)-yl)butoxy]phénoxy}-2-méthylpropanoïque

On procède comme dans le stade D de l'Exemple 1 à partir du composé obtenu dans le stade A.

### EXEMPLE 80 : 2-{4-[3-(6-[(Méthoxyimino)(phényl)méthyl]-2-oxo-1,3-benzothiazol-3(2H)-yl)propoxy]phénoxy}-2-méthylpropanoate d'éthyle

### Stade A : 2-{4-[3-(6-Benzoyl-2-oxo-1,3-benzothiazol-3(2H)-yl)propoxy]phénoxy}-2-méthylpropanoate d'éthyle

On procède comme dans le stade A de l'Exemple 31 en remplaçant le 2-[4-(2-chloroéthoxy)phénoxy]-2-méthylpropanoate d'éthyle par le 2-[4-(3-chloropropoxy) phénoxy]-2-méthylpropanoate d'éthyle.
Cire.

### Stade B : 2-{4-[3-(6-[(Méthoxyimino)(phényl)méthyl]-2-oxo-1,3-benzothiazol-3(2H)-yl)propoxy]phénoxy}-2-méthylpropanoate d'éthyle

On procède comme dans le stade D de l'Exemple 1 à partir du composé obtenu au stade A.

### EXEMPLE 81 : 2-{3-[4-(6-[(Méthoxyimino)(phényl)méthyl]-2-oxo-1,3-benzothiazol-3(2H)-yl)butoxy]phénoxy}-2-méthylpropanoate d'éthyle

### Stade A : 2-{3-[4-(6-Benzoyl-2-oxo-1,3-benzothiazol-3(2H)-yl)butoxy]phénoxy}-2-méthylpropanoate d'éthyle

On procède comme dans le stade A de l'Exemple 31 en remplaçant le 2-[4-(2-chloroéthoxy)phénoxy]-2-méthylpropanoate d'éthyle par le 2-[3-(4-chlorobutoxy) phénoxy]-2-méthylpropanoate d'éthyle.
Point de fusion : 99-101°C.

### Stade B : 2-{3-[4-(6-[(Méthoxyimino)(phényl)méthyl]-2-oxo-1,3-benzothiazol-3(2H)-yl)butoxy]phénoxy}-2-méthylpropanoate d'éthyle

On procède comme dans le stade D de l'Exemple 1 à partir du composé obtenu au stade A.

### EXEMPLE 82 : 2-{3-[3-(6-[(Méthoxyimino)(phényl)méthyl]-2-oxo-1,3-benzothiazol-3(2H)-yl)propoxy]phénoxy}-2-méthylpropanoate d'éthyle

### Stade A : 2-{3-[3-(6-Benzoyl-2-oxo-1,3-benzothiazol-3(2H)-yl)propoxy]phénoxy}-2-méthylpropanoate d'éthyle

On procède comme dans le stade A de l'Exemple 31 en remplaçant le 2-[4-(2-chloroéthoxy)phénoxy]-2-méthylpropanoate d'éthyle par le 2-[3-(3-chloropropoxy) phénoxy]-2-méthylpropanoate d'éthyle.
Cire.

### Stade B : 2-{3-[3-(6-[(Méthoxyimino)(phényl)méthyl]-2-oxo-1,3-benzothiazol-3(2H)-yl)propoxy]phénoxy}-2-méthylpropanoate d'éthyle

On procède comme dans le stade D de l'Exemple 1 à partir du composé obtenu au stade A.

### EXEMPLE 83 : N-Butyryl-4-[2-(6-[(hydroxyimino)(phényl)méthyl]-2-oxo-1,3-benzothiazol-3(2H)-yl)éthoxy]phénylalanine

### Stade A : 4-[2-(6-Benzoyl-2-oxo-1,3-benzothiazol-3(2H)-yl)éthoxy]-N-butyrylphénylalanine

On procède comme dans l'Exemple 33 à partir du composé obtenu dans le stade A de l'Exemple 16.
Point de fusion : 146-148°C.

### Stade B : N-Butyryl-4-[2-(6-[(hydroxyimino)(phényl)méthyl]-2-oxo-1,3-benzothiazol-3(2H)-yl)éthoxy]phénylalanine

On procède comme dans le stade B de l'Exemple 2 à partir du composé obtenu au stade A.

### EXEMPLE 84 : 2-{4-[2-(6-[(Méthoxyimino)(phényl)méthyl]-2-oxo-1,3-benzothiazol-3(2H)-yl)éthoxy]phénoxy}-2-méthylpropanoate de méthyle

### Stade A : 2-{4-[2-(6-Benzoyl-2-oxo-1,3-benzothiazol-3(2H)-yl)éthoxy]phénoxy}-2-méthylpropanoate de méthyle

On procède comme dans le stade A de l'Exemple 31 en remplaçant 2-[4-(2-chloroéthoxy)phénoxy]-2-méthyl propanoate d'éthyle par le 2-[4-(2-chloroéthoxy) phénoxy]-2-méthyl propanoate de méthyle.
Point de fusion : 97-98°C.

### Stade B : 2-{4-[2-(6-[(Méthoxyimino)(phényl)méthyl]-2-oxo-1,3-benzothiazol-3(2H)-yl)éthoxy]phénoxy}-2-méthylpropanoate de méthyle

On procède comme dans le stade B de l'Exemple 31 à partir du composé obtenu au stade A.

### EXEMPLE 85 : 2-{3-[2-(6-[(Méthoxyimino)(phényl)méthyl]-2-oxo-1,3-benzothiazol-3(2H)-yl)éthoxy]phénoxy}-2-méthylpropanoate d'éthyle

### Stade A : 2-{3-[2-(6-Benzoyl-2-oxo-1,3-benzothiazol-3(2H)-yl)éthoxy]phénoxy}-2-méthylpropanoate d'éthyle

On procède comme dans le stade A de l'Exemple 31 en remplaçant 2-[4-(2-chloroéthoxy)phénoxy]-2-méthyl propanoate d'éthyle par le 2-[3-(2-chloroéthoxy) phénoxy]-2-méthyl propanoate d'éthyle.
Point de fusion : 115-116°C.

### Stade B : 2-{3-[2-(6-[(Méthoxyimino)(phényl)méthyl]-2-oxo-1,3-benzothiazol-3(2H)-yl)éthoxy]phénoxy}-2-méthylpropanoate d'éthyle

On procède comme dans le stade B de l'Exemple 31 à partir du composé obtenu au stade A.

### EXEMPLE 86 : Acide 2-{3-[2-(6-[(méthoxyimino)(phényl)méthyl]-2-oxo-1,3-benzothiazol-3(2H)-yl)éthoxy]phénoxy}-2-methylpropanoïque

### 1^{er} Mode Opératoire :

On procède comme dans l'Exemple 33 à partir du composé obtenu dans l'Exemple 85.

### 2^{ème} Mode Opératoire :

### StadeA : Acide 2-{3-[2-(6-benzoyl-2-oxo-1,3-benzothiazol-3(2H)-yl)éthoxy]phénoxy}-2-méthylpropanoïque

On procède comme dans l'Exemple 33 à partir du composé obtenu dans le stade A de l'Exemple 85.
Point de fusion : 130-131°C.

### Stade B : Acide 2-{3-[2-(6-[(méthoxyimino)(phényl)méthyl]-2-oxo-1,3-benzothiazol-3(2H)-yl)éthoxy]phénoxy}-2-methylpropanoïque

On procède comme dans le stade D de l'Exemple 1 à partir du composé obtenu dans le stade A.

### EXEMPLE 87 : N-Benzoyl-4-[2-(6-[(hydroxyimino)(phényl)méthyl]-2-oxo-1,3-benzothiazol-3(2H)-yl)éthoxy]phénylalanine

### 1^{er} Mode Opératoire :

On procède comme dans l'Exemple 33 à partir du composé obtenu dans l'Exemple 17.

### 2^{ème} Mode Opératoire :

### Stade A : N-Benzoyl-4-[2-(6-benzoyl-2-oxo-1,3-benzothiazol-3(2H)-yl)éthoxy]phénylalanine

On procède comme dans l'Exemple 33 à partir du composé obtenu dans le stade A de l'Exemple 17.
Point de fusion : 108-110°C.

### Stade B : N-Benzoyl-4-[2-(6-[(hydroxyimino)(phényl)méthyl]-2-oxo-1,3-benzothiazol-3(2H)-yl)éthoxy]phénylalanine

On procède comme dans le stade B de l'Exemple 2 à partir du composé obtenu dans le stade A.

### EXEMPLE 88 : N-[(Benzyloxy)carbonyl]-4-[2-(6-[(hydroxyimino)(phényl)méthyl]-2-oxo-1,3-benzothiazel-3(2H)-yl)éthoxy]phénylalanine

### 1^{er} Mode Opératoire :

On procède comme dans l'Exemple 33 à partir du composé obtenu dans l'Exemple 14.

### 2^{ème} Mode Opératoire :

### Stade A : 4-[2-(6-Benzoyl-2-oxo-1,3-benzothiazol-3(2H)-yl)éthoxy]-N-[(benzyloxy)carbonyl]phénylalanine

On procède comme dans l'Exemple 33 à partir du composé obtenu dans le stade A de l'Exemple 14.
Point de fusion : 74-76°C.

### Stade B : N-[(Benzyloxy)carbonyl]-4-[2-(6-[(hydroxyimino)(phényl)méthyl]-2-oxo-1,3-benzothiazol-3(2H)-yl)éthoxy]phénylalanine

On procède comme dans le stade B de l'Exemple 2 à partir du composé obtenu dans le stade A.

### EXEMPLE 89 : 3-{4-[2-(6-[(Méthoxyimino)(phényl)méthyl]-2-oxo-1,3-benzothiazol-3(2H)-yl)éthoxy]phényl}-2-[(trifluoroacétyl)amino]propanoate de méthyle

### Stade A : 3-{4-[2-(6-Benzoyl-2-oxo-1,3-benzothiazol-3(2H)-yl)éthoxy]phényl}-2-[(trifluoroacétyl)amino]propanoate de méthyle

On procède comme dans le stade A de l'Exemple 15 en remplaçant le chlorure d'acétyle par le chlorure de trifluoroacétyle.
Point de fusion : 60-62°C.

### Stade B : 3-{4-[2-(6-[(Méthoxyimino)(phényl)méthyl]-2-oxo-1,3-benzothiazol-3(2H)-yl)éthoxy]phényl}-2-[(trifluoroacétyl)amino]propanoate de méthyle

On procède comme dans le stade B de l'Exemple 2 à partir du composé obtenu dans le stade A.

### ETUDE PHARMACOLOGIQUE

### Exemple A : Etude de la toxicité aiguë

La toxicité aiguë a été appréciée après administration orale à des lots de 8 souris (26 ± 2 g). Les animaux ont été observés à intervalles réguliers au cours de la première journée et quotidiennement pendant deux semaines suivant le traitement. La DL₅₀, entraînant la mort de 50 % des animaux, a été évaluée et a montré la faible toxicité des composés de l'invention.

### Exemple B : Efficacité dans les modèles génétiques

Des mutations chez des animaux de laboratoire ainsi que des sensibilités différentes à des régimes alimentaires ont permis le développement de modèles animaux présentant des diabètes non insulino-dépendants et des hyperlipidémies associés à l'obésité et à la résistance à l'insuline.
Des modèles génétiques souris (ob/ob) (Diabetes, 1982, 31 (1), 1-6) et rats Zucker (fa/fa) ont été développés par différents laboratoires pour comprendre la physiopathologie de ces maladies et tester l'efficacité de nouveaux composés antidiabétiques (Diabetes, 1983, 32, 830-838).

### Effet antidiabétique et hypolipémiant chez la souris ob/ob

La souris femelle ob/ob (Harlan) âgée de 10 semaines est utilisée pour les tests *in vivo.* Ces animaux sont maintenus sous un cycle lumière-obscurité de 12 heures à 25 °C. Cette souris a une hyperglycémie basale située à 2 g/l. Les animaux sont randomisés par rapport à leur glycémie pour former des groupes de six. Les composés testés par voie intrapéritonéale sont dissous dans un mélange de diméthylsulfoxide (10 %) et de solutol (15 %) pour être administrés à 10 mg/kg sous un volume de 2,5 ml/kg, deux fois par jour pendant quatre jours. Par voie *per os*, les composés sont testés à 30 mg/kg administrés sous un volume de 2,5 ml/kg de HEC 1 %, deux fois par jour pendant quatre jours. Les groupes contrôles reçoivent les solvants dans les mêmes conditions que les groupes traités. L'activité des produits est évaluée par une mesure de la glycémie 24 heures après la dernière administration et par la mesure quotidienne du poids corporel.

Les composés de l'invention montrent une très bonne capacité à réduire la glycémie comparable aux effets obtenus avec la Rosiglitazone, substance de référence, mais avec une variation du poids corporel non significative, alors que dans les mêmes conditions, la Rosiglitazone montre une augmentation significative en quatre jours. Par ailleurs, aucun effet secondaire n'a été observé durant les test *in vivo.*

A titre d'exemple, le composé de l'Exemple 3 montre une baisse de 51 % de la glycémie par rapport au groupe contrôle comparable à ce qui est observé avec la Rosiglitazone qui montre dans les mêmes conditions une baisse de 61 % de la glycémie. Par ailleurs, les animaux traités par la Rosiglitazone montrent une augmentation de poids de 33 % par rapport à l'augmentation de poids du groupe contrôle, alors que le composé de l'Exemple 3, dans les mêmes conditions montre une diminution de 80 % de la prise de poids par rapport au groupe contrôle.

### Exemple C : Composition pharmaceutique

| | |
|---|---|
| 1000 comprimés dosés à 5 mg de 3-{4-[2-(6-[(Méthoxyimino)(phényl)méthyl]-2-oxo-1,3-benzothiazol-3(2H)-yl)éthoxy]phényl}-2-(2,2,2-trifluoroéthoxy)propanoate de méthyle (Exemple 3) | 5 g |
| Amidon de blé | 20 g |
| Amidon de maïs | 20 g |
| Lactose | 30 g |
| Stéarate de magnésium | 2 g |
| Silice | 1 g |
| Hydroxypropylcellulose | 2 g |

## Revendications

1. Composés de formule (I) : 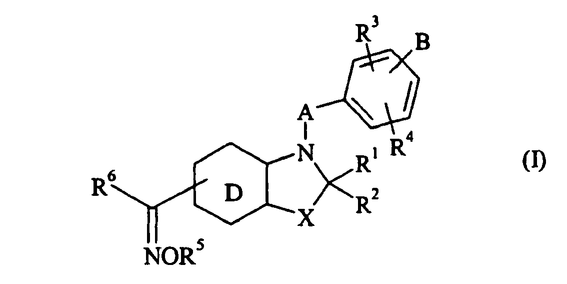 dans laquelle :
• X représente un atome d'oxygène ou de soufre, ou un groupement CH₂ ou 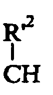 (dans lequel R'² forme avec R² une liaison supplémentaire),
• R¹ et R², identiques ou différents représentent un atome d'hydrogène, un groupement alkyle (C₁-C₆) linéaire ou ramifié, aryle, arylalkyle (C₁-C₆) linéaire ou ramifié, aryloxy, arylalkyloxy (C₁-C₆) linéaire ou ramifié, alkoxy (C₁-C₆) linéaire ou ramifié, hydroxy, amino, alkylamino (C₁-C₆) linéaire ou ramifié ou dialkylamino (C₁-C₆) linéaire ou ramifié,
ou R¹ et R² forment ensemble un groupement oxo, thioxo ou imino,
R² pouvant de plus former avec R'² une liaison supplémentaire,
• A représente une chaîne alkylène (C₁-C₆) dont un groupement CH₂ peut être remplacé par un hétéroatome choisi parmi oxygène ou soufre, ou par un groupement NRₐ (où Rₐ représente un atome d'hydrogène ou un groupement alkyle (C₁-C₆) linéaire ou ramifié), ou par un groupement phénylène ou naphtylène,
• R³ et R⁴, identiques ou différents, représentent un atome d'hydrogène ou d'halogène, ou un groupement R, OR ou NRR' (où R et R', identiques ou différents, représentent un atome d'hydrogène ou un groupement alkyle (C₁-C₆) linéaire ou ramifié, alkényle (C₂-C₆) linéaire ou ramifié, alkynyle (C₂-C₆) linéaire ou ramifié, aryle, arylalkyle (C₁-C₆) linéaire ou ramifié, arylalkényle (C₂-C₆) linéaire ou ramifié, arylalkynyle (C₂-C₆) linéaire ou ramifié, hétéroaryle, hétéroarylalkyle (C₁-C₆) linéaire ou ramifié, hétéroarylalkényle (C₂-C₆) linéaire ou ramifié, hétéroarylalkynyle (C₂-C₆) linéaire ou ramifié, cycloalkyle (C₃-C₈), cycloalkyl(C₃-C₈)alkyle(C₁-C₆) linéaire ou ramifié ou polyhalogénoalkyle (C₁-C₆) linéaire ou ramifié),
ou R³ et R⁴ forment ensemble avec les atomes de carbone qui les portent, lorsqu'ils sont portés par deux atomes de carbone adjacents, un cycle comportant 5 ou 6 chaînons et pouvant contenir un hétéroatome choisi parmi oxygène, soufre et azote,
• R⁵ et R⁶, identiques ou différents, peuvent prendre toutes les valeurs de R tel que défini précédemment,
• D représente :
un noyau benzénique et dans ce cas X ne peut représenter un groupement 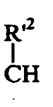 tel que défini précédemment,
ou D représente un noyau pyridinique, pyrazinique, pyrimidinique ou pyridazinique,
• B représente un groupement alkyle (C₁-C₆) linéaire ou ramifié, ou alkényle (C₂-C₆) linéaire ou ramifié, ces groupements étant substitués :
◆ par un groupement de formule (II) : 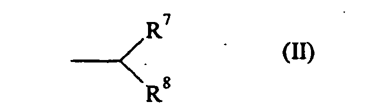 dans laquelle : - R⁷ représente un groupement 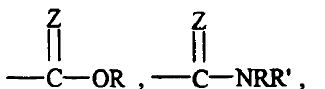 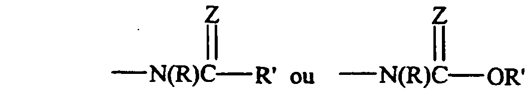 dans lesquels Z représente un atome d'oxygène ou de soufre et R et R', identiques ou différents, sont tels que définis précédemment, - et R⁸ représente un groupement aryle, arylalkyle dont la partie alkyle contient de 1 à 6 atomes de carbone et peut être linéaire ou ramifiée, hétéroaryle, hétéroarylalkyle dont la partie alkyle contient 1 à 6 atomes de carbone et peut être linéaire ou ramifiée, CN, tétrazole, -OR , -NRR', 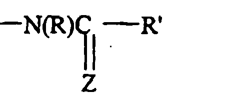 ou 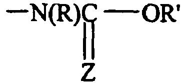 dans lesquels Z est tel que défini précédemment et R et R', identiques ou différents, peuvent prendre les mêmes valeurs que défini précédemment,
◆ ou par un groupement R⁹ où R⁹ représente un groupement CN, tétrazole, 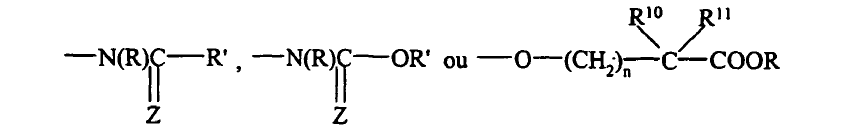 dans lesquels Z est tel que défini précédemment et R et R', identiques ou différents, peuvent prendre les mêmes valeurs que défini précédemment, n représente 0, 1, 2, 3, 4, 5 ou 6, et R¹⁰ et R¹¹, identiques ou différents, représentent un atome d'hydrogène ou un groupement alkyle (C₁-C₆) linéaire ou ramifié étant entendu que R¹⁰ et R¹¹ ne peuvent représenter simultanément un atome d'hydrogène,
ou B représente un groupement de formule (II) ou un groupement R⁹ tels que définis précédemment,
étant entendu que :
* l'oxime R⁶-C(=N-OR⁵)- peut être de configuration Z ou E,
* par aryle on entend un groupement phényle, naphtyle ou biphényle, ces groupements pouvant être partiellement hydrogénés,
* par hétéroaryle on entend tout groupement aromatique mono ou bicyclique contenant 5 à 10 chaînons, pouvant être partiellement hydrogéné sur un des cycles dans le cas des hétéroaryles bicycliques, et contenant 1 à 3 hétéroatomes choisis parmi oxygène, azote et soufre,
les groupements aryle et hétéroaryle ainsi définis pouvant être substitués par 1 à 3 groupements choisis parmi alkyle (C₁-C₆) linéaire ou ramifié, polyhalogénoalkyle (C₁-C₆) linéaire ou ramifié, alkoxy (C₁-C₆) linéaire ou ramifié, hydroxy, carboxy, formyle, NR_{b}R_{c} (dans lequel R_{b} et R_{c}, identiques ou différents, représentent un atome d'hydrogène, un groupement alkyle (C₁-C₆) linéaire ou ramifié, aryle ou hétéroaryle), ester, amido, nitro, cyano, ou atomes d'halogène,
leurs énantiomères et diastéréoisomères ainsi que leurs sels d'addition à un acide ou à une base pharmaceutiquement acceptables.

2. Composés de formule (I) selon la revendication 1 pour lesquels R¹ et R² forment ensemble un groupement oxo, leurs énantiomères et diastéréoisomères ainsi que leurs sels d'addition à un acide ou à une base pharmaceutiquement acceptables.

3. Composés de formule (I) selon la revendication 1 pour lesquels R³ et R⁴ représentent un atome d'hydrogène, leurs énantiomères et diastéréoisomères ainsi. que leurs sels d'addition à un acide ou à une base pharmaceutiquement acceptables.

4. Composés de formule (I) selon la revendication 1 pour lesquels A représente un groupement éthylèneoxy, leurs énantiomères et diastéréoisomères ainsi que leurs sels d'addition à un acide ou à une base pharmaceutiquement acceptables.

5. Composés de formule (I) selon la revendication 1 pour lesquels D représente un noyau benzénique, leurs énantiomères et diastéréoisomères ainsi que leurs sels d'addition à un acide ou à une base pharmaceutiquement acceptables.

6. Composés de formule (I) selon la revendication 1 pour lesquels X représente un atome de soufre, leurs énantiomères et diastéréoisomères ainsi que leurs sels d'addition à un acide ou à une base pharmaceutiquement acceptables.

7. Composés de formule (I) selon la revendication 1 pour lesquels R⁵ représente un atome d'hydrogène, leurs énantiomères et diastéréoisomères ainsi que leurs sels d'addition à un acide ou à une base pharmaceutiquement acceptables.

8. Composés de formule (I) selon la revendication 1 pour lesquels R⁵ représente un groupement alkyle, leurs énantiomères et diastéréoisomères ainsi que leurs sels d'addition à un acide ou à une base pharmaceutiquement acceptables.

9. Composés de formule (I) selon la revendication 1 pour lesquels R⁶ représente un groupement phényle non substitué, leurs énantiomères et diastéréoisomères ainsi que leurs sels d'addition à un acide ou à une base pharmaceutiquement acceptables.

10. Composés de formule (I) selon la revendication 1 pour lesquels R⁶ représente un groupement phényle substitué, leurs énantiomères et diastéréoisomères ainsi que leurs sels d'addition à un acide ou à une base pharmaceutiquement acceptables.

11. Composés de formule (I) selon la revendication 1 pour lesquels R⁶ représente un groupement phényle substitué par un atome d'halogène, leurs énantiomères et diastéréoisomères ainsi que leurs sels d'addition à un acide ou à une base pharmaceutiquement acceptables.

12. Composés de formule (I) selon la revendication 1 pour lesquels B représente un groupement alkyle substitué par un groupement 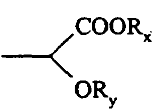 dans lequel Rₓ et R_{y}, identiques ou différents représentent un atome d'hydrogène ou un groupement alkyle, leurs énantiomères et diastéréoisomères ainsi que leurs sels d'addition à un acide ou à une base pharmaceutiquement acceptables.

13. Composés de formule (I) selon la revendication 1 pour lesquels B représente un groupement alkyle substitué par un groupement 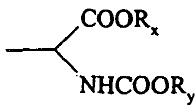 dans lequel Rₓ et R_{y}, identiques ou différents représentent un atome d'hydrogène ou un groupement alkyle, leurs énantiomères et diastéréoisomères ainsi que leurs sels d'addition à un acide ou à une base pharmaceutiquement acceptables.

14. Composés de formule (I) selon la revendication 1 pour lesquels B représente un groupement alkyle substitué par un groupement 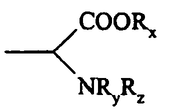 dans lequel Rₓ, R_{y} et R_{z}, identiques ou différents représentent un atome d'hydrogène ou un groupement alkyle, leurs énantiomères et diastéréoisomères ainsi que leurs sels d'addition à un acide ou à une base pharmaceutiquement acceptables.

15. Composés de formule (I) selon la revendication 1 pour lesquels B représente un groupement 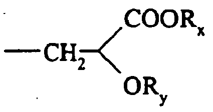 dans lequel Rₓ et R_{y}, identiques ou différents représentent un atome d'hydrogène ou un groupement alkyle, leurs énantiomères et diastéréoisomères ainsi que leurs sels d'addition à un acide ou à une base pharmaceutiquement acceptables.

16. Composés de formule (I) selon la revendication 1 pour lesquels B représente un groupement 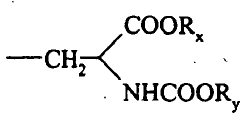 dans lequel Rₓ et R_{y}, identiques ou différents représentent un atome d'hydrogène ou un groupement alkyle, leurs énantiomères et diastéréoisomères ainsi que leurs sels d'addition à un acide ou à une base pharmaceutiquement acceptables.

17. Composés de formule (I) selon la revendication 1 pour lesquels B représente un groupement 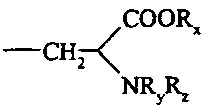 dans lequel Rₓ, R_{y} et R_{z}, identiques ou différents représentent un atome d'hydrogène ou un groupement alkyle, leurs énantiomères et diastéréoisomères ainsi que leurs sels d'addition à un acide ou à une base pharmaceutiquement acceptables.

18. Composés de formule (I) selon la revendication 1 qui sont le 2-éthoxy-3-{4-[2-(6-[(*E*)(méthoxyimino)(phényl)méthyl]-2-oxo-1,3-benzothiazol-3(2H)-yl)éthoxy]phényl} propanoate de méthyle, le 2-éthoxy-3-{4-[2-(6-[(*Z*)(hydroxyimino)(phényl)méthyl]-2-oxo-1,3-benzothiazol-3(2*H*)-yl)éthoxy]phényl}propanoate de méthyle, le 3-{4-[2-(6-[(méthoxyimino)(phényl)méthyl]-2-oxo-1,3-benzothiazol-3(2*H*)-yl)éthoxy]phényl}-2-(2,2,2-trifluoroéthoxy)propanoate de méthyle, le 3-{4-[2-(6-[(hydroxyimino) (phényl)méthyl]-2-oxo-1,3-benzothiazol-3(2*H*)-yl)éthoxy]phényl}-2-(2,2,2-trifluoroéthoxy)propanoate de méthyle, l'acide 2-éthoxy-3-{4-[2-(6-[(méthoxyimino) (phényl)méthyl]-2-oxo-1,3-benzothiazol-3(2*H*)-yl)éthoxy]phényl}propanoïque, l'acide 2-éthoxy-3-{4-[2-(6-[(*E*)(méthoxyimino)(phényl)méthyl]-2-oxo-1,3-benzothiazol-3(2*H*)-yl)éthoxy]phényl}propanoïque, l'acide 2-éthoxy-3-{4-[2-(6-[(*E*)(méthoxy imino)(phényl)méthyl]-2-oxo-1,3-benzothiazol-3(2*H*)-yl)éthoxy]phényl} propanoïque énantiomère 1, l'acide 2-éthoxy-3-{4-[2-(6-[(*E*)(méthoxyimino)(phényl)méthyl]-2-oxo-1,3-benzothiazol-3(2*H*)-yl)éthoxy]phényl} propanoïque énantiomère 2, l'acide 2-éthoxy-3-{4-[2-(6-[(*Z*)(méthoxyimino)(phényl)méthyl]-2-oxo-1,3-benzothiazol-3(2*H*)-yl)éthoxy]phényl}propanoïque, l'acide 2-éthoxy-3-{4-[2-(6-[(*Z*)(méthoxyimino) (phényl)méthyl]-2-oxo-1,3-benzothiazol-3(2*H*)-yl)éthoxy]phényl} propanoïque énantiomère 1, l'acide 2-éthoxy-3-{4-[2-(6-[(*Z*)(méthoxyimino)(phényl)méthyl]-2-oxo-1,3-benzothiazol-3(2*H*)-yl)éthoxy]phényl} propanoïque énantiomère 2, l'acide 2-éthoxy-3-{4-[2-(6-[(hydroxyimino)(phényl)méthyl]-2-oxo-1,3-benzothiazol-3(2*H*)-yl)éthoxy]phényl}propanoïque, le 3-{4-[2-(6-[(*Z*)(3-chlorophényl) (méthoxyimino)méthyl]-2-oxo-1,3-benzothiazol-3(2*H*)-yl)éthoxy]phényl}-2-éthoxypropanoate de méthyle, le 3-{4-[2-(6-[(3-chlorophényl)(hydroxyimino) méthyl]-2-oxo-1,3-benzothiazol-3(2*H*)-yl)éthoxy]phényl}-2-éthoxypropanoate de méthyle, le 2-méthoxy-3-{4-[2-(6-[(méthoxyimino)(phényl)méthyl]-2-oxo-1,3-benzothiazol-3(2*H*)-yl)éthoxy]phényl}propanoate de méthyle, l' acide 3-{4-[2-(6-[(*Z*)(3-chlorophényl)(méthoxyimino)méthyl]-2-oxo-1,3-benzothiazol-3(2*H*)-yl)éthoxy]phényl}-2-éthoxypmpanoïque, l'acide 3-{4-[2-(6-[(méthoxyimino) (phényl)méthyl]-2-oxo-1,3-benzothiazol-3(2*H*)-yl)éthoxy]phényl}-2-(2,2,2-trifluoro éthoxy) propanoïque, l'acide 3-{4-[2-(6-[(*E*)(méthoxyimino)(phényl)méthyl]-2-oxo-1,3-benzothiazol-3(2*H*)-yl)éthoxy]phényl}-2-(2,2,2-triiluoroéthoxy) propanoïque, l'acide 3-{4-[2-(6-[(*E*)(méthoxyimino)(phényl)méthyl]-2-oxo-1,3-benzothiazol-3(2*H*)-yl)éthoxy]phényl}-2-(2,2,2-trifluoroéthoxy) propanoïque énantiomère 1, l'acide 3-{4-[2-(6-[(*E*)(méthoxyimino)(phényl)méthyl]-2-oxo-1,3-benzothiazol-3(2*H*)-yl)éthoxy] phényl}-2-(2,2,2-trifluoroéthoxy) propanoïque énantiomère 2, l'acide 3-{4-[2-(6-[(*Z*)(méthoxyimino)(phényl)méthyl]-2-oxo-1,3-benzothiazol-3(2*H*)-yl)éthoxy] phényl}-2-(2,2,2-trifluoroéthoxy) propanoïque, l'acide 3-{4-[2-(6-[(*Z*)(méthoxyimino) (phényl)méthyl]-2-oxo-1,3-benzothiazol-3(2*H*)-yl)éthoxy]phényl}-2-(2,2,2-trifluoro éthoxy) propanoïque énantiomère 1, l'acide 3-{4-[2-(6-[(*Z*)(méthoxyimino) (phényl)méthyl]-2-oxo-1,3-benzothiazol-3(2*H*)-yl)éthoxy]phényl}-2-(2,2,2-trifluoro éthoxy) propanoïque énantiomère 2, l'acide 3-{4-[2-(6-[(*tert*-butoxyimino)(phényl) méthyl]-2-oxo-1,3-benzothiazol-3(2*H*)-yl)éthoxy]phényl}-2-éthoxypropanoïque.

19. Composés de formule (I) selon la revendication 1 qui sont le 2-[(*tert-*butoxycarbonyl)amino]-3- {4-[2-(6-[(méthoxyimino)(phényl)méthyl]-2-oxo-1,3-benzothiazol-3(2*H*)-yl)éthoxy]phényl}propanoate de méthyle, le 2-[(*tert-*butoxycarbonyl)amino]-3-{4-[2-(6-[(hydroxyimino)(phényl)méthyl] -2-oxo-1,3-benzothiazol-3(2*H*)-yl)éthoxy]phényl}propanoate de méthyle, le 2-[(butoxycarbonyl)amino]-3-{4-[2-(6-[(méthoxyimino)(phényl)méthyl]-2-oxo-1,3-benzothiazol-3(2*H*)-yl)éthoxy]phényl}propanoate de méthyle, le 3-{4-[2-(6-[(méthoxyimino)(phényl)méthyl]-2-oxo-1,3-benzothiazol-3(2*H*)-yl)éthoxy]phényl}-2-[(phénoxycarbonyl)amino]propanoate de méthyle, le 2-{[(benzyloxy) carbonyl]amino}-3-{4-[2-(6-[(hydroxyimino)(phényl) méthyl]-2-oxo-1,3-benzothiazol -3(2*H*)-yl)éthoxy]phényl}propanoate de méthyle, le 2-[(*tert-*butoxycarbonyl)(méthyl)amino]-3-{4-[2-(6-[(méthoxyimino)(phényl)méthyl] -2-oxo-1,3-benzothiazol-3(2*H*)-yl)éthoxy]phényl}propanoate de méthyle, le *N*-(*tert-*butoxycarbonyl)-4-[2-(6-[(hydroxyimino)(phényl)méthyl]-2-oxo-1,3-benzothiazol-3(2*H*)-yl)éthoxy]phénylalanine, le *N*-(*tert*-butoxycarbonyl)-4-[2-(6-[(méthoxyimino) (phényl)méthyl]-2-oxo-1,3-benzothiazol-3(2*H*)-yl)éthoxy]phénylalanine.

20. Composés de formule (I) selon la revendication 1 qui sont le 2-amino-3-{4-[2-(6-[(méthoxyimino)(phényl)méthyl]-2-oxo-1,3-benzothiazol-3(2*H*)-yl)éthoxy]phéyl} propanoate de méthyle, 2-amino-3-{4-[2-(6-[(hydroxyimino)(phényl)méthyl]-2-oxo-1,3-benzothiazol-3(2*H*)-yl)éthoxy]phényl}propanoate de méthyle, 3-{4-[2-(6-[(méthoxyimino)(phényl)méthyl]-2-oxo-1,3-benzothiazol-3(2*H*)-yl)éthoxy]phényl}-2-(méthylamino)propanoate de méthyle.

21. Procédé de préparation des composés de formule (I) selon la revendication 1 **caractérisé en ce que** l'on utilise comme produit de départ un composé de formule (III) : 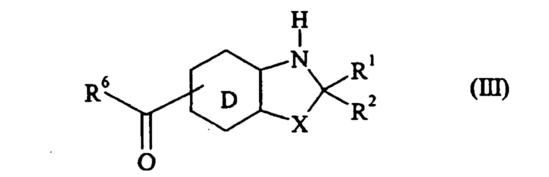 dans laquelle D, R¹, R², R⁶ et X sont tels que définis dans la formule (I),
sur lequel on condense en milieu basique un composé de formule (IV) : 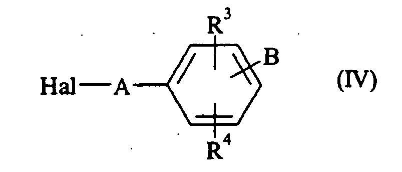 dans laquelle A, B, R³ et R⁴ sont tels que définis dans la formule (I) et
un atome d'halogène, pour conduire au composé de formule (V) : 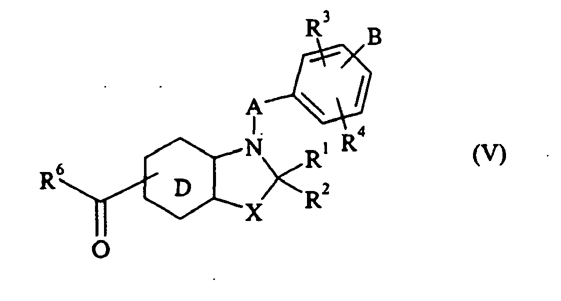 dans laquelle R¹, R², R³, R⁴, R⁶, A, B, D et X sont tels que définis dans la formule (I), que l'on soumet à l'action d'un composé de formule R⁵O-NH₂ dans laquelle R⁵ est tel que défini dans la formule (I) pour conduire au composé de formule (I) : 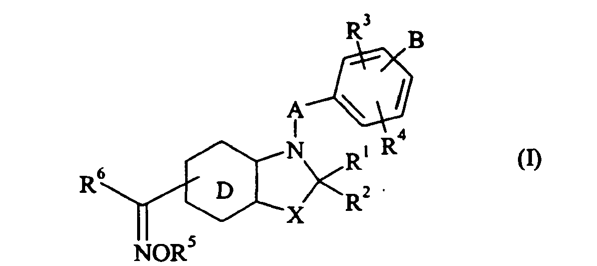 qui peut être purifié selon une technique classique de séparation, que l'on transforme, si on le souhaite en ses sels d'addition à un acide ou à une base pharmaceutiquement acceptable et dont on sépare éventuellement les isomères selon une technique classique de séparation.

22. Procédé de préparation des composés de formule (I) selon la revendication 1 **caractérisé en ce que** l'on utilise comme produit de départ un composé de formule (III): 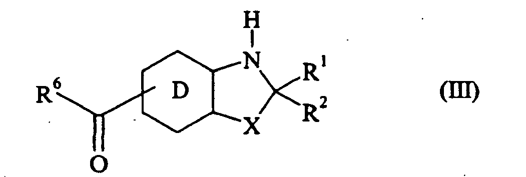 dans laquelle D, R¹, R², R⁶ et X sont tels que définis dans la formule (I),
sur lequel on condense un composé de formule R⁵O-NH₂ dans laquelle R⁵ est tel que défini dans la formule (I) pour conduire au composé de formule (VI) : 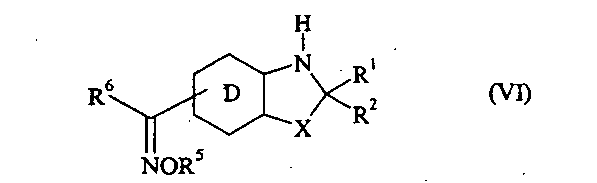 dans laquelle R¹, R², R⁵, R⁶, D et X sont tels que définis dans la formule (I), sur lequel on condense en milieu basique un composé de formule (IV) : 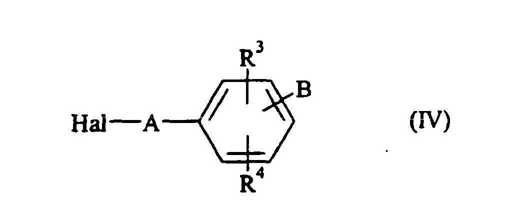 dans laquelle A, B, R³ et R⁴ sont tels que définis dans la formule (I) et Hal représente un atome d'halogène, pour conduire au composé de formule (I) : 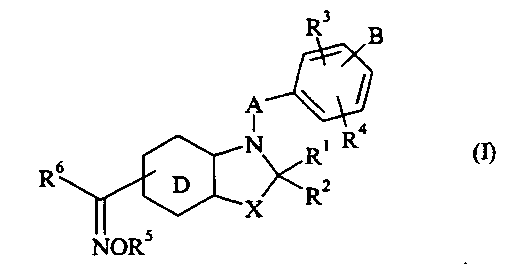 qui peut être purifié selon une technique classique de séparation, que l'on transforme, si on le souhaite en ses sels d'addition à un acide ou à une base pharmaceutiquement acceptable et dont on sépare éventuellement les isomères selon une technique classique de séparation.

23. Composés de formule (V) selon la revendication 21 : 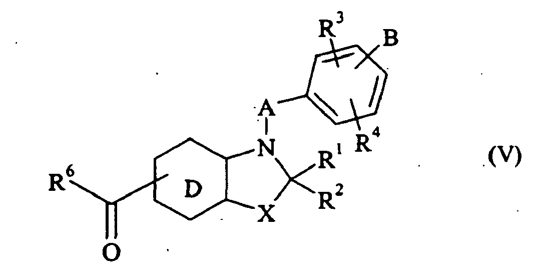 dans lesquels R⁶, B, D, A, X, R¹, R², R³ et R⁴ sont tels que définis dans la revendication 1
utiles en tant qu'intermédiaires de synthèse des composés de formule (I).

24. Compositions pharmaceutiques contenant comme principe actif au moins un composé de formule (I) selon l'une quelconque des revendications 1 à 20 ou 23 ou un de ses sels d'addition à un acide ou à une base pharmaceutiquement acceptables, seul ou en combinaison avec un ou plusieurs excipients pharmaceutiquement acceptables.

25. Compositions pharmaceutiques selon la revendication 24 utiles pour la fabrication d'un médicament pour le traitement et/ou la prophylaxie des hyperglycémies, des dyslipidémies, et plus particulièrement dans le traitement des diabètes non insulino-dépendants de type II, de la résistance à l'insuline, de l'intolérance au glucose, des désordres reliés au syndrome X, des maladies artérielles coronaires et d'autres maladies cardiovasculaires, des maladies rénales, des rétinopathies, des désordres reliés à l'activation des cellules endothéliales, du psoriasis, du syndrome polycystique ovarien, de la démence, de l'ostéoporose, des maladies inflammatoires intestinales, des dystrophies myotoniques, des pancréatites, de l'artériosclérose, du xanthome, mais également dans le traitement ou la prévention du diabète de type I, de l'obésité, de la régulation de l'appétit, de l'anorexie, de la boulimie, de l'anorexie nerveuse, ainsi que des pathologies cancéreuses et notamment les cancers hormono-dépendants tels que le cancer du sein et le cancer du colon, et en tant qu'inhibiteurs d'angiogénèse.

## Claims

1. Compounds of formula (I) : 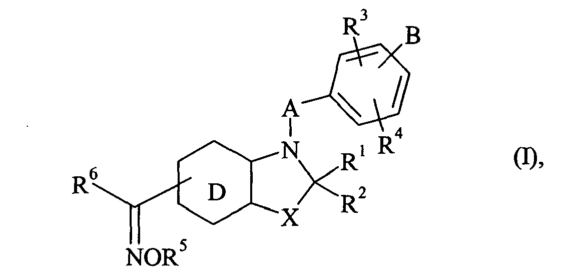 wherein :
• X represents an oxygen or sulphur atom, or a group 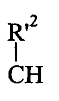 (wherein R'² together with R² forms an additional bond),
• R¹ and R², which may be the same or different, each represent a hydrogen atom, a linear or branched (C₁-C₆)alkyl group, an aryl group, an aryl-(C₁-C₆)alkyl group in which the alkyl moiety is linear or branched, an aryloxy group, an aryl-(C₁-C₆)alkyloxy group in which the alkyl moiety is linear or branched, a linear or branched (C₁-C₆)alkoxy group, a hydroxy group, an amino group, a linear or branched (C₁-C₆)alkylamino group or a di-(C₁-C₆)alkylamino group in which the alkyl moieties are linear or branched,
or R¹ and R² together form an oxo, thioxo or imino group,
it also being possible for R² together with R'² to form an additional bond,
• A represents a (C₁-C₆)alkylene chain in which a CH₂ group may be replaced by a hetero atom selected from oxygen and sulphur or by a group NRₐ (wherein Rₐ represents a hydrogen atom or a linear or branched (C₁-C₆)alkyl group), or by a phenylene or naphthylene group,
• R³ and R⁴, which may be the same or different, each represent a hydrogen or halogen atom or a group R, OR or NRR' (wherein R and R', which may be the same or different, each represent a hydrogen atom or a linear or branched (C₁-C₆)alkyl group, a linear or branched (C₂-C₆)alkenyl group, a linear or branched (C₂-C₆)alkynyl group, an aryl group, an aryl-(C₁-C₆)alkyl group in which the alkyl moiety is linear or branched, an aryl-(C₂-C₆)alkenyl group in which the alkenyl moiety is linear or branched, an aryl-(C₂-C₆)alkynyl group in which the alkynyl moiety is linear or branched, a heteroaryl group, a heteroaryl-(C₁-C₆)alkyl group in which the alkyl moiety is linear or branched, a heteroaryl-(C₂-C₆)alkenyl group in which the alkenyl moiety is linear or branched, a heteroaryl-(C₂-C₆)alkynyl group in which the alkynyl moiety is linear or branched, a (C₃-C₈)cycloalkyl group, a (C₃-C₈)cycloalkyl-(C₁-C₆)alkyl group in which the alkyl moiety is linear or branched, or a linear or branched (C₁-C₆)polyhaloalkyl group),
or R³ and R⁴, together with the carbon atoms carrying them, when they are carried by two adjacent carbon atoms, form a ring that has 5 or 6 ring members and that may contain a hetero atom selected from oxygen, sulphur and nitrogen,
• R⁵ and R⁶, which may be the same or different, may take any of the meanings of R as defined hereinbefore,
• D represents :
a benzene nucleus, in which case X cannot represent a group 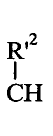 as defined hereinbefore,
or D represents a pyridine, pyrazine, pyrimidine or pyridazine nucleus,
• B represents a linear or branched (C₁-C₆)alkyl group or a linear or branched (C₂-C₆)-alkenyl group, those groups being substituted :
◆ by a group of formula (II) : 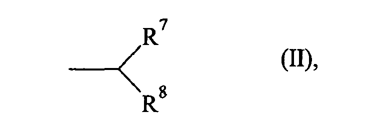 wherein : - R⁷ represents a group 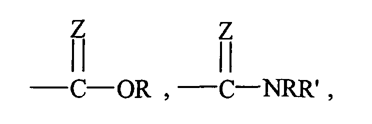 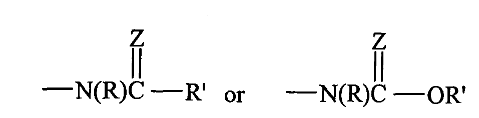 , wherein Z represents an oxygen or sulphur atom, and R and R', which may be the same or different, are as defined hereinbefore,
- and R⁸ represents an aryl group, an arylalkyl group wherein the alkyl moiety contains from 1 to 6 carbon atoms and may be linear or branched, a heteroaryl group, a heteroarylalkyl group wherein the alkyl moiety contains from 1 to 6 carbon atoms and may be linear or branched, CN, tetrazole, -OR, -NRR', -N(R)C-R' or -N(R)C -OR', wherein Z is as defined hereinbefore
and R and R', which may be the same or different, may take the same meanings as defined hereinbefore,
◆ or by a group R⁹, wherein R⁹ represents a CN, tetrazole, 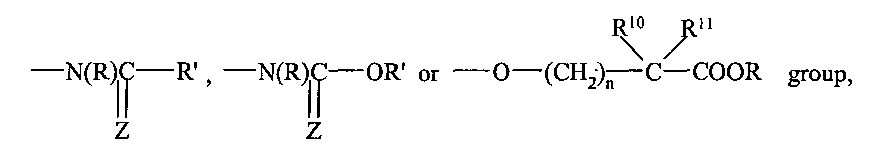 wherein Z is as defined hereinbefore and R and R', which may be the same or different, may take the same meanings as defined hereinbefore, n represents 0, 1, 2, 3, 4, 5 or 6, and R¹⁰ and R¹¹, which may be the same or different, each represent a hydrogen atom or a linear or branched (C₁-C₆)alkyl group, it being understood that R¹⁰ and R¹¹ cannot simultaneously represent a hydrogen atom,
or B represents a group of formula (II) or a group R⁹ as defined hereinbefore,
it being understood that :
* the oxime R⁶-C(=N-OR⁵)- can be of Z or E configuration,
* aryl means a phenyl, naphthyl or biphenyl group, it being possible for those groups to be partially hydrogenated,
* heteroaryl means any mono- or bi-cyclic aromatic group containing 5 to 10 members, which may be partially hydrogenated in one of the rings in the case of bicyclic heteroaryls and which contains 1 to 3 hetero atoms selected from oxygen, nitrogen and sulphur,
it being possible for the aryl and heteroaryl groups thereby defined to be substituted by from 1 to 3 groups selected from linear or branched (C₁-C₆)alkyl, linear or branched (C₁-C₆)polyhaloalkyl, linear or branched (C₁-C₆)alkoxy, hydroxy, carboxy, formyl, NR_{b}R_{c} (wherein R_{b} and R_{c}, which may be the same or different, each represent a hydrogen atom, a linear or branched (C₁-C₆)alkyl group, an aryl group or a heteroaryl group), ester, amido, nitro, cyano, and halogen atoms,
their enantiomers and diastereoisomers, and also addition salts thereof with a pharmaceutically acceptable acid or base.

2. Compounds of formula (I) according to claim 1, wherein R¹ and R² together form an oxo group, their enantiomers and diastereoisomers, and also addition salts thereof with a pharmaceutically acceptable acid or base.

3. Compounds of formula (I) according to claim 1, wherein R³ and R⁴ represent a hydrogen atom, their enantiomers and diastereoisomers, and also addition salts thereof with a pharmaceutically acceptable acid or base.

4. Compounds of formula (I) according to claim 1, wherein A represents an ethyleneoxy group, their enantiomers and diastereoisomers, and also addition salts thereof with a pharmaceutically acceptable acid or base.

5. Compounds of formula (I) according to claim 1, wherein D represents a benzene nucleus, their enantiomers and diastereoisomers, and also addition salts thereof with a pharmaceutically acceptable acid or base.

6. Compounds of formula (I) according to claim 1, wherein X represents a sulphur atom, their enantiomers and diastereoisomers, and also addition salts thereof with a pharmaceutically acceptable acid or base.

7. Compounds of formula (I) according to claim 1, wherein R⁵ represents a hydrogen atom, their enantiomers and diastereoisomers, and also addition salts thereof with a pharmaceutically acceptable acid or base.

8. Compounds of formula (I) according to claim 1, wherein R⁵ represents an alkyl group, their enantiomers and diastereoisomers, and also addition salts thereof with a pharmaceutically acceptable acid or base.

9. Compounds of formula (I) according to claim 1, wherein R⁶ represents an unsubstituted phenyl group, their enantiomers and diastereoisomers, and also addition salts thereof with a pharmaceutically acceptable acid or base.

10. Compounds of formula (I) according to claim 1, wherein R⁶ represents a substituted phenyl group, their enantiomers and diastereoisomers, and also addition salts thereof with a pharmaceutically acceptable acid or base.

11. Compounds of formula (I) according to claim 1, wherein R⁶ represents a phenyl group substituted by a halogen atom, their enantiomers and diastereoisomers, and also addition salts thereof with a pharmaceutically acceptable acid or base.

12. Compounds of formula (I) according to claim 1, wherein B represents an alkyl group substituted by a group 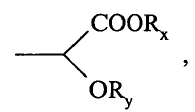 wherein Rₓ and R_{y}, which may be the same or different, represent a hydrogen atom or an alkyl group, their enantiomers and diastereoisomers, and also addition salts thereof with a pharmaceutically acceptable acid or base.

13. Compounds of formula (I) according to claim 1, wherein B represents an alkyl group substituted by a group 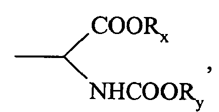 wherein Rₓ and R_{y}, which may be the same or different, represent a hydrogen atom or an alkyl group, their enantiomers and diastereoisomers, and also addition salts thereof with a pharmaceutically acceptable acid or base.

14. Compounds of formula (I) according to claim 1, wherein B represents an alkyl group substituted by a group 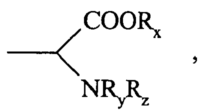 wherein Rₓ, R_{y} and R_{z}, which may be the same or different, represent a hydrogen atom or an alkyl group, their enantiomers and diastereoisomers, and also addition salts thereof with a pharmaceutically acceptable acid or base.

15. Compounds of formula (I) according to claim 1, wherein B represents a group 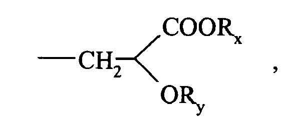 wherein Rₓ and R_{y}, which may be the same or different, represent a hydrogen atom or an alkyl group, their enantiomers and diastereoisomers, and also addition salts thereof with a pharmaceutically acceptable acid or base.

16. Compounds of formula (I) according to claim 1, wherein B represents a group 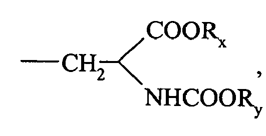 wherein Rₓ and R_{y}, which may be the same or different, represent a hydrogen atom or an alkyl group, their enantiomers and diastereoisomers, and also addition salts thereof with a pharmaceutically acceptable acid or base.

17. Compounds of formula (I) according to claim 1, wherein B represents a group 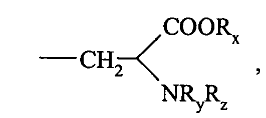 wherein Rₓ, R_{y} and R_{z}, which may be the same or different, represent a hydrogen atom or an alkyl group, their enantiomers and diastereoisomers, and also addition salts thereof with a pharmaceutically acceptable acid or base.

18. Compounds of formula (I) according to claim 1, which are methyl 2-ethoxy-3-{4-[2-(6-[(*E*)-(methoxyimino)(phenyl)methyl]-2-oxo-1,3-benzothiazol-3(2H)-yl)ethoxy]-phenyl}propanoate, methyl 2-ethoxy-3-{4-[2-(6-[(*Z*)-(hydroxyimino)(phenyl)methyl]-2-oxo-1,3-benzothiazol-3(2*H*)-yl)ethoxy]phenyl}propanoate, methyl 3-{4-[2-(6-[(methoxyimino)(phenyl)methyl]-2-oxo-1,3-benzothiazol-3(2*H*)-yl)ethoxy]phenyl}-2-(2,2,2-trifluoroethoxy)propanoate, methyl 3- {4-[2-(6-[(hydroxyimino)(phenyl)-methyl]-2-oxo-1,3-benzothiazol-3(2*H*)-yl)ethoxy]phenyl}-2-(2,2,2-trifluoroethoxy)-propanoate, 2-ethoxy-3-{4-[2-(6-[(methoxyimino)(phenyl)methyl]-2-oxo-1,3-benzothiazol-3(2*H*)-yl)ethoxy]phenyl}propanoic acid, 2-ethoxy-3-{4-[2-(6-[(*E*)-(methoxyimino)(phenyl)methyl]-2-oxo-1,3-benzothiazol-3(2*H*)-yl)ethoxy]phenyl}propanoic acid, 2-ethoxy-3-{4-[2-(6-[(*E*)-(methoxyimino)(phenyl)methyl]-2-oxo-1,3-benzothiazol-3(2*H*)-yl)ethoxy]phenyl}propanoic acid enantiomer 1, 2-ethoxy-3-{4-[2-(6-[(*E*)-(methoxyimino)(phenyl)methyl]-2-oxo-1,3-benzothiazol-3(2*H*)-yl)ethoxy]-phenyl}propanoic acid enantiomer 2, 2-ethoxy-3-{4-[2-(6-[(*Z*)-(methoxyimino)-(phenyl)methyl]-2-oxo-1,3-benzothiazol-3(2*H*)-yl)ethoxy]phenyl}propanoic acid, 2-ethoxy-3-{4-[2-(6-[(*Z*)-(methoxyimino)(phenyl)methyl]-2-oxo-1,3-benzothiazol-3(2*H*)-yl)ethoxy]phenyl}propanoic acid enantiomer 1, 2-ethoxy-3-{4-[2-(6-[(*Z*)-(methoxyimino)(phenyl)methyl]-2-oxo-1,3-benzothiazol-3(2*H*)-yl)ethoxy]phenyl}propanoic acid enantiomer 2, 2-ethoxy-3-{4-[2-(6-[(hydroxyimino)(phenyl)methyl]-2-oxo-1,3-benzothiazol-3(2*H*)-yl)ethoxy]phenyl}propanoic acid, methyl 3-{4-[2-(6-[(*Z*)-(3-chlorophenyl)(methoxyimino)methyl]-2-oxo-1,3-benzothiazol-3(2*H*)-yl)ethoxy]-phenyl}-2-ethoxypropanoate, methyl 3-{4-[2-(6-[(3-chlorophenyl)(hydroxyimino)-methyl]-2-oxo-1,3-benzothiazol-3(2*H*)-yl)ethoxy]phenyl}-2-ethoxypropanoate, methyl 2-methoxy-3-{4-[2-(6-[(methoxyimino)(phenyl)methyl]-2-oxo-1,3-benzothiazol-3(2*H*)-yl)ethoxy]phenyl}propanoate, 3-{4-[2-(6-[(*Z*)-(3-chlorophenyl)(methoxyimino)methyl]-2-oxo-1,3-benzothiazol-3(2*H*)-yl)ethoxy]phenyl}-2-ethoxypropanoic acid, 3-{4-[2-(6-[(methoxyimino)(phenyl)methyl]-2-oxo-1,3-benzothiazol-3(2*H*)-yl)-ethoxy]phenyl}-2-(2,2,2-trifluoroethoxy)propanoic acid, 3-{4-[2-(6-[(*E*)-(methoxyimino)(phenyl)methyl]-2-oxo-1,3-benzothiazol-3(2*H*)-yl)ethoxy]phenyl}-2-(2,2,2-trifluoroethoxy)propanoic acid, 3-{4-[2-(6-[(*E*)-(methoxyimino)(phenyl)methyl]-2-oxo-1,3-benzothiazol-3(2*H*)-yl)ethoxy]phenyl}-2-(2,2,2-trifluoroethoxy)propanoic acid enantiomer 1, 3-{4-[2-(6-[(*E*)-(methoxyimino)(phenyl)methyl]-2-oxo-1,3-benzothiazol-3(2*H*)-yl)ethoxy]phenyl}-2-(2,2,2-trifluoroethoxy) propanoic acid enantiomer 2, 3-{4-[2-(6-[(*Z*)-(methoxyimino)(phenyl)methyl]-2-oxo-1,3-benzothiazol-3(2*H*)-yl)ethoxy]phenyl}-2-(2,2,2-trifluoroethoxy)propanoic acid, 3-{4-[2-(6-[(*Z*)-(methoxyimino)(phenyl)methyl]-2-oxo-1,3-benzothiazol-3(2*H*)-yl)ethoxy]-phenyl}-2-(2,2,2-trifluoroethoxy)propanoic acid enantiomer 1, 3-{4-[2-(6-[(*Z*)-(methoxyimino)(phenyl)methyl]-2-oxo-1,3-benzothiazol-3(2*H*)-yl)ethoxy]phenyl}-2-(2,2,2-trifluoroethoxy)propanoic acid enantiomer 2, 3-{4-[2-(6-[(*tert*-butoxyimino)-(phenyl)methyl]-2-oxo-1,3-benzothiazol-3(2*H*)-yl)ethoxy]phenyl}-2-ethoxypropanoic acid.

19. Compounds of formula (I) according to claim 1, which are methyl 2-[(*tert-*butoxycarbonyl)amino]-3-{4-[2-(6-[(methoxyimino)(phenyl)methyl]-2-oxo-1,3-benzothiazol-3(2*H*)-yl)ethoxy]phenyl}propanoate, methyl 2-[(*tert*-butoxycarbonyl)amino]-3-{4-[2-(6-[(hydroxyimino)(phenyl)methyl]-2-oxo-1,3-benzothiazol-3(2*H*)-yl)-ethoxy]phenyl}propanoate, methyl 2-[(butoxycarbonyl)amino]-3-{4-[2-(6-[(methoxyimino)(phenyl)methyl]-2-oxo-1,3-benzothiazol-3(2*H*)-yl)ethoxy]phenyl}propanoate, methyl 3-{4-[2-(6-[(methoxyimino)(phenyl)methyl]-2-oxo-1,3-benzothiazol-3(2*H*)-yl)ethoxy]phenyl}-2-[(phenoxycarbonyl)amino]propanoate, methyl 2-{[(benzyloxy)-carbonyl]amino}-3-{4-[2-(6-[(hydroxyimino)(phenyl)methyl]-2-oxo-1,3-benzothiazol-3(2*H*)-yl)ethoxy]phenyl}propanoate, methyl 2-[(*tert*-butoxycarbonyl)(methyl)amino]-3-{4-[2-(6-[(methoxyimino)(phenyl)methyl]-2-oxo-1,3-benzothiazol-3(2*H*)-yl)-ethoxy]phenyl}propanoate, *N*-(*tert*-butoxycarbonyl)-4-[2-(6-[(hydroxyimino)(phenyl)-methyl]-2-oxo-1,3-benzothiazol-3(2*H*)-yl)ethoxy]phenylalanine, *N*-(*tert*-butoxycarbonyl)-4-[2-(6-[(methoxyimino)(phenyl)methyl]-2-oxo-1,3-benzothiazol-3(2*H*)-yl)ethoxy]phenylalanine.

20. Compounds of formula (I) according to claim 1, which are methyl 2-amino-3-{4-[2-(6-[(methoxyimino)(phenyl)methyl]-2-oxo-1,3-benzothiazol-3(2*H*)-yl)ethoxy]-phenyl}propanoate, methyl 2-amino-3-(4-[2-(6-[(hydroxyimino)(phenyl)methyl]-2-oxo-1,3-benzothiazol-3(2*H*)-yl)ethoxy]phenyl)propanoate, methyl 3-{4-[2-(6-[(methoxyimino)(phenyl)methyl]-2-oxo-1,3-benzothiazol-3(2*H*)-yl)ethoxy]phenyl}-2-(methylamino)propanoate.

21. Process for the preparation of compounds of formula (I) according to claim 1, **characterised in that** there is used as starting material a compound of formula (III): 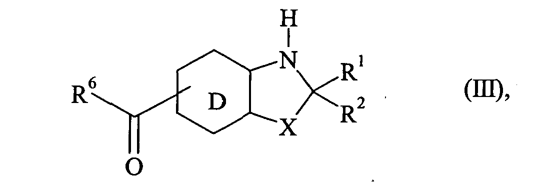 wherein D, R¹, R², R⁶ and X are as defined for formula (I),
with which there is condensed, in a basic medium, a compound of formula (IV) : 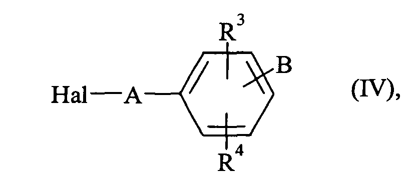 wherein A, B, R³ and R⁴ are as defined for formula (I) and Hal represents a halogen atom, to yield the compound of formula (V) : 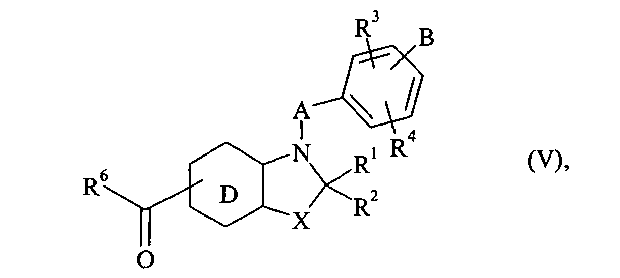 wherein R¹, R², R³, R⁴, R⁶, A, B, D and X are as defined for formula (I), which is subjected to the action of a compound of formula R⁵O-NH₂, wherein R⁵ is as defined for formula (I), to yield the compound of formula (I) : 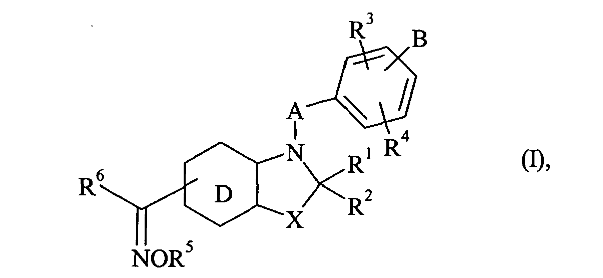 which may be purified according to a conventional separation technique, which is converted, if desired, into its addition salts with a pharmaceutically acceptable acid or base and which is separated, where applicable, into its isomers according to a conventional separation technique.

22. Process for the preparation of compounds of formula (I) according to claim 1, **characterised in that** there is used as starting material a compound of formula (III) : 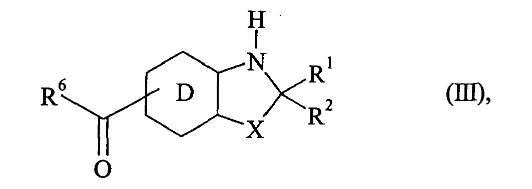 wherein D, R¹, R², R⁶ and X are as defined for formula (I),
with which there is condensed a compound of formula R⁵O-NH₂, wherein R⁵ is as defined for formula (I), to yield the compound of formula (VI) : 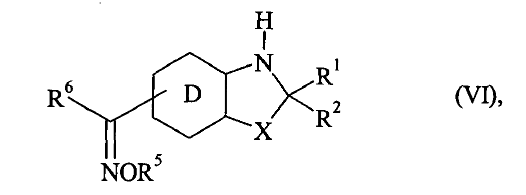 wherein R¹, R², R⁵, R⁶, D and X are as defined for formula (I),
with which there is condensed, in a basic medium, a compound of formula (IV): 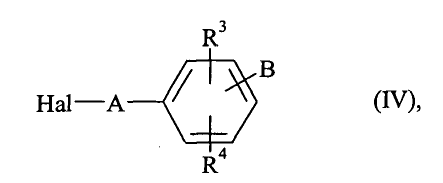 wherein A, B, R³ and R⁴ are as defined for formula (I) and Hal represents a halogen atom, to yield the compound of formula (I) : 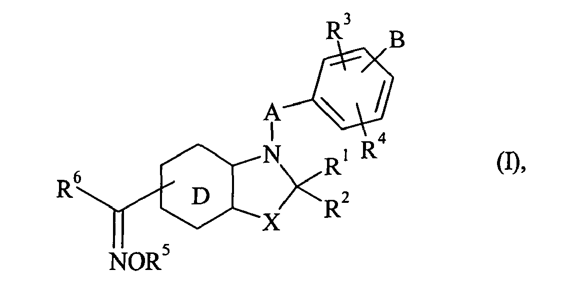 which may be purified according to a conventional separation technique, which is converted, if desired, into its addition salts with a pharmaceutically acceptable acid or base and which is separated, where applicable, into its isomers according to a conventional separation technique.

23. Compounds of formula (V) according to claim 21 : 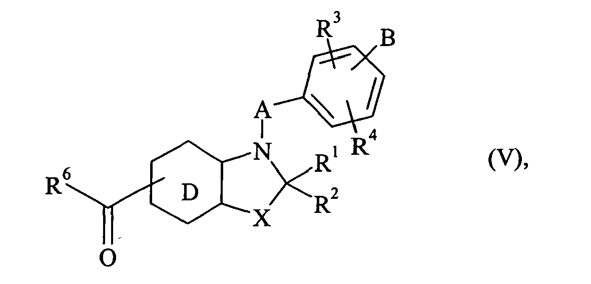 wherein R⁶, B, D, A, X, R¹, R², R³ and R⁴ are as defined in claim 1, for use as intemediates for the synthesis of compounds of formula (I).

24. Pharmaceutical compositions comprising as active ingredient at least one compound of formula (I) according to any one of claims 1 to 20 or 23 or an addition salt thereof with a pharmaceutically acceptable acid or a base, alone or in combination with one or more pharmaceutically acceptable excipients.

25. Pharmaceutical compositions according to claim 24 for use in the preparation of a medicament for the treatment and/or prophylaxis of hyperglycaemia, dyslipidaemia and, more especially, in the treatment of non-insulin dependent type II diabetes, insulin resistance, glucose intolerance, disorders associated with syndrome X, coronary artery disease and other cardiovascular diseases, renal disorders, retinopathy, disorders associated with the activation of endothelial cells, psoriasis, polycystic ovary syndrome, dementia, osteoporosis, intestinal inflammatory disorders, myotonic dystrophy, pancreatitis, arteriosclerosis, xanthoma, but also in the treatment or prevention of type I diabetes, obesity, regulation of appetite, anorexia, bulimia, anorexia nervosa, as well as cancer pathologies and especially hormone-dependent cancers, such as breast cancer or colon cancer, and as angiogenesis inhibitors.

## Patentansprüche

1. Verbindungen der Formel (I): 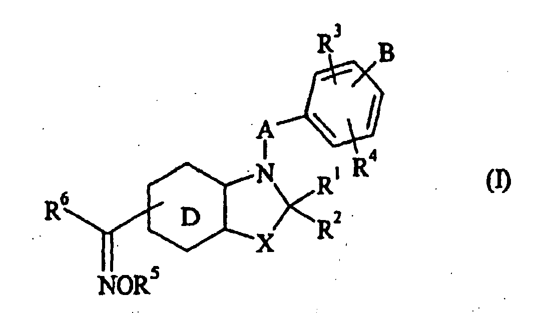 in der:
• X ein Sauerstoff- oder Schwefelatom, eine Gruppe CH₂ oder 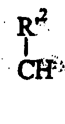 (worin R'² mit R² eine zusätzliche Bindung bildet) bedeutet,
• R¹ und R², die gleichartig oder verschieden sind, ein Wasserstoffatom, eine geradkettige oder verzweigte (C₁-C₆)-Alkylgruppe, Arylgruppe, geradkettige oder verzweigte Aryl-(C₁-C₆)-alkylgruppe, Aryloxygruppe, geradkettige oder verzweigte Aryl-(C₁-C₆)-alkyloxygruppe, geradkettige oder verzweigte (C₁-C₆)-Alkoxygruppe, Hydroxygruppe, Aminogruppe, geradkettige oder verzweigte (C₁-C₆)-Alkylaminogruppe oder geradkettige oder verzweigte Di-(C₁-C₆)-alkylaminogruppe bedeuten,
oder R¹ und R² gemeinsam eine Oxo-, Thioxo- oder Imino-gruppe bilden,
wobei R² weiterhin mit R'² eine zusätzliche Bindung bilden kann,
• A eine (C₁-C₆)-Alkylenkette bedeutet, bei der eine Gruppe CH₂ durch ein Heteroatom ausgewählt aus Sauerstoff oder Schwefel oder durch eine Gruppe NRₐ (worin Rₐ ein Wasserstoffatom oder eine geradkettige oder verzweigte (C₁-C₆)-Alkylgruppe darstellt) oder eine Phenylen- oder Naphthylen-gruppe ersetzt sein kann,
• R³ und R⁴, die gleichartig oder verschieden sind, ein Wasserstoff- oder Halogenatom oder eine Gruppe R, OR oder NRR' bedeuten (worin R und R', die gleichartig oder verschieden sind, ein Wasserstoffatom oder eine geradkettige oder verzweigte (C₁-C₆)-Alkylgruppe, geradkettige oder verzweigte (C₂-C₆)-Alkenylgruppe, geradkettige oder verzweigte (C₂-C₆)-Alkinylgruppe, Arylgruppe, geradkettige oder verzweigte Aryl-(C₁-C₆)-alkylgruppe, geradkettige oder verzweigte Aryl-(C₂-C₆)-alkenylgruppe, geradkettige oder verzweigte Aryl-(C₂-C₆)-alkinylgruppe, Heteroarylgruppe, geradkettige oder verzweigte Heteroaryl-(C₁-C₆)-alkylgruppe, geradkettige oder verzweigte Heteroaryl-(C₂-C₆)-alkenylgruppe, geradkettige oder verzweigte Heteroaryl-(C₂-C₆)-alkinylgruppe, (C₃-C₈)-Cycloalkylgruppe, geradkettige oder verzweigte (C₃-C₈)-Cycloalkyl-(C₁-C₆)-alkylgruppe oder geradkettige oder verzweigte (C₁-C₆)-Polyhalogenalkylgruppe darstellen), oder R³ und R⁴ gemeinsam mit den sie tragenden Kohlenstoffatomen, wenn sie von zwei benachbarten Kohlenstoffatomen getragen werden, einen Ring bilden, der 5 oder 6 Kettenglieder aufweist und ein Heteroatom ausgewählt aus Sauerstoff, Schwefel und Stickstoff enthalten kann,
• R⁵ und R⁶, die gleichartig oder verschieden sind, sämtliche Bedeutungen von R, wie sie oben definiert worden sind, aufweisen können,
• D:
einen Benzolkern darstellt, wobei in diesem Fall X keine Gruppe 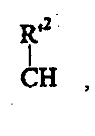 wie sie oben definiert worden ist, darstellt,
oder D einen Pyridin-, Pyrazin-, Pyrimidin- oder Pyridazin-rest bedeutet,
• B eine geradkettige oder verzweigte (C₁-C₆)-Alkylgruppe oder geradkettige oder verzweigte (C₂-C₆)-Alkenylgruppe darstellt, wobei diese Gruppen:
◆ durch eine Gruppe der Formel (II): 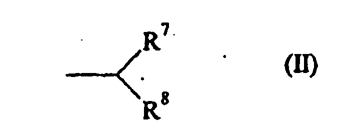 in der: - R⁷ eine Gruppe 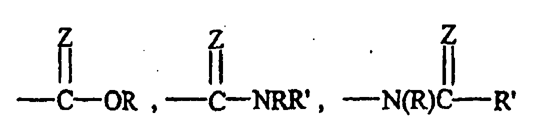 oder 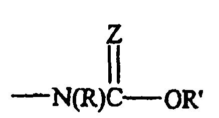 darstellt, worin Z ein Sauerstoff- oder Schwefelatom bedeutet und R und R', die gleichartig oder verschieden sind, die oben angegebenen Bedeutungen besitzen,
- und R⁸ eine Arylgruppe, Arylalkylgruppe, deren Alkylrest 1 bis 6 Kohlenstoffatome enthält und geradkettig oder verzweigt sein kann, Heteroarylgruppe, Heteroarylalkylgruppe, deren Alkylgruppe 1 bis 6 Kohlenstoffatome enthält und geradkettig oder verzweigt sein kann, CN, Tetrazolgruppe, Gruppe der Formeln -OR, -NRR', 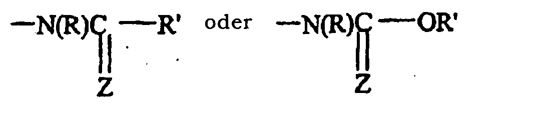 darstellt, worin Z die oben angegebenen Bedeutungen besitzt und R und R', die gleichartig oder verschieden sind, sämtliche oben angegebenen Bedeutungen besitzen können,
◆ oder durch eine Gruppe R⁹ substituiert ist, worin R⁹ eine Gruppe CN, Tetrazolgruppe, Gruppe der Formeln 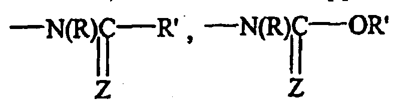 oder 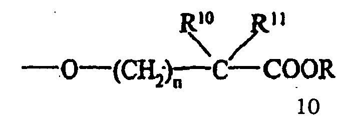 darstellt, worin Z die oben angegebenen Bedeutungen besitzt und R und R', die gleichartig oder verschieden sind, sämtliche Bedeutungen aufweisen können, wie sie oben definiert worden sind, n 0, 1, 2, 3, 4, 5 oder 6 bedeutet und R¹⁰ und R¹¹, die gleichartig oder verschieden sind, ein Wasserstoffatom oder eine geradkettige oder verzweigte (C₁-C₆)-Alkylgruppe darstellen, mit der Maßgabe, daß R¹⁰ und R¹¹ nicht gleichzeitig ein Wasserstoffatom bedeuten können,
oder B eine Gruppe der Formel (II) oder eine Gruppe R⁹, wie sie oben definiert worden sind, bedeutet,
wobei es sich versteht, daß:
* das Oxim R⁶-C(=N-OR⁵)- die Konfiguration Z oder E aufweisen kann,
* man unter Aryl eine Phenyl-, Naphthyl- oder Biphenylgruppe versteht, wobei diese Gruppen teilweise hydriert sein können,
* man unter Heteroaryl jede aromatische mono- oder bicyclische Gruppe versteht, die 5 bis 10 Kettenglieder aufweist, im Fall von bicyclischen Heteroarylgruppen an einem der Ringe teilweise hydriert sein kann, und 1 bis 3 Heteroatome ausgewählt aus Sauerstoff, Stickstoff und Schwefel enthält,
wobei die in dieser Weise definierten Aryl- und Heteroarylgruppen durch 1 bis 3 Gruppen ausgewählt aus geradkettigem oder verzweigtem (C₁-C₆)-Alkyl, geradkettigem oder verzweigtem (C₁-C₆)-Polyhalogenalkyl, geradkettigem oder verzweigtem (C₁-C₆)-Alkoxy, Hydroxy, Carboxy, Formyl, NR_{b}R_{c} (worin R_{b} und R_{c}, die gleichartig oder verschieden sind, ein Wasserstoffatom, eine geradkettige oder verzweigte (C₁-C₆)-Alkylgruppe, Arylgruppe oder Heteroarylgruppe bedeuten), Estergruppen, Amidogruppen, Nitrogruppen, Cyanogruppen oder Halogenatome substituiert sein können,
deren Enantiomere und Diastereoisomere sowie deren Additionssalze mit einer pharmazeutisch annehmbaren Säure oder Base.

2. Verbindungen der Formel (I) nach Anspruch 1, worin R¹ und R² gemeinsam eine Oxogruppe bilden, deren Enantiomere und Diastereoisomere sowie deren Additionssalze mit einer pharmazeutisch annehmbaren Säure oder Base.

3. Verbindungen der Formel (I) nach Anspruch 1, worin R³ und R⁴ ein Wasserstoffatom bedeuten, deren Enantiomere und Diastereoisomere sowie deren Additionssalze mit einer pharmazeutisch annehmbaren Säure oder Base.

4. Verbindungen der Formel (I) nach Anspruch 1, worin A eine Ethylenoxygruppe bedeutet, deren Enantiomere und Diastereoisomere sowie deren Additionssalze mit einer pharmazeutisch annehmbaren Säure oder Base.

5. Verbindungen der Formel (I) nach Anspruch 1, worin D einen Benzolkern bedeutet, deren Enantiomere und Diastereoisomere sowie deren Additionssalze mit einer pharmazeutisch annehmbaren Säure oder Base.

6. Verbindungen der Formel (I) nach Anspruch 1, worin X ein Schwefelatom bedeutet, deren Enantiomere und Diastereoisomere sowie deren Additionssalze mit einer pharmazeutisch annehmbaren Säure oder Base.

7. Verbindungen der Formel (I) nach Anspruch 1, worin R⁵ ein Wasserstoffatom bedeutet, deren Enantiomere und Diastereoisomere sowie deren Additionssalze mit einer pharmazeutisch annehmbaren Säure oder Base.

8. Verbindungen der Formel (I) nach Anspruch 1, worin R⁵ eine Alkylgruppe bedeutet, deren Enantiomere und Diastereoisomere sowie deren Additionssalze mit einer pharmazeutisch annehmbaren Säure oder Base.

9. Verbindungen der Formel (I) nach Anspruch 1, worin R⁶ eine nichtsubstituierte Phenylgruppe bedeutet, deren Enantiomere und Diastereoisomere sowie deren Additionssalze mit einer pharmazeutisch annehmbaren Säure oder Base.

10. Verbindungen der Formel (I) nach Anspruch 1, worin R⁶ eine substituierte Phenylgruppe bedeutet, deren Enantiomere und Diastereoisomere sowie deren Additionssalze mit einer pharmazeutisch annehmbaren Säure oder Base.

11. Verbindungen der Formel (I) nach Anspruch 1, worin R⁶ eine durch ein Halogenatom substituierte Phenylgruppe bedeutet, deren Enantiomere und Diastereoisomere sowie deren Additionssalze mit einer pharmazeutisch annehmbaren Säure oder Base.

12. Verbindungen der Formel (I) nach Anspruch 1, worin B eine Alkylgruppe bedeutet, die durch eine Gruppe 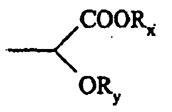 substituiert ist, in der Rₓ und R_{y}, die gleichartig oder verschieden sind, ein Wasserstoffatom oder eine Alkylgruppe bedeuten, deren Enantiomere und Diastereoisomere sowie deren Additionssalze mit einer pharmazeutisch annehmbaren Säure oder Base.

13. Verbindungen der Formel (I) nach Anspruch 1, worin B eine Alkylgruppe bedeutet, die durch eine Gruppe 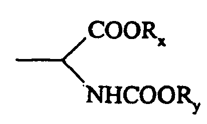 substituiert ist, in der Rₓ und R_{y}, die gleichartig oder verschieden sind, ein Wasserstoffatom oder eine Alkylgruppe bedeutet, deren Enantiomere und Diastereoisomere sowie deren Additionssalze mit einer pharmazeutisch annehmbaren Säure oder Base.

14. Verbindungen der Formel (I) nach Anspruch 1, worin B eine Alkylgruppe bedeutet, die durch eine Gruppe 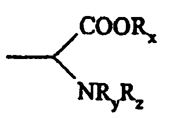 substituiert ist, in der Rₓ, R_{y} und R_{z}, die gleichartig oder verschieden sind, ein Wasserstoffatom oder eine Alkylgruppe bedeutet, deren Enantiomere und Diastereoisomere sowie deren Additionssalze mit einer pharmazeutisch annehmbaren Säure oder Base.

15. Verbindungen der Formel (I) nach Anspruch 1, worin B eine Gruppe 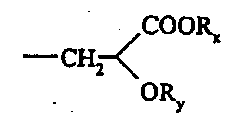 bedeutet, in der Rₓ und R_{y}, die gleichartig oder verschieden sind, ein Wasserstoffatom oder eine Alkylgruppe bedeuten, deren Enantiomere und Diastereoisomere sowie deren Additionssalze mit einer pharmazeutisch annehmbaren Säure oder Base.

16. Verbindungen der Formel (I) nach Anspruch 1, worin B eine Gruppe 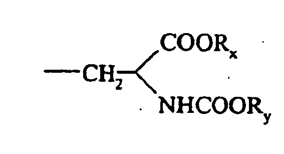 bedeutet, in der Rₓ und R_{y}, die gleichartig oder verschieden sind, ein Wasserstoffatom oder eine Alkylgruppe bedeutet, deren Enantiomere und Diastereoisomere sowie deren Additionssalze mit einer pharmazeutisch annehmbaren Säure oder Base.

17. Verbindungen der Formel (I) nach Anspruch 1, worin B eine Gruppe 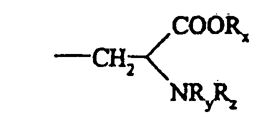 bedeutet, in der Rₓ, R_{y} und R_{z}, die gleichartig oder verschieden sind, ein Wasserstoffatom oder eine Alkylgruppe bedeutet, deren Enantiomere und Diastereoisomere sowie deren Additionssalze mit einer pharmazeutisch annehmbaren Säure oder Base.

18. Verbindungen der Formel (I) nach Anspruch 1, nämlich 2-Ethoxy-3-{4-[2-(6-[(*E*)(methoxyimino)(phenyl)-methyl]-2-oxo-1,3-benzothiazol-3(2H)-yl)-ethoxy]-phenyl}-propansäuremethylester, 2-Ethoxy-3-{4-[2-(6-[(*Z*)-(hydroxyimino)(phenyl)-methyl]-2-oxo-1,3-benzothiazol-3(2*H*)-yl)-ethoxy]-phenyl}-propansäuremethylester, 3-{4-[2-(6-[(Methoxyimino)(phenyl)-methyl]-2-oxo-1,3-benzothiazol-3(2*H*)-yl)-ethoxy]-phenyl}-2-(2,2,2-trifluorethoxy)-propansäuremethylester, 3-{4-[2-(6-[(Hydroxyimino)(phenyl)-methyl]-2-oxo-1,3-benzothiazol-3(2*H*)-yl)-ethoxy]-phenyl}-2-(2,2,2-trifluorethoxy)-propansäuremethylester, 2-Ethoxy-3-{4-[2-(6-[(methoxyimino)(phenyl)-methyl]-2-oxo-1,3-benzothiazol-3(2*H*)-yl)-ethoxy]-phenyl}-propansäure, 2-Ethoxy-3-{4-[2-(6-[(*E*)(methoxyimino)(phenyl)-methyl]-2-oxo-1,3-benzothiazol-3(2*H*)-yl)-ethoxy]-phenyl}-propansäure, 2-Ethoxy-3-{4-[2-(6-[(*E*)(methoxyimino)(phenyl)-methyl]-2-oxo-1,3-benzothiazol-3(2*H*)-yl)-ethoxy]-phenyl}-propansäure Enantiomeres 1, 2-Ethoxy-3-(4-[2-(6-[(*E*)(methoxyimino)(phenyl)-methyl]-2-oxo-1,3-benzothiazol-3(2*H*)-yl)-ethoxy]-phenyl}-propansäure Enantiomeres 2, 2-Ethoxy-3-{4-[2-(6-[(*Z*)-(methoxyimino)(phenyl)-methyl]-2-oxo-1,3-benzothiazol-3(2*H*)-yl)-ethoxy]-phenyl}propansäure, 2-Ethoxy-3-{4-(2-(6-[(*Z*)(methoxyimino)(phenyl)-methyl]-2-oxo-1,3-benzothiazol-3(2*H*)-yl)-ethoxy)-phenyl}-propansäure Enantiomeres 1, 2-Ethoxy-3-{4-[2-(6-[(*Z*)(methoxyimino)(phenyl)-methyl]-2-oxo-1,3-benzothiazol-3(2*H*)-yl)-ethoxy]-phenyl}-propansäure Enantiomeres 2, 2-Ethoxy-3-{4-[2-(6-[(hydroxyimino)-(phenyl)-methyl]-2-oxo-1,3-benzothiazol-3(2*H*)-yl)-ethoxy]-phenyl}-propansäure, 3-{4-[2-(6-[(*Z*)(3-Chlorphenyl)(methoxyimino)-methyl]-2-oxo-1,3-benzothiazol-3(2*H*)-yl)-ethoxy]-phenyl}-2-ethoxypropansäuremethylester, 3-{4-[2-(6-[(3-Chlorphenyl)-(hydroxyimino)methyl]-2-oxo-1,3-benzothiazol-3(2*H*)-yl)-ethoxy]-phenyl}-2-ethoxypropansäuremethylester, 2-Methoxy-3-{4-[2-(6-[(methoxyimino)(phenyl)-methyl]-2-oxo-1,3-benzothiazol-3(2*H*)-yl)-ethoxy]-phenyl}-propansäuremethylester, 3-{4-[2-(6-[(*Z*)(3-Chlorphenyl)(methoxyimino)-methyl]-2-oxo-1,3-benzothiazol-3(2*H*)-yl)-ethoxy]-phenyl}-2-ethoxypropansäure, 3-{4-[2-(6-[(Methoxyimino)(phenyl)-methyl]-2-oxo-1,3-benzothiazol-3(2*H*)-yl)-ethoxy]-phenyl}-2-(2,2,2-trifluorethoxy)-propansäure, 3-{4-(2-(6-[(*E*)(Methoxyimino)(phenyl)-methyl]-2-oxo-1,3-benzothiazol-3(2*H*)-yl)-ethoxy]-phenyl}-2-(2,2,2-trifluorethoxy)-propansäure, 3-{4-[2-(6-[(*E*)(Methoxyimino)(phenyl)-methyl]-2-oxo-1,3-benzothiazol-3(2*H*)-yl)-ethoxy]-phenyl}-2-(2,2,2-trifluorethoxy)-propansäure Enantiomeres 1, 3-{4-[2-(6-[(*E*)(Methoxyimino)-(phenyl)-methyl]-2-oxo-1,3-benzothiazol-3(2*H*)-yl)-ethoxy]-phenyl}-2-(2,2,2-trifluorethoxy)-propansäure Enantiomeres 2, 3-{4-[2-(6-[(*Z*)(Methoxyimino)(phenyl)-methyl]-2-oxo-1,3-benzothiazol-3(2*H*)-yl)-ethoxy]-phenyl}-2-(2,2,2-trifluorethoxy)-propansäure, 3-{4-[2-(6-[(*Z*)(Methoxyimino)(phenyl)-methyl]-2-oxo-1,3-benzothiazol-3(2*H*)-yl)-ethoxy]-phenyl}-2-(2,2,2-trifluorethoxy)-propansäure Enantiomeres 1, 3-{4-[2-(6-[(*Z*)(Methoxyimino)(phenyl)-methyl]-2-oxo-1,3-benzothiazol-3(2*H*)-yl)-ethoxy]-phenyl}-2-(2,2,2-trifluorethoxy)-propansäure Enantiomeres 2, 3-{4-[2-(6-[(*tert.*-Butoxyimino)(phenyl)-methyl]-2-oxo-1,3-benzothiazol-3(2*H*)-yl)-ethoxy]-phenyl}-2-ethoxy-propansäure.

19. Verbindungen der Formel (I) nach Anspruch 1, nämlich 2-[(*tert.*-Butoxycarbonyl)-amino]-3-{4-[2-(6-[(methoxyimino)(phenyl)-methyl]-2-oxo-1,3-benzothiazol-3(2*H*)-yl)-ethoxy]-phenyl}-propansäuremethylester, 2-[(*tert*.-Butoxycarbonyl)-amino]-3-{4-[2-(6-[(hydroxyimino)(phenyl)-methyl]-2-oxo-1,3-benzothiazol-3(2*H*)-yl)-ethoxy]-phenyl}-propansäuremethylester, 2-[(Butoxycarbonyl)-amino]-3-{4-[2-(6-[(methoxyimino)(phenyl)-methyl]-2-oxo-1,3-benzothiazol-3(2*H*)-yl)-ethoxy]-phenyl}-propansäuremethylester, 3-{4-[2-(6-[(Methoxyimino)(phenyl)-methyl]-2-oxo-1,3-benzothiazol-3(2*H*)-yl)-ethoxy]-phenyl}-2-[(phenoxycarbonyl)-amino]-propansäuremethylester, 2-{[(Benzyloxy)-carbonyl]-amino}-3-{4-[2-(6-[(hydroxyimino)-phenyl)-methyl]-2-oxo-1,3-benzothiazol-3(2*H*)-yl)-ethoxy]-phenyl}-propansäuremethylester, 2-[(*tert.*-Butoxycarbonyl)(methyl)-amino]-3-{4-[2-(6-[(methoxyimino)(phenyl)-methyl]-2-oxo-1,3-benzothiazol-3(2*H*)-yl)-ethoxy]-phenyl}-propansäuremethylester, *N*-(*tert.*-Butoxycarbonyl)-4-(2-(6-[(hydroxyimino)(phenyl)-methyl]-2-oxo-1,3-benzothiazol-3(2*H*)-yl)-ethoxy]-phenylalanin, *N*-(*tert.*-Butoxycarbonyl)-4-[2-(6-[(methoxyimino)(phenyl)-methyl]-2-oxo-1,3-benzothiazol-3(2*H*)-yl)-ethoxy]-phenylalanin.

20. Verbindungen der Formel (I) nach Anspruch 1, nämlich 2-Amino-3-{4-[2-(6-[(methoxyimino)(phenyl)-methyl]-2-oxo-1,3-benzothiazol-3(2*H*)-yl)-ethoxy]-phenyl}-propansäuremethylester, 2-Amino-3-{4-[2-(6-[(hydroxyimino)(phenyl)-methyl]-2-oxo-1,3-benzothiazol-3(2*H*)-yl)-ethoxy]-phenyl}-propansäuremethylester, 3-{4-[2-(6-[(Methoxyimino)(phenyl)-methyl]-2-oxo-1,3-benzothiazol-3(2*H*)-yl)-ethoxy]-phenyl}-2-(methylamino)-propansäuremethylester.

21. Verfahren zur Herstellung der Verbindungen der Formel (I) nach Anspruch 1, **dadurch gekennzeichnet, daß** man als Ausgangsprodukt eine Verbindung der Formel (III) verwendet: 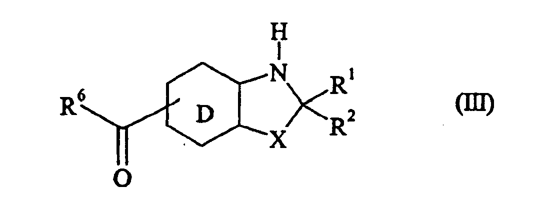 in der D, R¹, R², R⁶ und X die bezüglich der Formel (I) angegebenen Bedeutungen besitzen,
welche man in basischem Medium mit einer Verbindung der Formel (IV) kondensiert: 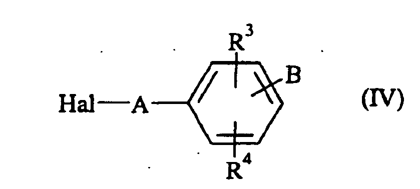 in der A, B, R³ und R⁴ die bezüglich der Formel (I) angegebenen Bedeutungen besitzen und Hal ein Halogenatom darstellt, zur Bildung der Verbindung der Formel (V): 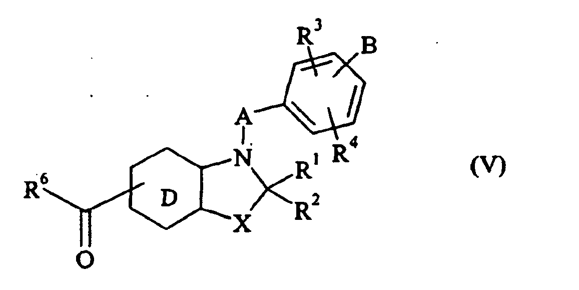 in der R¹, R², R³, R⁴, R⁶, A, B, D und X die bezüglich der Formel (I) angegebenen Bedeutungen besitzen,
welche man der Einwirkung einer Verbindung der Formel R⁵O-NH₂, in der R⁵ die bezüglich der Formel (I) angegebenen Bedeutungen besitzt, unterwirft zur Bildung der Verbindung der Formel (I): 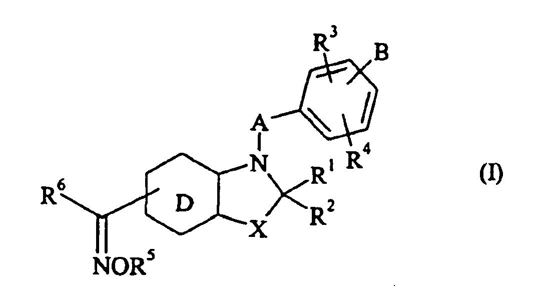 welche mit Hilfe einer klassischen Trennmethode gereinigt werden kann, welche gewünschtenfalls in ihre Additionssalze mit einer pharmazeutisch annehmbaren Säure oder Base überführt werden kann und welche man gegebenenfalls mit Hilfe einer klassischen Trennmethode in ihre Isomeren auftrennt.

22. Verfahren zur Herstellung der Verbindungen der Formel (I) nach Anspruch 1, **dadurch gekennzeichnet, daß** man als Ausgangsprodukt eine Verbindung der Formel (III) verwendet: 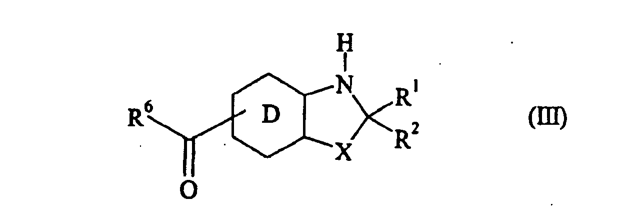 in der D, R¹, R², R⁶ und X die bezüglich der Formel (I) angegebenen Bedeutungen besitzen,
welche man mit einer Verbindung der Formel R⁵O-NH₂, in der R⁵ die bezüglich der Formel (I) angegebenen Bedeutungen besitzt, kondensiert zur Bildung der Verbindung der Formel (VI): 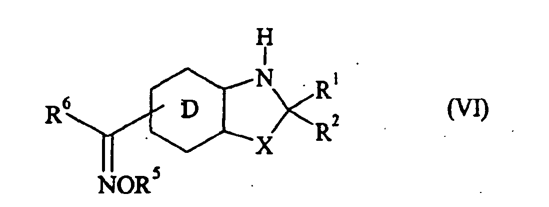 in der R¹, R², R⁵, R⁶, D und X die bezüglich der Formel (I) angegebenen Bedeutungen besitzen,
welche man in basischem Medium mit einer Verbindung der Formel (IV) kondensiert: 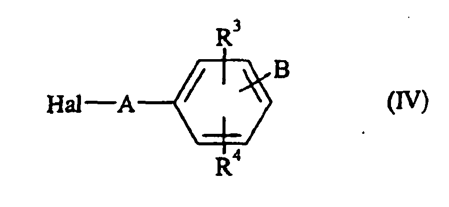 in der A, B, R³ und R⁴ die bezüglich der Formel (I) angegebenen Bedeutungen besitzen und Hal ein Halogenatom darstellt, zur Bildung der Verbindung der Formel (I): 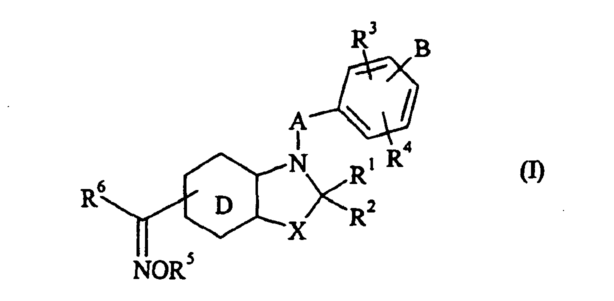 welche mit Hilfe einer klassischen Trennmethode gereinigt werden kann, welche gewünschtenfalls in ihre Additionssalze mit einer pharmazeutisch annehmbaren Säure oder Base überführt werden kann und welche gegebenenfalls mit Hilfe einer klassischen Trennmethode in ihre Isomeren aufgetrennt werden kann.

23. Verbindungen der Formel (V) nach Anspruch 21: 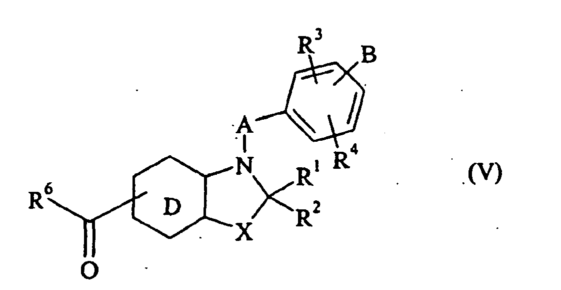 in der R⁶, B, D, A, X, R¹, R², R³ und R⁴ die in Anspruch 1 angegebenen Bedeutungen besitzen,
nützlich als Zwischenprodukte zur Synthese der Verbindungen der Formel (I).

24. Pharmazeutische Zubereitungen enthaltend als Wirkstoff mindestens eine Verbindung der Formel (I) nach einem der Ansprüche 1 bis 20 oder 23 oder eines ihrer Additionssalze mit einer pharmazeutisch annehmbaren Säure oder Base, allein oder in Kombination mit einem oder mehreren pharmazeutisch annehmbaren Hilfsstoffen.

25. Pharmazeutische Zubereitungen nach Anspruch 24 für die Herstellung eines Arzneimittels für die Behandlung und/oder die Prophylaxe von Hyperglykämien, Dyslipidämien und insbesondere für die Behandlung von nicht-insulinabhängigem Diabetes Typ II, der Insulinresistenz, der Glucoseunverträglichkeit, von mit dem Syndrom X verknüpften Störungen, Erkrankungen der Koronararterien und anderen kardiovaskulären Erkrankungen, Nierenerkrankungen, Retinopathien, Störungen, die mit der Aktivierung von Endothelialzellen verknüpft sind, der Psoriasis, des polyzystischen Ovariensyndroms, der Demenz, der Osteoporose, Entzündungserkrankungen der Verdauungsorgane, myotonischen Dystrophien, Pankreatitis, Arteriosklerose, Xanthom, jedoch auch zur Behandlung oder zur Vorbeugung von Diabetes Typ I, der Fettsucht, zur Appetitsteuerung, der Anorexie, der Bulimie, der nervösen Anorexie sowie von Krebserkrankungen und insbesondere hormonabhängigen Krebsen, wie Brustkrebs und Darmkrebs, sowie als Inhibitoren der Angiogenese.
